# EUROPEAN PATENT APPLICATION

(11) **EP 3 725 323 A1**
(43) Date of publication of application: **21.10.2020**
(21) Application number: 19169833.1
(22) Date of filing: 17.04.2019
(51) Int. Cl.: A61K 35/761, A61K 48/00, C07K 14/705, C12N 9/78, C12N 15/86

(54) **ONCOLYTIC ADENOVIRAL VECTOR EXPRESSING A MEMBER OF THE B7 FAMILY OF COSTIMULATORY LIGANDS AND ADA**

(71) Applicant: TARGOVAX ASA, 0283 Oslo (NO)
(72) Inventor: KURYK, Lukasz, 00180 Helsinki (FI); ERIKSEN, Jon Amund, 3916 Porsgrunn (NO); SKORPIL, Peter, 1447 Drøbak (NO); BURNS, Robert, Oxford, OX1 5ER (GB); MØLLER, Anne-Sophie, 1358 Jar (NO)
(74) Representative: Boco IP Oy Ab

(57) **Abstract**

The present invention relates to cancer therapies. More specifically, the present invention relates to oncolytic adenoviral vectors and cells and pharmaceutical compositions comprising said vectors. The present invention also relates to a use of said vectors in the manufacture of a medicament for treating cancer in a subject and a method of treating cancer in a subject. Furthermore, the present invention relates to methods of producing B7 family of immune-regulatory ligands and ADA in a cell and increasing anti-tumor effect and induction of specific immune response in a subject, as well as uses of the oncolytic adenoviral vector of the invention for producing transgenes in a cell and increasing anti-tumor effect and generation of specific immune response in a subject.

SEQUENCE LISTING

<110> Targovax ASA

<120> ONCOLYTIC ADENOVIRAL VECTOR EXPRESSING A MEMBER OF THE B7 FAMILY OF
COSTIMULATORY LIGANDS AND ADA

<130> B8374PEP

<160> 24

<170> BiSSAP 1.3.6

<210> 1
<211> 37814
<212> DNA
<213> Artificial Sequence

<220>
<223> AdV5/3-D24-CMV(CuO)-ICOSL-IRES-ADA-SV40pA (TGX-11)

<400> 1
attaacatca tcaataatat accttatttt ggattgaagc caatatgata atgagggggt 60

ggagtttgtg acgtggcgcg gggcgtggga acggggcggg tgacgtagta gtgtggcgga 120

agtgtgatgt tgcaagtgtg gcggaacaca tgtaagcgac ggatgtggca aaagtgacgt 180

ttttggtgtg cgccggtgta cacaggaagt gacaattttc gcgcggtttt aggcggatgt 240

tgtagtaaat ttgggcgtaa ccgagtaaga tttggccatt ttcgcgggaa aactgaataa 300

gaggaagtga aatctgaata attttgtgtt actcatagcg cgtaatattt gtctagggcc 360

gcggggactt tgaccgttta cgtggagact cgcccaggtg tttttctcag gtgttttccg 420

cgttccgggt caaagttggc gttttattat tatagtcagc tgacgtgtag tgtatttata 480

cccggtgagt tcctcaagag gccactcttg agtgccagcg agtagagttt tctcctccga 540

gccgctccga caccgggact gaaaatgaga catattatct gccacggagg tgttattacc 600

gaagaaatgg ccgccagtct tttggaccag ctgatcgaag aggtactggc tgataatctt 660

ccacctccta gccattttga accacctacc cttcacgaac tgtatgattt agacgtgacg 720

gcccccgaag atcccaacga ggaggcggtt tcgcagattt ttcccgactc tgtaatgttg 780

gcggtgcagg aagggattga cttactcact tttccgccgg cgcccggttc tccggagccg 840

cctcaccttt cccggcagcc cgagcagccg gagcagagag ccttgggtcc ggtttctatg 900

ccaaaccttg taccggaggt gatcgatcca cccagtgacg acgaggatga agagggtgag 960

gagtttgtgt tagattatgt ggagcacccc gggcacggtt gcaggtcttg tcattatcac 1020

cggaggaata cgggggaccc agatattatg tgttcgcttt gctatatgag gacctgtggc 1080

atgtttgtct acagtaagtg aaaattatgg gcagtgggtg atagagtggt gggtttggtg 1140

tggtaatttt ttttttaatt tttacagttt tgtggtttaa agaattttgt attgtgattt 1200

ttttaaaagg tcctgtgtct gaacctgagc ctgagcccga gccagaaccg gagcctgcaa 1260

gacctacccg ccgtcctaaa atggcgcctg ctatcctgag acgcccgaca tcacctgtgt 1320

ctagagaatg caatagtagt acggatagct gtgactccgg tccttctaac acacctcctg 1380

agatacaccc ggtggtcccg ctgtgcccca ttaaaccagt tgccgtgaga gttggtgggc 1440

gtcgccaggc tgtggaatgt atcgaggact tgcttaacga gcctgggcaa cctttggact 1500

tgagctgtaa acgccccagg ccataaggtg taaacctgtg attgcgtgtg tggttaacgc 1560

ctttgtttgc tgaatgagtt gatgtaagtt taataaaggg tgagataatg tttaacttgc 1620

atggcgtgtt aaatggggcg gggcttaaag ggtatataat gcgccgtggg ctaatcttgg 1680

ttacatctga cctcatggag gcttgggagt gtttggaaga tttttctgct gtgcgtaact 1740

tgctggaaca gagctctaac agtacctctt ggttttggag gtttctgtgg ggctcatccc 1800

aggcaaagtt agtctgcaga attaaggagg attacaagtg ggaatttgaa gagcttttga 1860

aatcctgtgg tgagctgttt gattctttga atctgggtca ccaggcgctt ttccaagaga 1920

aggtcatcaa gactttggat ttttccacac cggggcgcgc tgcggctgct gttgcttttt 1980

tgagttttat aaaggataaa tggagcgaag aaacccatct gagcgggggg tacctgctgg 2040

attttctggc catgcatctg tggagagcgg ttgtgagaca caagaatcgc ctgctactgt 2100

tgtcttccgt ccgcccggcg ataataccga cggaggagca gcagcagcag caggaggaag 2160

ccaggcggcg gcggcaggag cagagcccat ggaacccgag agccggcctg gaccctcggg 2220

aatgaatgtt gtacaggtgg ctgaactgta tccagaactg agacgcattt tgacaattac 2280

agaggatggg caggggctaa agggggtaaa gagggagcgg ggggcttgtg aggctacaga 2340

ggaggctagg aatctagctt ttagcttaat gaccagacac cgtcctgagt gtattacttt 2400

tcaacagatc aaggataatt gcgctaatga gcttgatctg ctggcgcaga agtattccat 2460

agagcagctg accacttact ggctgcagcc aggggatgat tttgaggagg ctattagggt 2520

atatgcaaag gtggcactta ggccagattg caagtacaag atcagcaaac ttgtaaatat 2580

caggaattgt tgctacattt ctgggaacgg ggccgaggtg gagatagata cggaggatag 2640

ggtggccttt agatgtagca tgataaatat gtggccgggg gtgcttggca tggacggggt 2700

ggttattatg aatgtaaggt ttactggccc caattttagc ggtacggttt tcctggccaa 2760

taccaacctt atcctacacg gtgtaagctt ctatgggttt aacaatacct gtgtggaagc 2820

ctggaccgat gtaaggttcc ggggctgtgc cttttactgc tgctggaagg gggtggtgtg 2880

tcgccccaaa agcagggctt caattaagaa atgcctcttt gaaaggtgta ccttgggtat 2940

cctgtctgag ggtaactcca gggtgcgcca caatgtggcc tccgactgtg gttgcttcat 3000

gctagtgaaa agcgtggctg tgattaagca taacatggta tgtggcaact gcgaggacag 3060

ggcctctcag atgctgacct gctcggacgg caactgtcac ctgctgaaga ccattcacgt 3120

agccagccac tctcgcaagg cctggccagt gtttgagcat aacatactga cccgctgttc 3180

cttgcatttg ggtaacagga ggggggtgtt cctaccttac caatgcaatt tgagtcacac 3240

taagatattg cttgagcccg agagcatgtc caaggtgaac ctgaacgggg tgtttgacat 3300

gaccatgaag atctggaagg tgctgaggta cgatgagacc cgcaccaggt gcagaccctg 3360

cgagtgtggc ggtaaacata ttaggaacca gcctgtgatg ctggatgtga ccgaggagct 3420

gaggcccgat cacttggtgc tggcctgcac ccgcgctgag tttggctcta gcgatgaaga 3480

tacagattga ggtactgaaa tgtgtgggcg tggcttaagg gtgggaaaga atatataagg 3540

tgggggtctt atgtagtttt gtatctgttt tgcagcagcc gccgccgcca tgagcaccaa 3600

ctcgtttgat ggaagcattg tgagctcata tttgacaacg cgcatgcccc catgggccgg 3660

ggtgcgtcag aatgtgatgg gctccagcat tgatggtcgc cccgtcctgc ccgcaaactc 3720

tactaccttg acctacgaga ccgtgtctgg aacgccgttg gagactgcag cctccgccgc 3780

cgcttcagcc gctgcagcca ccgcccgcgg gattgtgact gactttgctt tcctgagccc 3840

gcttgcaagc agtgcagctt cccgttcatc cgcccgcgat gacaagttga cggctctttt 3900

ggcacaattg gattctttga cccgggaact taatgtcgtt tctcagcagc tgttggatct 3960

gcgccagcag gtttctgccc tgaaggcttc ctcccctccc aatgcggttt aaaacataaa 4020

taaaaaacca gactctgttt ggatttggat caagcaagtg tcttgctgtc tttatttagg 4080

ggttttgcgc gcgcggtagg cccgggacca gcggtctcgg tcgttgaggg tcctgtgtat 4140

tttttccagg acgtggtaaa ggtgactctg gatgttcaga tacatgggca taagcccgtc 4200

tctggggtgg aggtagcacc actgcagagc ttcatgctgc ggggtggtgt tgtagatgat 4260

ccagtcgtag caggagcgct gggcgtggtg cctaaaaatg tctttcagta gcaagctgat 4320

tgccaggggc aggcccttgg tgtaagtgtt tacaaagcgg ttaagctggg atgggtgcat 4380

acgtggggat atgagatgca tcttggactg tatttttagg ttggctatgt tcccagccat 4440

atccctccgg ggattcatgt tgtgcagaac caccagcaca gtgtatccgg tgcacttggg 4500

aaatttgtca tgtagcttag aaggaaatgc gtggaagaac ttggagacgc ccttgtgacc 4560

tccaagattt tccatgcatt cgtccataat gatggcaatg ggcccacggg cggcggcctg 4620

ggcgaagata tttctgggat cactaacgtc atagttgtgt tccaggatga gatcgtcata 4680

ggccattttt acaaagcgcg ggcggagggt gccagactgc ggtataatgg ttccatccgg 4740

cccaggggcg tagttaccct cacagatttg catttcccac gctttgagtt cagatggggg 4800

gatcatgtct acctgcgggg cgatgaagaa aacggtttcc ggggtagggg agatcagctg 4860

ggaagaaagc aggttcctga gcagctgcga cttaccgcag ccggtgggcc cgtaaatcac 4920

acctattacc ggctgcaact ggtagttaag agagctgcag ctgccgtcat ccctgagcag 4980

gggggccact tcgttaagca tgtccctgac tcgcatgttt tccctgacca aatccgccag 5040

aaggcgctcg ccgcccagcg atagcagttc ttgcaaggaa gcaaagtttt tcaacggttt 5100

gagaccgtcc gccgtaggca tgcttttgag cgtttgacca agcagttcca ggcggtccca 5160

cagctcggtc acctgctcta cggcatctcg atccagcata tctcctcgtt tcgcgggttg 5220

gggcggcttt cgctgtacgg cagtagtcgg tgctcgtcca gacgggccag ggtcatgtct 5280

ttccacgggc gcagggtcct cgtcagcgta gtctgggtca cggtgaaggg gtgcgctccg 5340

ggctgcgcgc tggccagggt gcgcttgagg ctggtcctgc tggtgctgaa gcgctgccgg 5400

tcttcgccct gcgcgtcggc caggtagcat ttgaccatgg tgtcatagtc cagcccctcc 5460

gcggcgtggc ccttggcgcg cagcttgccc ttggaggagg cgccgcacga ggggcagtgc 5520

agacttttga gggcgtagag cttgggcgcg agaaataccg attccgggga gtaggcatcc 5580

gcgccgcagg ccccgcagac ggtctcgcat tccacgagcc aggtgagctc tggccgttcg 5640

gggtcaaaaa ccaggtttcc cccatgcttt ttgatgcgtt tcttacctct ggtttccatg 5700

agccggtgtc cacgctcggt gacgaaaagg ctgtccgtgt ccccgtatac agacttgaga 5760

ggcctgtcct cgagcggtgt tccgcggtcc tcctcgtata gaaactcgga ccactctgag 5820

acaaaggctc gcgtccaggc cagcacgaag gaggctaagt gggaggggta gcggtcgttg 5880

tccactaggg ggtccactcg ctccagggtg tgaagacaca tgtcgccctc ttcggcatca 5940

aggaaggtga ttggtttgta ggtgtaggcc acgtgaccgg gtgttcctga aggggggcta 6000

taaaaggggg tgggggcgcg ttcgtcctca ctctcttccg catcgctgtc tgcgagggcc 6060

agctgttggg gtgagtactc cctctgaaaa gcgggcatga cttctgcgct aagattgtca 6120

gtttccaaaa acgaggagga tttgatattc acctggcccg cggtgatgcc tttgagggtg 6180

gccgcatcca tctggtcaga aaagacaatc tttttgttgt caagcttggt ggcaaacgac 6240

ccgtagaggg cgttggacag caacttggcg atggagcgca gggtttggtt tttgtcgcga 6300

tcggcgcgct ccttggccgc gatgtttagc tgcacgtatt cgcgcgcaac gcaccgccat 6360

tcgggaaaga cggtggtgcg ctcgtcgggc accaggtgca cgcgccaacc gcggttgtgc 6420

agggtgacaa ggtcaacgct ggtggctacc tctccgcgta ggcgctcgtt ggtccagcag 6480

aggcggccgc ccttgcgcga gcagaatggc ggtagggggt ctagctgcgt ctcgtccggg 6540

gggtctgcgt ccacggtaaa gaccccgggc agcaggcgcg cgtcgaagta gtctatcttg 6600

catccttgca agtctagcgc ctgctgccat gcgcgggcgg caagcgcgcg ctcgtatggg 6660

ttgagtgggg gaccccatgg catggggtgg gtgagcgcgg aggcgtacat gccgcaaatg 6720

tcgtaaacgt agaggggctc tctgagtatt ccaagatatg tagggtagca tcttccaccg 6780

cggatgctgg cgcgcacgta atcgtatagt tcgtgcgagg gagcgaggag gtcgggaccg 6840

aggttgctac gggcgggctg ctctgctcgg aagactatct gcctgaagat ggcatgtgag 6900

ttggatgata tggttggacg ctggaagacg ttgaagctgg cgtctgtgag acctaccgcg 6960

tcacgcacga aggaggcgta ggagtcgcgc agcttgttga ccagctcggc ggtgacctgc 7020

acgtctaggg cgcagtagtc cagggtttcc ttgatgatgt catacttatc ctgtcccttt 7080

tttttccaca gctcgcggtt gaggacaaac tcttcgcggt ctttccagta ctcttggatc 7140

ggaaacccgt cggcctccga acggtaagag cctagcatgt agaactggtt gacggcctgg 7200

taggcgcagc atcccttttc tacgggtagc gcgtatgcct gcgcggcctt ccggagcgag 7260

gtgtgggtga gcgcaaaggt gtccctgacc atgactttga ggtactggta tttgaagtca 7320

gtgtcgtcgc atccgccctg ctcccagagc aaaaagtccg tgcgcttttt ggaacgcgga 7380

tttggcaggg cgaaggtgac atcgttgaag agtatctttc ccgcgcgagg cataaagttg 7440

cgtgtgatgc ggaagggtcc cggcacctcg gaacggttgt taattacctg ggcggcgagc 7500

acgatctcgt caaagccgtt gatgttgtgg cccacaatgt aaagttccaa gaagcgcggg 7560

atgcccttga tggaaggcaa ttttttaagt tcctcgtagg tgagctcttc aggggagctg 7620

agcccgtgct ctgaaagggc ccagtctgca agatgagggt tggaagcgac gaatgagctc 7680

cacaggtcac gggccattag catttgcagg tggtcgcgaa aggtcctaaa ctggcgacct 7740

atggccattt tttctggggt gatgcagtag aaggtaagcg ggtcttgttc ccagcggtcc 7800

catccaaggt tcgcggctag gtctcgcgcg gcagtcacta gaggctcatc tccgccgaac 7860

ttcatgacca gcatgaaggg cacgagctgc ttcccaaagg cccccatcca agtataggtc 7920

tctacatcgt aggtgacaaa gagacgctcg gtgcgaggat gcgagccgat cgggaagaac 7980

tggatctccc gccaccaatt ggaggagtgg ctattgatgt ggtgaaagta gaagtccctg 8040

cgacgggccg aacactcgtg ctggcttttg taaaaacgtg cgcagtactg gcagcggtgc 8100

acgggctgta catcctgcac gaggttgacc tgacgaccgc gcacaaggaa gcagagtggg 8160

aatttgagcc cctcgcctgg cgggtttggc tggtggtctt ctacttcggc tgcttgtcct 8220

tgaccgtctg gctgctcgag gggagttacg gtggatcgga ccaccacgcc gcgcgagccc 8280

aaagtccaga tgtccgcgcg cggcggtcgg agcttgatga caacatcgcg cagatgggag 8340

ctgtccatgg tctggagctc ccgcggcgtc aggtcaggcg ggagctcctg caggtttacc 8400

tcgcatagac gggtcagggc gcgggctaga tccaggtgat acctaatttc caggggctgg 8460

ttggtggcgg cgtcgatggc ttgcaagagg ccgcatcccc gcggcgcgac tacggtaccg 8520

cgcggcgggc ggtgggccgc gggggtgtcc ttggatgatg catctaaaag cggtgacgcg 8580

ggcgagcccc cggaggtagg gggggctccg gacccgccgg gagagggggc aggggcacgt 8640

cggcgccgcg cgcgggcagg agctggtgct gcgcgcgtag gttgctggcg aacgcgacga 8700

cgcggcggtt gatctcctga atctggcgcc tctgcgtgaa gacgacgggc ccggtgagct 8760

tgaacctgaa agagagttcg acagaatcaa tttcggtgtc gttgacggcg gcctggcgca 8820

aaatctcctg cacgtctcct gagttgtctt gataggcgat ctcggccatg aactgctcga 8880

tctcttcctc ctggagatct ccgcgtccgg ctcgctccac ggtggcggcg aggtcgttgg 8940

aaatgcgggc catgagctgc gagaaggcgt tgaggcctcc ctcgttccag acgcggctgt 9000

agaccacgcc cccttcggca tcgcgggcgc gcatgaccac ctgcgcgaga ttgagctcca 9060

cgtgccgggc gaagacggcg tagtttcgca ggcgctgaaa gaggtagttg agggtggtgg 9120

cggtgtgttc tgccacgaag aagtacataa cccagcgtcg caacgtggat tcgttgatat 9180

cccccaaggc ctcaaggcgc tccatggcct cgtagaagtc cacggcgaag ttgaaaaact 9240

gggagttgcg cgccgacacg gttaactcct cctccagaag acggatgagc tcggcgacag 9300

tgtcgcgcac ctcgcgctca aaggctacag gggcctcttc ttcttcttca atctcctctt 9360

ccataagggc ctccccttct tcttcttctg gcggcggtgg gggagggggg acacggcggc 9420

gacgacggcg caccgggagg cggtcgacaa agcgctcgat catctccccg cggcgacggc 9480

gcatggtctc ggtgacggcg cggccgttct cgcgggggcg cagttggaag acgccgcccg 9540

tcatgtcccg gttatgggtt ggcggggggc tgccatgcgg cagggatacg gcgctaacga 9600

tgcatctcaa caattgttgt gtaggtactc cgccgccgag ggacctgagc gagtccgcat 9660

cgaccggatc ggaaaacctc tcgagaaagg cgtctaacca gtcacagtcg caaggtaggc 9720

tgagcaccgt ggcgggcggc agcgggcggc ggtcggggtt gtttctggcg gaggtgctgc 9780

tgatgatgta attaaagtag gcggtcttga gacggcggat ggtcgacaga agcaccatgt 9840

ccttgggtcc ggcctgctga atgcgcaggc ggtcggccat gccccaggct tcgttttgac 9900

atcggcgcag gtctttgtag tagtcttgca tgagcctttc taccggcact tcttcttctc 9960

cttcctcttg tcctgcatct cttgcatcta tcgctgcggc ggcggcggag tttggccgta 10020

ggtggcgccc tcttcctccc atgcgtgtga ccccgaagcc cctcatcggc tgaagcaggg 10080

ctaggtcggc gacaacgcgc tcggctaata tggcctgctg cacctgcgtg agggtagact 10140

ggaagtcatc catgtccaca aagcggtggt atgcgcccgt gttgatggtg taagtgcagt 10200

tggccataac ggaccagtta acggtctggt gacccggctg cgagagctcg gtgtacctga 10260

gacgcgagta agccctcgag tcaaatacgt agtcgttgca agtccgcacc aggtactggt 10320

atcccaccaa aaagtgcggc ggcggctggc ggtagagggg ccagcgtagg gtggccgggg 10380

ctccgggggc gagatcttcc aacataaggc gatgatatcc gtagatgtac ctggacatcc 10440

aggtgatgcc ggcggcggtg gtggaggcgc gcggaaagtc gcggacgcgg ttccagatgt 10500

tgcgcagcgg caaaaagtgc tccatggtcg ggacgctctg gccggtcagg cgcgcgcaat 10560

cgttgacgct ctagaccgtg caaaaggaga gcctgtaagc gggcactctt ccgtggtctg 10620

gtggataaat tcgcaagggt atcatggcgg acgaccgggg ttcgagcccc gtatccggcc 10680

gtccgccgtg atccatgcgg ttaccgcccg cgtgtcgaac ccaggtgtgc gacgtcagac 10740

aacgggggag tgctcctttt ggcttccttc caggcgcggc ggctgctgcg ctagcttttt 10800

tggccactgg ccgcgcgcag cgtaagcggt taggctggaa agcgaaagca ttaagtggct 10860

cgctccctgt agccggaggg ttattttcca agggttgagt cgcgggaccc ccggttcgag 10920

tctcggaccg gccggactgc ggcgaacggg ggtttgcctc cccgtcatgc aagaccccgc 10980

ttgcaaattc ctccggaaac agggacgagc cccttttttg cttttcccag atgcatccgg 11040

tgctgcggca gatgcgcccc cctcctcagc agcggcaaga gcaagagcag cggcagacat 11100

gcagggcacc ctcccctcct cctaccgcgt caggaggggc gacatccgcg gttgacgcgg 11160

cagcagatgg tgattacgaa cccccgcggc gccgggcccg gcactacctg gacttggagg 11220

agggcgaggg cctggcgcgg ctaggagcgc cctctcctga gcggcaccca agggtgcagc 11280

tgaagcgtga tacgcgtgag gcgtacgtgc cgcggcagaa cctgtttcgc gaccgcgagg 11340

gagaggagcc cgaggagatg cgggatcgaa agttccacgc agggcgcgag ctgcggcatg 11400

gcctgaatcg cgagcggttg ctgcgcgagg aggactttga gcccgacgcg cgaaccggga 11460

ttagtcccgc gcgcgcacac gtggcggccg ccgacctggt aaccgcatac gagcagacgg 11520

tgaaccagga gattaacttt caaaaaagct ttaacaacca cgtgcgtacg cttgtggcgc 11580

gcgaggaggt ggctatagga ctgatgcatc tgtgggactt tgtaagcgcg ctggagcaaa 11640

acccaaatag caagccgctc atggcgcagc tgttccttat agtgcagcac agcagggaca 11700

acgaggcatt cagggatgcg ctgctaaaca tagtagagcc cgagggccgc tggctgctcg 11760

atttgataaa catcctgcag agcatagtgg tgcaggagcg cagcttgagc ctggctgaca 11820

aggtggccgc catcaactat tccatgctta gcctgggcaa gttttacgcc cgcaagatat 11880

accatacccc ttacgttccc atagacaagg aggtaaagat cgaggggttc tacatgcgca 11940

tggcgctgaa ggtgcttacc ttgagcgacg acctgggcgt ttatcgcaac gagcgcatcc 12000

acaaggccgt gagcgtgagc cggcggcgcg agctcagcga ccgcgagctg atgcacagcc 12060

tgcaaagggc cctggctggc acgggcagcg gcgatagaga ggccgagtcc tactttgacg 12120

cgggcgctga cctgcgctgg gccccaagcc gacgcgccct ggaggcagct ggggccggac 12180

ctgggctggc ggtggcaccc gcgcgcgctg gcaacgtcgg cggcgtggag gaatatgacg 12240

aggacgatga gtacgagcca gaggacggcg agtactaagc ggtgatgttt ctgatcagat 12300

gatgcaagac gcaacggacc cggcggtgcg ggcggcgctg cagagccagc cgtccggcct 12360

taactccacg gacgactggc gccaggtcat ggaccgcatc atgtcgctga ctgcgcgcaa 12420

tcctgacgcg ttccggcagc agccgcaggc caaccggctc tccgcaattc tggaagcggt 12480

ggtcccggcg cgcgcaaacc ccacgcacga gaaggtgctg gcgatcgtaa acgcgctggc 12540

cgaaaacagg gccatccggc ccgacgaggc cggcctggtc tacgacgcgc tgcttcagcg 12600

cgtggctcgt tacaacagcg gcaacgtgca gaccaacctg gaccggctgg tgggggatgt 12660

gcgcgaggcc gtggcgcagc gtgagcgcgc gcagcagcag ggcaacctgg gctccatggt 12720

tgcactaaac gccttcctga gtacacagcc cgccaacgtg ccgcggggac aggaggacta 12780

caccaacttt gtgagcgcac tgcggctaat ggtgactgag acaccgcaaa gtgaggtgta 12840

ccagtctggg ccagactatt ttttccagac cagtagacaa ggcctgcaga ccgtaaacct 12900

gagccaggct ttcaaaaact tgcaggggct gtggggggtg cgggctccca caggcgaccg 12960

cgcgaccgtg tctagcttgc tgacgcccaa ctcgcgcctg ttgctgctgc taatagcgcc 13020

cttcacggac agtggcagcg tgtcccggga cacataccta ggtcacttgc tgacactgta 13080

ccgcgaggcc ataggtcagg cgcatgtgga cgagcatact ttccaggaga ttacaagtgt 13140

cagccgcgcg ctggggcagg aggacacggg cagcctggag gcaaccctaa actacctgct 13200

gaccaaccgg cggcagaaga tcccctcgtt gcacagttta aacagcgagg aggagcgcat 13260

tttgcgctac gtgcagcaga gcgtgagcct taacctgatg cgcgacgggg taacgcccag 13320

cgtggcgctg gacatgaccg cgcgcaacat ggaaccgggc atgtatgcct caaaccggcc 13380

gtttatcaac cgcctaatgg actacttgca tcgcgcggcc gccgtgaacc ccgagtattt 13440

caccaatgcc atcttgaacc cgcactggct accgccccct ggtttctaca ccgggggatt 13500

cgaggtgccc gagggtaacg atggattcct ctgggacgac atagacgaca gcgtgttttc 13560

cccgcaaccg cagaccctgc tagagttgca acagcgcgag caggcagagg cggcgctgcg 13620

aaaggaaagc ttccgcaggc caagcagctt gtccgatcta ggcgctgcgg ccccgcggtc 13680

agatgctagt agcccatttc caagcttgat agggtctctt accagcactc gcaccacccg 13740

cccgcgcctg ctgggcgagg aggagtacct aaacaactcg ctgctgcagc cgcagcgcga 13800

aaaaaacctg cctccggcat ttcccaacaa cgggatagag agcctagtgg acaagatgag 13860

tagatggaag acgtacgcgc aggagcacag ggacgtgcca ggcccgcgcc cgcccacccg 13920

tcgtcaaagg cacgaccgtc agcggggtct ggtgtgggag gacgatgact cggcagacga 13980

cagcagcgtc ctggatttgg gagggagtgg caacccgttt gcgcaccttc gccccaggct 14040

ggggagaatg ttttaaaaaa aaaaaaagca tgatgcaaaa taaaaaactc accaaggcca 14100

tggcaccgag cgttggtttt cttgtattcc ccttagtatg cggcgcgcgg cgatgtatga 14160

ggaaggtcct cctccctcct acgagagtgt ggtgagcgcg gcgccagtgg cggcggcgct 14220

gggttctccc ttcgatgctc ccctggaccc gccgtttgtg cctccgcggt acctgcggcc 14280

taccgggggg agaaacagca tccgttactc tgagttggca cccctattcg acaccacccg 14340

tgtgtacctg gtggacaaca agtcaacgga tgtggcatcc ctgaactacc agaacgacca 14400

cagcaacttt ctgaccacgg tcattcaaaa caatgactac agcccggggg aggcaagcac 14460

acagaccatc aatcttgacg accggtcgca ctggggcggc gacctgaaaa ccatcctgca 14520
taccaacatg ccaaatgtga acgagttcat gtttaccaat aagtttaagg cgcgggtgat 14580
ggtgtcgcgc ttgcctacta aggacaatca ggtggagctg aaatacgagt gggtggagtt 14640
cacgctgccc gagggcaact actccgagac catgaccata gaccttatga acaacgcgat 14700
cgtggagcac tacttgaaag tgggcagaca gaacggggtt ctggaaagcg acatcggggt 14760
aaagtttgac acccgcaact tcagactggg gtttgacccc gtcactggtc ttgtcatgcc 14820
tggggtatat acaaacgaag ccttccatcc agacatcatt ttgctgccag gatgcggggt 14880
ggacttcacc cacagccgcc tgagcaactt gttgggcatc cgcaagcggc aacccttcca 14940
ggagggcttt aggatcacct acgatgatct ggagggtggt aacattcccg cactgttgga 15000
tgtggacgcc taccaggcga gcttgaaaga tgacaccgaa cagggcgggg gtggcgcagg 15060
cggcagcaac agcagtggca gcggcgcgga agagaactcc aacgcggcag ccgcggcaat 15120
gcagccggtg gaggacatga acgatcatgc cattcgcggc gacacctttg ccacacgggc 15180
tgaggagaag cgcgctgagg ccgaagcagc ggccgaagct gccgcccccg ctgcgcaacc 15240
cgaggtcgag aagcctcaga agaaaccggt gatcaaaccc ctgacagagg acagcaagaa 15300
acgcagttac aacctaataa gcaatgacag caccttcacc cagtaccgca gctggtacct 15360
tgcatacaac tacggcgacc ctcagaccgg aatccgctca tggaccctgc tttgcactcc 15420
tgacgtaacc tgcggctcgg agcaggtcta ctggtcgttg ccagacatga tgcaagaccc 15480
cgtgaccttc cgctccacgc gccagatcag caactttccg gtggtgggcg ccgagctgtt 15540
gcccgtgcac tccaagagct tctacaacga ccaggccgtc tactcccaac tcatccgcca 15600
gtttacctct ctgacccacg tgttcaatcg ctttcccgag aaccagattt tggcgcgccc 15660
gccagccccc accatcacca ccgtcagtga aaacgttcct gctctcacag atcacgggac 15720
gctaccgctg cgcaacagca tcggaggagt ccagcgagtg accattactg acgccagacg 15780
ccgcacctgc ccctacgttt acaaggccct gggcatagtc tcgccgcgcg tcctatcgag 15840
ccgcactttt tgagcaagca tgtccatcct tatatcgccc agcaataaca caggctgggg 15900
cctgcgcttc ccaagcaaga tgtttggcgg ggccaagaag cgctccgacc aacacccagt 15960
gcgcgtgcgc gggcactacc gcgcgccctg gggcgcgcac aaacgcggcc gcactgggcg 16020
caccaccgtc gatgacgcca tcgacgcggt ggtggaggag gcgcgcaact acacgcccac 16080
gccgccacca gtgtccacag tggacgcggc cattcagacc gtggtgcgcg gagcccggcg 16140
ctatgctaaa atgaagagac ggcggaggcg cgtagcacgt cgccaccgcc gccgacccgg 16200
cactgccgcc caacgcgcgg cggcggccct gcttaaccgc gcacgtcgca ccggccgacg 16260
ggcggccatg cgggccgctc gaaggctggc cgcgggtatt gtcactgtgc cccccaggtc 16320

caggcgacga gcggccgccg cagcagccgc ggccattagt gctatgactc agggtcgcag 16380

gggcaacgtg tattgggtgc gcgactcggt tagcggcctg cgcgtgcccg tgcgcacccg 16440

ccccccgcgc aactagattg caagaaaaaa ctacttagac tcgtactgtt gtatgtatcc 16500

agcggcggcg gcgcgcaacg aagctatgtc caagcgcaaa atcaaagaag agatgctcca 16560

ggtcatcgcg ccggagatct atggcccccc gaagaaggaa gagcaggatt acaagccccg 16620

aaagctaaag cgggtcaaaa agaaaaagaa agatgatgat gatgaacttg acgacgaggt 16680

ggaactgctg cacgctaccg cgcccaggcg acgggtacag tggaaaggtc gacgcgtaaa 16740

acgtgttttg cgacccggca ccaccgtagt ctttacgccc ggtgagcgct ccacccgcac 16800

ctacaagcgc gtgtatgatg aggtgtacgg cgacgaggac ctgcttgagc aggccaacga 16860

gcgcctcggg gagtttgcct acggaaagcg gcataaggac atgctggcgt tgccgctgga 16920

cgagggcaac ccaacaccta gcctaaagcc cgtaacactg cagcaggtgc tgcccgcgct 16980

tgcaccgtcc gaagaaaagc gcggcctaaa gcgcgagtct ggtgacttgg cacccaccgt 17040

gcagctgatg gtacccaagc gccagcgact ggaagatgtc ttggaaaaaa tgaccgtgga 17100

acctgggctg gagcccgagg tccgcgtgcg gccaatcaag caggtggcgc cgggactggg 17160

cgtgcagacc gtggacgttc agatacccac taccagtagc accagtattg ccaccgccac 17220

agagggcatg gagacacaaa cgtccccggt tgcctcagcg gtggcggatg ccgcggtgca 17280

ggcggtcgct gcggccgcgt ccaagacctc tacggaggtg caaacggacc cgtggatgtt 17340

tcgcgtttca gccccccggc gcccgcgccg ttcgaggaag tacggcgccg ccagcgcgct 17400

actgcccgaa tatgccctac atccttccat tgcgcctacc cccggctatc gtggctacac 17460

ctaccgcccc agaagacgag caactacccg acgccgaacc accactggaa cccgccgccg 17520

ccgtcgccgt cgccagcccg tgctggcccc gatttccgtg cgcagggtgg ctcgcgaagg 17580

aggcaggacc ctggtgctgc caacagcgcg ctaccacccc agcatcgttt aaaagccggt 17640

ctttgtggtt cttgcagata tggccctcac ctgccgcctc cgtttcccgg tgccgggatt 17700

ccgaggaaga atgcaccgta ggaggggcat ggccggccac ggcctgacgg gcggcatgcg 17760

tcgtgcgcac caccggcggc ggcgcgcgtc gcaccgtcgc atgcgcggcg gtatcctgcc 17820

cctccttatt ccactgatcg ccgcggcgat tggcgccgtg cccggaattg catccgtggc 17880

cttgcaggcg cagagacact gattaaaaac aagttgcatg tggaaaaatc aaaataaaaa 17940

gtctggactc tcacgctcgc ttggtcctgt aactattttg tagaatggaa gacatcaact 18000

ttgcgtctct ggccccgcga cacggctcgc gcccgttcat gggaaactgg caagatatcg 18060

gcaccagcaa tatgagcggt ggcgccttca gctggggctc gctgtggagc ggcattaaaa 18120

atttcggttc caccgttaag aactatggca gcaaggcctg gaacagcagc acaggccaga 18180

tgctgaggga taagttgaaa gagcaaaatt tccaacaaaa ggtggtagat ggcctggcct 18240

ctggcattag cggggtggtg gacctggcca accaggcagt gcaaaataag attaacagta 18300

agcttgatcc ccgccctccc gtagaggagc ctccaccggc cgtggagaca gtgtctccag 18360

aggggcgtgg cgaaaagcgt ccgcgccccg acagggaaga aactctggtg acgcaaatag 18420

acgagcctcc ctcgtacgag gaggcactaa agcaaggcct gcccaccacc cgtcccatcg 18480

cgcccatggc taccggagtg ctgggccagc acacacccgt aacgctggac ctgcctcccc 18540

ccgccgacac ccagcagaaa cctgtgctgc caggcccgac cgccgttgtt gtaacccgtc 18600

ctagccgcgc gtccctgcgc cgcgccgcca gcggtccgcg atcgttgcgg cccgtagcca 18660

gtggcaactg gcaaagcaca ctgaacagca tcgtgggtct gggggtgcaa tccctgaagc 18720

gccgacgatg cttctgatag ctaacgtgtc gtatgtgtgt catgtatgcg tccatgtcgc 18780

cgccagagga gctgctgagc cgccgcgcgc ccgctttcca agatggctac cccttcgatg 18840

atgccgcagt ggtcttacat gcacatctcg ggccaggacg cctcggagta cctgagcccc 18900

gggctggtgc agtttgcccg cgccaccgag acgtacttca gcctgaataa caagtttaga 18960

aaccccacgg tggcgcctac gcacgacgtg accacagacc ggtcccagcg tttgacgctg 19020

cggttcatcc ctgtggaccg tgaggatact gcgtactcgt acaaggcgcg gttcacccta 19080

gctgtgggtg ataaccgtgt gctggacatg gcttccacgt actttgacat ccgcggcgtg 19140

ctggacaggg gccctacttt taagccctac tctggcactg cctacaacgc cctggctccc 19200

aagggtgccc caaatccttg cgaatgggat gaagctgcta ctgctcttga aataaaccta 19260

gaagaagagg acgatgacaa cgaagacgaa gtagacgagc aagctgagca gcaaaaaact 19320

cacgtatttg ggcaggcgcc ttattctggt ataaatatta caaaggaggg tattcaaata 19380

ggtgtcgaag gtcaaacacc taaatatgcc gataaaacat ttcaacctga acctcaaata 19440

ggagaatctc agtggtacga aacagaaatt aatcatgcag ctgggagagt cctaaaaaag 19500

actaccccaa tgaaaccatg ttacggttca tatgcaaaac ccacaaatga aaatggaggg 19560

caaggcattc ttgtaaagca acaaaatgga aagctagaaa gtcaagtgga aatgcaattt 19620

ttctcaacta ctgaggcagc cgcaggcaat ggtgataact tgactcctaa agtggtattg 19680

tacagtgaag atgtagatat agaaacccca gacactcata tttcttacat gcccactatt 19740

aaggaaggta actcacgaga actaatgggc caacaatcta tgcccaacag gcctaattac 19800

attgctttta gggacaattt tattggtcta atgtattaca acagcacggg taatatgggt 19860

gttctggcgg gccaagcatc gcagttgaat gctgttgtag atttgcaaga cagaaacaca 19920

gagctttcat accagctttt gcttgattcc attggtgata gaaccaggta cttttctatg 19980

tggaatcagg ctgttgacag ctatgatcca gatgttagaa ttattgaaaa tcatggaact 20040

gaagatgaac ttccaaatta ctgctttcca ctgggaggtg tgattaatac agagactctt 20100

accaaggtaa aacctaaaac aggtcaggaa aatggatggg aaaaagatgc tacagaattt 20160

tcagataaaa atgaaataag agttggaaat aattttgcca tggaaatcaa tctaaatgcc 20220

aacctgtgga gaaatttcct gtactccaac atagcgctgt atttgcccga caagctaaag 20280

tacagtcctt ccaacgtaaa aatttctgat aacccaaaca cctacgacta catgaacaag 20340

cgagtggtgg ctcccgggct agtggactgc tacattaacc ttggagcacg ctggtccctt 20400

gactatatgg acaacgtcaa cccatttaac caccaccgca atgctggcct gcgctaccgc 20460

tcaatgttgc tgggcaatgg tcgctatgtg cccttccaca tccaggtgcc tcagaagttc 20520

tttgccatta aaaacctcct tctcctgccg ggctcataca cctacgagtg gaacttcagg 20580

aaggatgtta acatggttct gcagagctcc ctaggaaatg acctaagggt tgacggagcc 20640

agcattaagt ttgatagcat ttgcctttac gccaccttct tccccatggc ccacaacacc 20700

gcctccacgc ttgaggccat gcttagaaac gacaccaacg accagtcctt taacgactat 20760

ctctccgccg ccaacatgct ctaccctata cccgccaacg ctaccaacgt gcccatatcc 20820

atcccctccc gcaactgggc ggctttccgc ggctgggcct tcacgcgcct taagactaag 20880

gaaaccccat cactgggctc gggctacgac ccttattaca cctactctgg ctctataccc 20940

tacctagatg gaacctttta cctcaaccac acctttaaga aggtggccat tacctttgac 21000

tcttctgtca gctggcctgg caatgaccgc ctgcttaccc ccaacgagtt tgaaattaag 21060

cgctcagttg acggggaggg ttacaacgtt gcccagtgta acatgaccaa agactggttc 21120

ctggtacaaa tgctagctaa ctataacatt ggctaccagg gcttctatat cccagagagc 21180

tacaaggacc gcatgtactc cttctttaga aacttccagc ccatgagccg tcaggtggtg 21240

gatgatacta aatacaagga ctaccaacag gtgggcatcc tacaccaaca caacaactct 21300

ggatttgttg gctaccttgc ccccaccatg cgcgaaggac aggcctaccc tgctaacttc 21360

ccctatccgc ttataggcaa gaccgcagtt gacagcatta cccagaaaaa gtttctttgc 21420

gatcgcaccc tttggcgcat cccattctcc agtaacttta tgtccatggg cgcactcaca 21480

gacctgggcc aaaaccttct ctacgccaac tccgcccacg cgctagacat gacttttgag 21540

gtggatccca tggacgagcc cacccttctt tatgttttgt ttgaagtctt tgacgtggtc 21600

cgtgtgcacc agccgcaccg cggcgtcatc gaaaccgtgt acctgcgcac gcccttctcg 21660

gccggcaacg ccacaacata aagaagcaag caacatcaac aacagctgcc gccatgggct 21720

ccagtgagca ggaactgaaa gccattgtca aagatcttgg ttgtgggcca tatttttggg 21780

gcacctatga caagcgcttt ccaggctttg tttctccaca caagctcgcc tgcgccatag 21840

tcaatacggc cggtcgcgag actgggggcg tacactggat ggcctttgcc tggaacccgc 21900

actcaaaaac atgctacctc tttgagccct ttggcttttc tgaccagcga ctcaagcagg 21960

tttaccagtt tgagtacgag tcactcctgc gccgtagcgc cattgcttct tcccccgacc 22020

gctgtataac gctggaaaag tccacccaaa gcgtacaggg gcccaactcg gccgcctgtg 22080

gactattctg ctgcatgttt ctccacgcct ttgccaactg gccccaaact cccatggatc 22140

acaaccccac catgaacctt attaccgggg tacccaactc catgctcaac agtccccagg 22200

tacagcccac cctgcgtcgc aaccaggaac agctctacag cttcctggag cgccactcgc 22260

cctacttccg cagccacagt gcgcagatta ggagcgccac ttctttttgt cacttgaaaa 22320

acatgtaaaa ataatgtact agagacactt tcaataaagg caaatgcttt tatttgtaca 22380

ctctcgggtg attatttacc cccacccttg ccgtctgcgc cgtttaaaaa tcaaaggggt 22440

tctgccgcgc atcgctatgc gccactggca gggacacgtt gcgatactgg tgtttagtgc 22500

tccacttaaa ctcaggcaca accatccgcg gcagctcggt gaagttttca ctccacaggc 22560

tgcgcaccat caccaacgcg tttagcaggt cgggcgccga tatcttgaag tcgcagttgg 22620

ggcctccgcc ctgcgcgcgc gagttgcgat acacagggtt gcagcactgg aacactatca 22680

gcgccgggtg gtgcacgctg gccagcacgc tcttgtcgga gatcagatcc gcgtccaggt 22740

cctccgcgtt gctcagggcg aacggagtca actttggtag ctgccttccc aaaaagggcg 22800

cgtgcccagg ctttgagttg cactcgcacc gtagtggcat caaaaggtga ccgtgcccgg 22860

tctgggcgtt aggatacagc gcctgcataa aagccttgat ctgcttaaaa gccacctgag 22920

cctttgcgcc ttcagagaag aacatgccgc aagacttgcc ggaaaactga ttggccggac 22980

aggccgcgtc gtgcacgcag caccttgcgt cggtgttgga gatctgcacc acatttcggc 23040

cccaccggtt cttcacgatc ttggccttgc tagactgctc cttcagcgcg cgctgcccgt 23100

tttcgctcgt cacatccatt tcaatcacgt gctccttatt tatcataatg cttccgtgta 23160

gacacttaag ctcgccttcg atctcagcgc agcggtgcag ccacaacgcg cagcccgtgg 23220

gctcgtgatg cttgtaggtc acctctgcaa acgactgcag gtacgcctgc aggaatcgcc 23280

ccatcatcgt cacaaaggtc ttgttgctgg tgaaggtcag ctgcaacccg cggtgctcct 23340

cgttcagcca ggtcttgcat acggccgcca gagcttccac ttggtcaggc agtagtttga 23400

agttcgcctt tagatcgtta tccacgtggt acttgtccat cagcgcgcgc gcagcctcca 23460

tgcccttctc ccacgcagac acgatcggca cactcagcgg gttcatcacc gtaatttcac 23520

tttccgcttc gctgggctct tcctcttcct cttgcgtccg cataccacgc gccactgggt 23580

cgtcttcatt cagccgccgc actgtgcgct tacctccttt gccatgcttg attagcaccg 23640

gtgggttgct gaaacccacc atttgtagcg ccacatcttc tctttcttcc tcgctgtcca 23700

cgattacctc tggtgatggc gggcgctcgg gcttgggaga agggcgcttc tttttcttct 23760

tgggcgcaat ggccaaatcc gccgccgagg tcgatggccg cgggctgggt gtgcgcggca 23820

ccagcgcgtc ttgtgatgag tcttcctcgt cctcggactc gatacgccgc ctcatccgct 23880

tttttggggg cgcccgggga ggcggcggcg acggggacgg ggacgacacg tcctccatgg 23940

ttgggggacg tcgcgccgca ccgcgtccgc gctcgggggt ggtttcgcgc tgctcctctt 24000

cccgactggc catttccttc tcctataggc agaaaaagat catggagtca gtcgagaaga 24060

aggacagcct aaccgccccc tctgagttcg ccaccaccgc ctccaccgat gccgccaacg 24120

cgcctaccac cttccccgtc gaggcacccc cgcttgagga ggaggaagtg attatcgagc 24180

aggacccagg ttttgtaagc gaagacgacg aggaccgctc agtaccaaca gaggataaaa 24240

agcaagacca ggacaacgca gaggcaaacg aggaacaagt cgggcggggg gacgaaaggc 24300

atggcgacta cctagatgtg ggagacgacg tgctgttgaa gcatctgcag cgccagtgcg 24360

ccattatctg cgacgcgttg caagagcgca gcgatgtgcc cctcgccata gcggatgtca 24420

gccttgccta cgaacgccac ctattctcac cgcgcgtacc ccccaaacgc caagaaaacg 24480

gcacatgcga gcccaacccg cgcctcaact tctaccccgt atttgccgtg ccagaggtgc 24540

ttgccaccta tcacatcttt ttccaaaact gcaagatacc cctatcctgc cgtgccaacc 24600

gcagccgagc ggacaagcag ctggccttgc ggcagggcgc tgtcatacct gatatcgcct 24660

cgctcaacga agtgccaaaa atctttgagg gtcttggacg cgacgagaag cgcgcggcaa 24720

acgctctgca acaggaaaac agcgaaaatg aaagtcactc tggagtgttg gtggaactcg 24780

agggtgacaa cgcgcgccta gccgtactaa aacgcagcat cgaggtcacc cactttgcct 24840

acccggcact taacctaccc cccaaggtca tgagcacagt catgagtgag ctgatcgtgc 24900

gccgtgcgca gcccctggag agggatgcaa atttgcaaga acaaacagag gagggcctac 24960

ccgcagttgg cgacgagcag ctagcgcgct ggcttcaaac gcgcgagcct gccgacttgg 25020

aggagcgacg caaactaatg atggccgcag tgctcgttac cgtggagctt gagtgcatgc 25080

agcggttctt tgctgacccg gagatgcagc gcaagctaga ggaaacattg cactacacct 25140

ttcgacaggg ctacgtacgc caggcctgca agatctccaa cgtggagctc tgcaacctgg 25200

tctcctacct tggaattttg cacgaaaacc gccttgggca aaacgtgctt cattccacgc 25260

tcaagggcga ggcgcgccgc gactacgtcc gcgactgcgt ttacttattt ctatgctaca 25320

cctggcagac ggccatgggc gtttggcagc agtgcttgga ggagtgcaac ctcaaggagc 25380

tgcagaaact gctaaagcaa aacttgaagg acctatggac ggccttcaac gagcgctccg 25440

tggccgcgca cctggcggac atcattttcc ccgaacgcct gcttaaaacc ctgcaacagg 25500

gtctgccaga cttcaccagt caaagcatgt tgcagaactt taggaacttt atcctagagc 25560

gctcaggaat cttgcccgcc acctgctgtg cacttcctag cgactttgtg cccattaagt 25620

accgcgaatg ccctccgccg ctttggggcc actgctacct tctgcagcta gccaactacc 25680

ttgcctacca ctctgacata atggaagacg tgagcggtga cggtctactg gagtgtcact 25740

gtcgctgcaa cctatgcacc ccgcaccgct ccctggtttg caattcgcag ctgcttaacg 25800

aaagtcaaat tatcggtacc tttgagctgc agggtccctc gcctgacgaa aagtccgcgg 25860

ctccggggtt gaaactcact ccggggctgt ggacgtcggc ttaccttcgc aaatttgtac 25920

ctgaggacta ccacgcccac gagattaggt tctacgaaga ccaatcccgc ccgcctaatg 25980

cggagcttac cgcctgcgtc attacccagg gccacattct tggccaattg caagccatca 26040

acaaagcccg ccaagagttt ctgctacgaa agggacgggg ggtttacttg gacccccagt 26100

ccggcgagga gctcaaccca atccccccgc cgccgcagcc ctatcagcag cagccgcggg 26160

cccttgcttc ccaggatggc acccaaaaag aagctgcagc tgccgccgcc acccacggac 26220

gaggaggaat actgggacag tcaggcagag gaggttttgg acgaggagga ggaggacatg 26280

atggaagact gggagagcct agacgaggaa gcttccgagg tcgaagaggt gtcagacgaa 26340

acaccgtcac cctcggtcgc attcccctcg ccggcgcccc agaaatcggc aaccggttcc 26400

agcatggcta caacctccgc tcctcaggcg ccgccggcac tgcccgttcg ccgacccaac 26460

cgtagatggg acaccactgg aaccagggcc ggtaagtcca agcagccgcc gccgttagcc 26520

caagagcaac aacagcgcca aggctaccgc tcatggcgcg ggcacaagaa cgccatagtt 26580

gcttgcttgc aagactgtgg gggcaacatc tccttcgccc gccgctttct tctctaccat 26640

cacggcgtgg ccttcccccg taacatcctg cattactacc gtcatctcta cagcccatac 26700

tgcaccggcg gcagcggcag caacagcagc ggccacacag aagcaaaggc gaccggatag 26760

caagactctg acaaagccca agaaatccac agcggcggca gcagcaggag gaggagcgct 26820

gcgtctggcg cccaacgaac ccgtatcgac ccgcgagctt agaaacagga tttttcccac 26880

tctgtatgct atatttcaac agagcagggg ccaagaacaa gagctgaaaa taaaaaacag 26940

gtctctgcga tccctcaccc gcagctgcct gtatcacaaa agcgaagatc agcttcggcg 27000

cacgctggaa gacgcggagg ctctcttcag taaatactgc gcgctgactc ttaaggacta 27060

gtttcgcgcc ctttctcaaa tttaagcgcg aaaactacgt catctccagc ggccacaccc 27120

ggcgccagca cctgttgtca gcgccattat gagcaaggaa attcccacgc cctacatgtg 27180

gagttaccag ccacaaatgg gacttgcggc tggagctgcc caagactact caacccgaat 27240

aaactacatg agcgcgggac cccacatgat atcccgggtc aacggaatac gcgcccaccg 27300

aaaccgaatt ctcctggaac aggcggctat taccaccaca cctcgtaata accttaatcc 27360

ccgtagttgg cccgctgccc tggtgtacca ggaaagtccc gctcccacca ctgtggtact 27420

tcccagagac gcccaggccg aagttcagat gactaactca ggggcgcagc ttgcgggcgg 27480

ctttcgtcac agggtgcggt cgcccgggca gggtataact cacctgacaa tcagagggcg 27540

aggtattcag ctcaacgacg agtcggtgag ctcctcgctt ggtctccgtc cggacgggac 27600

atttcagatc ggcggcgccg gccgctcttc attcacgcct cgtcaggcaa tcctaactct 27660

gcagacctcg tcctctgagc cgcgctctgg aggcattgga actctgcaat ttattgagga 27720

gtttgtgcca tcggtctact ttaacccctt ctcgggacct cccggccact atccggatca 27780

atttattcct aactttgacg cggtaaagga ctcggcggac ggctacgact gaatgttaag 27840

tggagaggca gagcaactgc gcctgaaaca cctggtccac tgtcgccgcc acaagtgctt 27900

tgcccgcgac tccggtgagt tttgctactt tgaattgccc gaggatcata tcgagggccc 27960

ggcgcacggc gtccggctta ccgcccaggg agagcttgcc cgtagcctga ttcgggagtt 28020

tacccagcgc cccctgctag ttgagcggga caggggaccc tgtgttctca ctgtgatttg 28080

caactgtcct aaccctggat tacatcaaga tccagatctt ctagacccgg gagcggccgc 28140

tgtcgacctg caggatccga attcgatatc actagctagt tattaatagt aatcaattac 28200

ggggtcatta gttcatagcc catatatgga gttccgcgtt acataactta cggtaaatgg 28260

cccgcctggc tgaccgccca acgacccccg cccattgacg tcaataatga cgtatgttcc 28320

catagtaacg ccaataggga ctttccattg acgtcaatgg gtggagtatt tacggtaaac 28380

tgcccacttg gcagtacatc aagtgtatca tatgccaagt ccgcccccta ttgacgtcaa 28440

tgacggtaaa tggcccgcct ggcattatgc ccagtacatg accttacggg actttcctac 28500

ttggcagtac atctacgtat tagtcatcgc tattaccatg gtgatgcggt tttggcagta 28560

caccaatggg cgtggatagc ggtttgactc acggggattt ccaagtctcc accccattga 28620

cgtcaatggg agtttgtttt ggcaccaaaa tcaacgggac tttccaaaat gtcgtaatac 28680

ccccgccccg ttgacgcaaa tgggcaagct tgccgggtcg aggtaggcgt gtacggtggg 28740

aggcctatat aagcaaccgg tataatcaaa cagaccagat tgtctgtttg ttaccggtgt 28800

ttagtgaacc gggcgcgcct catatcgcct ggagacgcca tccacgctgt tttgacctcc 28860

atagaagaca ccgggaccga tccagcctcc gcgggccgcc accatgcggc tgggcagtcc 28920

tggactgctc ttcctgctct tcagcagcct tcgagctgat actcaggaga aggaagtcag 28980

agcgatggta ggcagcgacg tggagctcag ctgcgcttgc cctgaaggaa gccgttttga 29040

cttaaatgat gtttacgtat attggcaaac cagtgagtcg aaaaccgtgg tgacctacca 29100

catcccacag aacagctcct tggaaaacgt ggacagccgc taccggaacc gagccctgat 29160

gtcaccggcc ggcatgctgc ggggcgactt ctccctgcgc ttgttcaacg tcacccccca 29220

ggacgagcag aagtttcact gcctggtgtt gagccaatcc ctgggattcc aggaggtttt 29280

gagcgttgag gttacactgc atgtggcagc aaacttcagc gtgcccgtcg tcagcgcccc 29340

ccacagcccc tcccaggatg agctcacctt cacgtgtaca tccataaacg gctaccccag 29400

gcccaacgtg tactggatca ataagacgga caacagcctg ctggaccagg ctctgcagaa 29460

tgacaccgtc ttcttgaaca tgcggggctt gtatgacgtg gtcagcgtgc tgaggatcgc 29520

acggaccccc agcgtgaaca ttggctgctg catagagaac gtgcttctgc agcagaacct 29580

gactgtcggc agccagacag gaaatgacat cggagagaga gacaagatca cagagaatcc 29640

agtcagtacc ggcgagaaaa acgcggccac gtggagcatc ctggctgtcc tgtgcctgct 29700

tgtggtcgtg gcggtggcca taggctgggt gtgcagggac cgatgcctcc aacacagcta 29760

tgcaggtgcc tgggctgtga gtccggagac agagctcact ggccacgttt gacccctctc 29820

cctccccccc ctaacgttac tggccgaagc cgcttggaat aaggccggtg tgcgtttgtc 29880

tatatgttat tttccaccat attgccgtct tttggcaatg tgagggcccg gaaacctggc 29940

cctgtcttct tgacgagcat tcctaggggt ctttcccctc tcgccaaagg aatgcaaggt 30000

ctgttgaatg tcgtgaagga agcagttcct ctggaagctt cttgaagaca aacaacgtct 30060

gtagcgaccc tttgcaggca gcggaacccc ccacctggcg acaggtgcct ctgcggccaa 30120

aagccacgtg tataagatac acctgcaaag gcggcacaac cccagtgcca cgttgtgagt 30180

tggatagttg tggaaagagt caaatggctc tcctcaagcg tattcaacaa ggggctgaag 30240

gatgcccaga aggtacccca ttgtatggga tctgatctgg ggcctcggtg cacatgcttt 30300

acatgtgttt agtcgaggtt aaaaaaacgt ctaggccccc cgaaccacgg ggacgtggtt 30360

ttcctttgaa aaacacgatg ataagcttgc cacaacccgg gatcctctag agtcgacatg 30420

gatatagaag atgaagaaaa catgagttcc agcagcactg atgtgaagga aaaccgcaat 30480

ctggacaacg tgtcccccaa ggatggcagc acacctgggc ctggcgaggg ctctcagctc 30540

tccaatgggg gtggtggtgg ccccggcaga aagcggcccc tggaggaggg cagcaatggc 30600

cactccaagt accgcctgaa gaaaaggagg aaaacaccag ggcccgtcct ccccaagaac 30660

gccctgatgc agctgaatga gatcaagcct ggtttgcagt acacactcct gtcccagact 30720

gggcccgtgc acgcgccttt gtttgtcatg tctgtggagg tgaatggcca ggtttttgag 30780

ggctctggtc ccacaaagaa aaaggcaaaa ctccatgctg ctgagaaggc cttgaggtct 30840

ttcgttcagt ttcctaatgc ctctgaggcc cacctggcca tggggaggac cctgtctgtc 30900

aacacggact tcacatctga ccaggccgac ttccctgaca cgctcttcaa tggttttgaa 30960

actcctgaca aggcggagcc tcccttttac gtgggctcca atggggatga ctccttcagt 31020

tccagcgggg acctcagctt gtctgcttcc ccggtgcctg ccagcctagc ccagcctcct 31080

ctccctgtct taccaccatt cccacccccg agtgggaaga atcccgtgat gatcttgaac 31140

gaactgcgcc caggactcaa gtatgacttc ctctccgaga gcggggagag ccatgccaag 31200

agcttcgtca tgtctgtggt cgtggatggt cagttctttg aaggctcggg gagaaacaag 31260

aagcttgcca aggcccgggc tgcgcagtct gccctggccg ccatttttaa cttgcacttg 31320

gatcagacgc catctcgcca gcctattccc agtgagggtc ttcagctgca tttaccgcag 31380

gttttagctg acgctgtctc acgcctggtc ctgggtaagt ttggtgacct gaccgacaac 31440

ttctcctccc ctcacgctcg cagaaaagtg ctggctggag tcgtcatgac aacaggcaca 31500
gatgttaaag atgccaaggt gataagtgtt tctacaggaa caaaatgtat taatggtgaa 31560
tacatgagtg atcgtggcct tgcattaaat gactgccatg cagaaataat atctcggaga 31620
tccttgctca gatttcttta tacacaactt gagctttact taaataacaa agatgatcaa 31680
aaaagatcca tctttcagaa atcagagcga gggggtttta ggctgaagga gaatgtccag 31740
tttcatctgt acatcagcac ctctccctgt ggagatgcca gaatcttctc accacatgag 31800
ccaatcctgg aagaaccagc agatagacac ccaaatcgta aagcaagagg acagctacgg 31860
accaaaatag agtctggtga ggggacgatt ccagtgcgct ccaatgcgag catccaaacg 31920
tgggacgggg tgctgcaagg ggagcggctg ctcaccatgt cctgcagtga caagattgca 31980
cgctggaacg tggtgggcat ccagggatcc ctgctcagca ttttcgtgga gcccatttac 32040
ttctcgagca tcatcctggg cagcctttac cacggggacc acctttccag ggccatgtac 32100
cagcggatct ccaacataga ggacctgcca cctctctaca ccctcaacaa gcctttgctc 32160
agtggcatca gcaatgcaga agcacggcag ccagggaagg cccccaactt cagtgtcaac 32220
tggacggtag gcgactccgc tattgaggtc atcaacgcca cgactgggaa ggatgagctg 32280
ggccgcgcgt cccgcctgtg taagcacgcg ttgtactgtc gctggatgcg tgtgcacggc 32340
aaggttccct cccacttact acgctccaag attaccaagc ccaacgtgta ccatgagtcc 32400
aagctggcgg caaaggagta ccaggccgcc aaggcgcgtc tgttcacagc cttcatcaag 32460
gcggggctgg gggcctgggt ggagaagccc accgagcagg accagttctc actcacgccc 32520
tgaaacttgt ttattgcagc ttataatggt tacaaataaa gcaatagcat cacaaatttc 32580
acaaataaag catttttttc actgcattct agttgtggtt tgtccaaact catcaatgta 32640
tcttaactag ctagtggtac cgatcttatt ccctttaact aataaaaaaa aataataaag 32700
catcacttac ttaaaatcag ttagcaaatt tctgtccagt ttattcagca gcacctcctt 32760
gccctcctcc cagctctggt attgcagctt cctcctggct gcaaactttc tccacaatct 32820
aaatggaatg tcagtttcct cctgttcctg tccatccgca cccactatct tcatgttgtt 32880
gcagatgaag cgcgcaagac cgtctgaaga taccttcaac cccgtgtatc catatgacac 32940
ggaaaccggt cctccaactg tgccttttct tactcctccc tttgtatccc ccaatgggtt 33000
tcaagagagt ccccctgggg tactctcttt gcgcctatcc gaacctctag ttacctccaa 33060
tggcatgctt gcgctcaaaa tgggcaacgg cctctctctg gacgaggccg gcaaccttac 33120
ctcccaaaat gtaaccactg tgagcccacc tctcaaaaaa accaagtcaa acataaacct 33180
ggaaatatct gcacccctca cagttacctc agaagcccta actgtggctg ccgccgcacc 33240
tctaatggtc gcgggcaaca cactcaccat gcaatcacag gccccgctaa ccgtgcacga 33300
ctccaaactt agcattgcca cccaaggacc cctcacagtg tcagaaggaa agctagccct 33360

gcaaacatca ggccccctca ccaccaccga tagcagtacc cttactatca ctgcctcacc 33420

ccctctaact actgccactg gtagcttggg cattgacttg aaagagccca tttatacaca 33480

aaatggaaaa ctaggactaa agtacggggc tcctttgcat gtaacagacg acctaaacac 33540

tttgaccgta gcaactggtc caggtgtgac tattaataat acttccttgc aaactaaagt 33600

tactggagcc ttgggttttg attcacaagg caatatgcaa cttaatgtag caggaggact 33660

aaggattgat tctcaaaaca gacgccttat acttgatgtt agttatccgt ttgatgctca 33720

aaaccaacta aatctaagac taggacaggg ccctcttttt ataaactcag cccacaactt 33780

ggatattaac tacaacaaag gcctttactt gtttacagct tcaaacaatt ccaaaaagct 33840

tgaggttaac ctaagcactg ccaaggggtt gatgtttgac gctacagcca tagccattaa 33900

tgcaggagat gggcttgaat ttggttcacc taatgcacca aacacaaatc ccctcaaaac 33960

aaaaattggc catggcctag aatttgattc aaacaaggct atggttccta aactaggaac 34020

tggccttagt tttgacagca caggtgccat tacagtagga aacaaaaata atgataagct 34080

aaccctatgg acaggtccaa aaccagaagc caactgcata attgaatacg ggaaacaaaa 34140

cccagatagc aaactaactt taatccttgt aaaaaatgga ggaattgtta atggatatgt 34200

aacgctaatg ggagcctcag actacgttaa caccttattt aaaaacaaaa atgtctccat 34260

taatgtagaa ctatactttg atgccactgg tcatatatta ccagactcat cttctcttaa 34320

aacagatcta gaactaaaat acaagcaaac cgctgacttt agtgcaagag gttttatgcc 34380

aagtactaca gcgtatccat ttgtccttcc taatgcggga acacataatg aaaattatat 34440

ttttggtcaa tgctactaca aagcaagcga tggtgccctt tttccgttgg aagttactgt 34500

tatgcttaat aaacgcctgc cagatagtcg cacatcctat gttatgactt ttttatggtc 34560

cttgaatgct ggtctagctc cagaaactac tcaggcaacc ctcataacct ccccatttac 34620

cttttcctat attagagaag atgactaata aactctaaag aatcgtttgt gttatgtttc 34680

aacgtgttta tttttcaatt gcagaaaatt tcaagtcatt tttcattcag tagtatagcc 34740

ccaccaccac atagcttata cagatcaccg taccttaatc aaactcacag aaccctagta 34800

ttcaacctgc cacctccctc ccaacacaca gagtacacag tcctttctcc ccggctggcc 34860

ttaaaaagca tcatatcatg ggtaacagac atattcttag gtgttatatt ccacacggtt 34920

tcctgtcgag ccaaacgctc atcagtgata ttaataaact ccccgggcag ctcacttaag 34980

ttcatgtcgc tgtccagctg ctgagccaca ggctgctgtc caacttgcgg ttgcttaacg 35040

ggcggcgaag gagaagtcca cgcctacatg ggggtagagt cataatcgtg catcaggata 35100

gggcggtggt gctgcagcag cgcgcgaata aactgctgcc gccgccgctc cgtcctgcag 35160

gaatacaaca tggcagtggt ctcctcagcg atgattcgca ccgcccgcag cataaggcgc 35220

cttgtcctcc gggcacagca gcgcaccctg atctcactta aatcagcaca gtaactgcag 35280

cacagcacca caatattgtt caaaatccca cagtgcaagg cgctgtatcc aaagctcatg 35340

gcggggacca cagaacccac gtggccatca taccacaagc gcaggtagat taagtggcga 35400

cccctcataa acacgctgga cataaacatt acctcttttg gcatgttgta attcaccacc 35460

tcccggtacc atataaacct ctgattaaac atggcgccat ccaccaccat cctaaaccag 35520

ctggccaaaa cctgcccgcc ggctatacac tgcagggaac cgggactgga acaatgacag 35580

tggagagccc aggactcgta accatggatc atcatgctcg tcatgatatc aatgttggca 35640

caacacaggc acacgtgcat acacttcctc aggattacaa gctcctcccg cgttagaacc 35700

atatcccagg gaacaaccca ttcctgaatc agcgtaaatc ccacactgca gggaagacct 35760

cgcacgtaac tcacgttgtg cattgtcaaa gtgttacatt cgggcagcag cggatgatcc 35820

tccagtatgg tagcgcgggt ttctgtctca aaaggaggta gacgatccct actgtacgga 35880

gtgcgccgag acaaccgaga tcgtgttggt cgtagtgtca tgccaaatgg aacgccggac 35940

gtagtcatat ttcctgaagc aaaaccaggt gcgggcgtga caaacagatc tgcgtctccg 36000

gtctcgccgc ttagatcgct ctgtgtagta gttgtagtat atccactctc tcaaagcatc 36060

caggcgcccc ctggcttcgg gttctatgta aactccttca tgcgccgctg ccctgataac 36120

atccaccacc gcagaataag ccacacccag ccaacctaca cattcgttct gcgagtcaca 36180

cacgggagga gcgggaagag ctggaagaac catgtttttt tttttattcc aaaagattat 36240

ccaaaacctc aaaatgaaga tctattaagt gaacgcgctc ccctccggtg gcgtggtcaa 36300

actctacagc caaagaacag ataatggcat ttgtaagatg ttgcacaatg gcttccaaaa 36360

ggcaaacggc cctcacgtcc aagtggacgt aaaggctaaa cccttcaggg tgaatctcct 36420

ctataaacat tccagcacct tcaaccatgc ccaaataatt ctcatctcgc caccttctca 36480

atatatctct aagcaaatcc cgaatattaa gtccggccat tgtaaaaatc tgctccagag 36540

cgccctccac cttcagcctc aagcagcgaa tcatgattgc aaaaattcag gttcctcaca 36600

gacctgtata agattcaaaa gcggaacatt aacaaaaata ccgcgatccc gtaggtccct 36660

tcgcagggcc agctgaacat aatcgtgcag gtctgcacgg accagcgcgg ccacttcccc 36720

gccaggaacc atgacaaaag aacccacact gattatgaca cgcatactcg gagctatgct 36780

aaccagcgta gccccgatgt aagcttgttg catgggcggc gatataaaat gcaaggtgct 36840

gctcaaaaaa tcaggcaaag cctcgcgcaa aaaagaaagc acatcgtagt catgctcatg 36900

cagataaagg caggtaagct ccggaaccac cacagaaaaa gacaccattt ttctctcaaa 36960

catgtctgcg ggtttctgca taaacacaaa ataaaataac aaaaaaacat ttaaacatta 37020

gaagcctgtc ttacaacagg aaaaacaacc cttataagca taagacggac tacggccatg 37080

ccggcgtgac cgtaaaaaaa ctggtcaccg tgattaaaaa gcaccaccga cagctcctcg 37140

gtcatgtccg gagtcataat gtaagactcg gtaaacacat caggttgatt cacatcggtc 37200

agtgctaaaa agcgaccgaa atagcccggg ggaatacata cccgcaggcg tagagacaac 37260

attacagccc ccataggagg tataacaaaa ttaataggag agaaaaacac ataaacacct 37320

gaaaaaccct cctgcctagg caaaatagca ccctcccgct ccagaacaac atacagcgct 37380

tccacagcgg cagccataac agtcagcctt accagtaaaa aagaaaacct attaaaaaaa 37440

caccactcga cacggcacca gctcaatcag tcacagtgta aaaaagggcc aagtgcagag 37500

cgagtatata taggactaaa aaatgacgta acggttaaag tccacaaaaa acacccagaa 37560

aaccgcacgc gaacctacgc ccagaaacga aagccaaaaa acccacaact tcctcaaatc 37620

gtcacttccg ttttcccacg ttacgtcact tcccatttta agaaaactac aattcccaac 37680

acatacaagt tactccgccc taaaacctac gtcacccgcc ccgttcccac gccccgcgcc 37740

acgtcacaaa ctccaccccc tcattatcat attggcttca atccaaaata aggtatatta 37800

ttgatgatgt taat 37814

<210> 2
<211> 35896
<212> DNA
<213> Artificial Sequence

<220>
<223> AdV5/3-D24-ICOSL (TGX-04)

<400> 2
attaacatca tcaataatat accttatttt ggattgaagc caatatgata atgagggggt 60

ggagtttgtg acgtggcgcg gggcgtggga acggggcggg tgacgtagta gtgtggcgga 120

agtgtgatgt tgcaagtgtg gcggaacaca tgtaagcgac ggatgtggca aaagtgacgt 180

ttttggtgtg cgccggtgta cacaggaagt gacaattttc gcgcggtttt aggcggatgt 240

tgtagtaaat ttgggcgtaa ccgagtaaga tttggccatt ttcgcgggaa aactgaataa 300

gaggaagtga aatctgaata attttgtgtt actcatagcg cgtaatattt gtctagggcc 360

gcggggactt tgaccgttta cgtggagact cgcccaggtg tttttctcag gtgttttccg 420

cgttccgggt caaagttggc gttttattat tatagtcagc tgacgtgtag tgtatttata 480

cccggtgagt tcctcaagag gccactcttg agtgccagcg agtagagttt tctcctccga 540

gccgctccga caccgggact gaaaatgaga catattatct gccacggagg tgttattacc 600

gaagaaatgg ccgccagtct tttggaccag ctgatcgaag aggtactggc tgataatctt 660

ccacctccta gccattttga accacctacc cttcacgaac tgtatgattt agacgtgacg 720

gcccccgaag atcccaacga ggaggcggtt tcgcagattt ttcccgactc tgtaatgttg 780

gcggtgcagg aagggattga cttactcact tttccgccgg cgcccggttc tccggagccg 840

cctcaccttt cccggcagcc cgagcagccg gagcagagag ccttgggtcc ggtttctatg 900
ccaaaccttg taccggaggt gatcgatcca cccagtgacg acgaggatga agagggtgag 960
gagtttgtgt tagattatgt ggagcacccc gggcacggtt gcaggtcttg tcattatcac 1020
cggaggaata cgggggaccc agatattatg tgttcgcttt gctatatgag gacctgtggc 1080
atgtttgtct acagtaagtg aaaattatgg gcagtgggtg atagagtggt gggtttggtg 1140
tggtaatttt ttttttaatt tttacagttt tgtggtttaa agaattttgt attgtgattt 1200
ttttaaaagg tcctgtgtct gaacctgagc ctgagcccga gccagaaccg gagcctgcaa 1260
gacctacccg ccgtcctaaa atggcgcctg ctatcctgag acgcccgaca tcacctgtgt 1320
ctagagaatg caatagtagt acggatagct gtgactccgg tccttctaac acacctcctg 1380
agatacaccc ggtggtcccg ctgtgcccca ttaaaccagt tgccgtgaga gttggtgggc 1440
gtcgccaggc tgtggaatgt atcgaggact tgcttaacga gcctgggcaa cctttggact 1500
tgagctgtaa acgccccagg ccataaggtg taaacctgtg attgcgtgtg tggttaacgc 1560
ctttgtttgc tgaatgagtt gatgtaagtt taataaaggg tgagataatg tttaacttgc 1620
atggcgtgtt aaatggggcg gggcttaaag ggtatataat gcgccgtggg ctaatcttgg 1680
ttacatctga cctcatggag gcttgggagt gtttggaaga tttttctgct gtgcgtaact 1740
tgctggaaca gagctctaac agtacctctt ggttttggag gtttctgtgg ggctcatccc 1800
aggcaaagtt agtctgcaga attaaggagg attacaagtg ggaatttgaa gagcttttga 1860
aatcctgtgg tgagctgttt gattctttga atctgggtca ccaggcgctt ttccaagaga 1920
aggtcatcaa gactttggat ttttccacac cggggcgcgc tgcggctgct gttgcttttt 1980
tgagttttat aaaggataaa tggagcgaag aaacccatct gagcgggggg tacctgctgg 2040
attttctggc catgcatctg tggagagcgg ttgtgagaca caagaatcgc ctgctactgt 2100
tgtcttccgt ccgcccggcg ataataccga cggaggagca gcagcagcag caggaggaag 2160
ccaggcggcg gcggcaggag cagagcccat ggaacccgag agccggcctg gaccctcggg 2220
aatgaatgtt gtacaggtgg ctgaactgta tccagaactg agacgcattt tgacaattac 2280
agaggatggg caggggctaa agggggtaaa gagggagcgg ggggcttgtg aggctacaga 2340
ggaggctagg aatctagctt ttagcttaat gaccagacac cgtcctgagt gtattacttt 2400
tcaacagatc aaggataatt gcgctaatga gcttgatctg ctggcgcaga agtattccat 2460
agagcagctg accacttact ggctgcagcc aggggatgat tttgaggagg ctattagggt 2520
atatgcaaag gtggcactta ggccagattg caagtacaag atcagcaaac ttgtaaatat 2580
caggaattgt tgctacattt ctgggaacgg ggccgaggtg gagatagata cggaggatag 2640
ggtggccttt agatgtagca tgataaatat gtggccgggg gtgcttggca tggacggggt 2700

ggttattatg aatgtaaggt ttactggccc caattttagc ggtacggttt tcctggccaa 2760

taccaacctt atcctacacg gtgtaagctt ctatgggttt aacaatacct gtgtggaagc 2820

ctggaccgat gtaagggttc ggggctgtgc cttttactgc tgctggaagg gggtggtgtg 2880

tcgccccaaa agcagggctt caattaagaa atgcctcttt gaaaggtgta ccttgggtat 2940

cctgtctgag ggtaactcca gggtgcgcca caatgtggcc tccgactgtg gttgcttcat 3000

gctagtgaaa agcgtggctg tgattaagca taacatggta tgtggcaact gcgaggacag 3060

ggcctctcag atgctgacct gctcggacgg caactgtcac ctgctgaaga ccattcacgt 3120

agccagccac tctcgcaagg cctggccagt gtttgagcat aacatactga cccgctgttc 3180

cttgcatttg ggtaacagga ggggggtgtt cctaccttac caatgcaatt tgagtcacac 3240

taagatattg cttgagcccg agagcatgtc caaggtgaac ctgaacgggg tgtttgacat 3300

gaccatgaag atctggaagg tgctgaggta cgatgagacc cgcaccaggt gcagaccctg 3360

cgagtgtggc ggtaaacata ttaggaacca gcctgtgatg ctggatgtga ccgaggagct 3420

gaggcccgat cacttggtgc tggcctgcac ccgcgctgag tttggctcta gcgatgaaga 3480

tacagattga ggtactgaaa tgtgtgggcg tggcttaagg gtgggaaaga atatataagg 3540

tgggggtctt atgtagtttt gtatctgttt tgcagcagcc gccgccgcca tgagcaccaa 3600

ctcgtttgat ggaagcattg tgagctcata tttgacaacg cgcatgcccc catgggccgg 3660

ggtgcgtcag aatgtgatgg gctccagcat tgatggtcgc cccgtcctgc ccgcaaactc 3720

tactaccttg acctacgaga ccgtgtctgg aacgccgttg gagactgcag cctccgccgc 3780

cgcttcagcc gctgcagcca ccgcccgcgg gattgtgact gactttgctt tcctgagccc 3840

gcttgcaagc agtgcagctt cccgttcatc cgcccgcgat gacaagttga cggctctttt 3900

ggcacaattg gattctttga cccgggaact taatgtcgtt tctcagcagc tgttggatct 3960

gcgccagcag gtttctgccc tgaaggcttc ctcccctccc aatgcggttt aaaacataaa 4020

taaaaaacca gactctgttt ggatttggat caagcaagtg tcttgctgtc tttatttagg 4080

ggttttgcgc gcgcggtagg cccgggacca gcggtctcgg tcgttgaggg tcctgtgtat 4140

tttttccagg acgtggtaaa ggtgactctg gatgttcaga tacatgggca taagcccgtc 4200

tctggggtgg aggtagcacc actgcagagc ttcatgctgc ggggtggtgt tgtagatgat 4260

ccagtcgtag caggagcgct gggcgtggtg cctaaaaatg tctttcagta gcaagctgat 4320

tgccaggggc aggcccttgg tgtaagtgtt tacaaagcgg ttaagctggg atgggtgcat 4380

acgtggggat atgagatgca tcttggactg tatttttagg ttggctatgt tcccagccat 4440

atccctccgg ggattcatgt tgtgcagaac caccagcaca gtgtatccgg tgcacttggg 4500

aaatttgtca tgtagcttag aaggaaatgc gtggaagaac ttggagacgc ccttgtgacc 4560

tccaagattt tccatgcatt cgtccataat gatggcaatg ggcccacggg cggcggcctg 4620

ggcgaagata tttctgggat cactaacgtc atagttgtgt tccaggatga gatcgtcata 4680

ggccattttt acaaagcgcg ggcggagggt gccagactgc ggtataatgg ttccatccgg 4740

cccaggggcg tagttaccct cacagatttg catttcccac gctttgagtt cagatggggg 4800

gatcatgtct acctgcgggg cgatgaagaa aacggtttcc ggggtagggg agatcagctg 4860

ggaagaaagc aggttcctga gcagctgcga cttaccgcag ccggtgggcc cgtaaatcac 4920

acctattacc ggctgcaact ggtagttaag agagctgcag ctgccgtcat ccctgagcag 4980

gggggccact tcgttaagca tgtccctgac tcgcatgttt tccctgacca aatccgccag 5040

aaggcgctcg ccgcccagcg atagcagttc ttgcaaggaa gcaaagtttt tcaacggttt 5100

gagaccgtcc gccgtaggca tgcttttgag cgtttgacca agcagttcca ggcggtccca 5160

cagctcggtc acctgctcta cggcatctcg atccagcata tctcctcgtt tcgcgggttg 5220

gggcggcttt cgctgtacgg cagtagtcgg tgctcgtcca gacgggccag ggtcatgtct 5280

ttccacgggc gcagggtcct cgtcagcgta gtctgggtca cggtgaaggg gtgcgctccg 5340

ggctgcgcgc tggccagggt gcgcttgagg ctggtcctgc tggtgctgaa gcgctgccgg 5400

tcttcgccct gcgcgtcggc caggtagcat ttgaccatgg tgtcatagtc cagcccctcc 5460

gcggcgtggc ccttggcgcg cagcttgccc ttggaggagg cgccgcacga ggggcagtgc 5520

agacttttga gggcgtagag cttgggcgcg agaaataccg attccgggga gtaggcatcc 5580

gcgccgcagg ccccgcagac ggtctcgcat tccacgagcc aggtgagctc tggccgttcg 5640

gggtcaaaaa ccaggtttcc cccatgcttt ttgatgcgtt tcttacctct ggtttccatg 5700

agccggtgtc cacgctcggt gacgaaaagg ctgtccgtgt ccccgtatac agacttgaga 5760

ggcctgtcct cgagcggtgt tccgcggtcc tcctcgtata gaaactcgga ccactctgag 5820

acaaaggctc gcgtccaggc cagcacgaag gaggctaagt gggaggggta gcggtcgttg 5880

tccactaggg ggtccactcg ctccagggtg tgaagacaca tgtcgccctc ttcggcatca 5940

aggaaggtga ttggtttgta ggtgtaggcc acgtgaccgg gtgttcctga aggggggcta 6000

taaaaggggg tgggggcgcg ttcgtcctca ctctcttccg catcgctgtc tgcgagggcc 6060

agctgttggg gtgagtactc cctctgaaaa gcgggcatga cttctgcgct aagattgtca 6120

gtttccaaaa acgaggagga tttgatattc acctggcccg cggtgatgcc tttgagggtg 6180

gccgcatcca tctggtcaga aaagacaatc tttttgttgt caagcttggt ggcaaacgac 6240

ccgtagaggg cgttggacag caacttggcg atggagcgca gggtttggtt tttgtcgcga 6300

tcggcgcgct ccttggccgc gatgtttagc tgcacgtatt cgcgcgcaac gcaccgccat 6360

tcgggaaaga cggtggtgcg ctcgtcgggc accaggtgca cgcgccaacc gcggttgtgc 6420

agggtgacaa ggtcaacgct ggtggctacc tctccgcgta ggcgctcgtt ggtccagcag 6480

aggcggccgc ccttgcgcga gcagaatggc ggtagggggt ctagctgcgt ctcgtccggg 6540

gggtctgcgt ccacggtaaa gaccccgggc agcaggcgcg cgtcgaagta gtctatcttg 6600

catccttgca agtctagcgc ctgctgccat gcgcgggcgg caagcgcgcg ctcgtatggg 6660

ttgagtgggg gaccccatgg catggggtgg gtgagcgcgg aggcgtacat gccgcaaatg 6720

tcgtaaacgt agaggggctc tctgagtatt ccaagatatg tagggtagca tcttccaccg 6780

cggatgctgg cgcgcacgta atcgtatagt tcgtgcgagg gagcgaggag gtcgggaccg 6840

aggttgctac gggcgggctg ctctgctcgg aagactatct gcctgaagat ggcatgtgag 6900

ttggatgata tggttggacg ctggaagacg ttgaagctgg cgtctgtgag acctaccgcg 6960

tcacgcacga aggaggcgta ggagtcgcgc agcttgttga ccagctcggc ggtgacctgc 7020

acgtctaggg cgcagtagtc cagggtttcc ttgatgatgt catacttatc ctgtcccttt 7080

tttttccaca gctcgcggtt gaggacaaac tcttcgcggt ctttccagta ctcttggatc 7140

ggaaacccgt cggcctccga acggtaagag cctagcatgt agaactggtt gacggcctgg 7200

taggcgcagc atcccttttc tacgggtagc gcgtatgcct gcgcggcctt ccggagcgag 7260

gtgtgggtga gcgcaaaggt gtccctgacc atgactttga ggtactggta tttgaagtca 7320

gtgtcgtcgc atccgccctg ctcccagagc aaaaagtccg tgcgcttttt ggaacgcgga 7380

tttggcaggg cgaaggtgac atcgttgaag agtatctttc ccgcgcgagg cataaagttg 7440

cgtgtgatgc ggaagggtcc cggcacctcg gaacggttgt taattacctg ggcggcgagc 7500

acgatctcgt caaagccgtt gatgttgtgg cccacaatgt aaagttccaa gaagcgcggg 7560

atgcccttga tggaaggcaa ttttttaagt tcctcgtagg tgagctcttc aggggagctg 7620

agcccgtgct ctgaaagggc ccagtctgca agatgagggt tggaagcgac gaatgagctc 7680

cacaggtcac gggccattag catttgcagg tggtcgcgaa aggtcctaaa ctggcgacct 7740

atggccattt tttctggggt gatgcagtag aaggtaagcg ggtcttgttc ccagcggtcc 7800

catccaaggt tcgcggctag gtctcgcgcg gcagtcacta gaggctcatc tccgccgaac 7860

ttcatgacca gcatgaaggg cacgagctgc ttcccaaagg cccccatcca agtataggtc 7920

tctacatcgt aggtgacaaa gagacgctcg gtgcgaggat gcgagccgat cgggaagaac 7980

tggatctccc gccaccaatt ggaggagtgg ctattgatgt ggtgaaagta gaagtccctg 8040

cgacgggccg aacactcgtg ctggcttttg taaaaacgtg cgcagtactg gcagcggtgc 8100

acgggctgta catcctgcac gaggttgacc tgacgaccgc gcacaaggaa gcagagtggg 8160

aatttgagcc cctcgcctgg cgggtttggc tggtggtctt ctacttcggc tgcttgtcct 8220

tgaccgtctg gctgctcgag gggagttacg gtggatcgga ccaccacgcc gcgcgagccc 8280

aaagtccaga tgtccgcgcg cggcggtcgg agcttgatga caacatcgcg cagatgggag 8340

ctgtccatgg tctggagctc ccgcggcgtc aggtcaggcg ggagctcctg caggtttacc 8400

tcgcatagac gggtcagggc gcgggctaga tccaggtgat acctaatttc caggggctgg 8460

ttggtggcgg cgtcgatggc ttgcaagagg ccgcatcccc gcggcgcgac tacggtaccg 8520

cgcggcgggc ggtgggccgc gggggtgtcc ttggatgatg catctaaaag cggtgacgcg 8580

ggcgagcccc cggaggtagg gggggctccg gacccgccgg gagagggggc aggggcacgt 8640

cggcgccgcg cgcgggcagg agctggtgct gcgcgcgtag gttgctggcg aacgcgacga 8700

cgcggcggtt gatctcctga atctggcgcc tctgcgtgaa gacgacgggc ccggtgagct 8760

tgaacctgaa agagagttcg acagaatcaa tttcggtgtc gttgacggcg gcctggcgca 8820

aaatctcctg cacgtctcct gagttgtctt gataggcgat ctcggccatg aactgctcga 8880

tctcttcctc ctggagatct ccgcgtccgg ctcgctccac ggtggcggcg aggtcgttgg 8940

aaatgcgggc catgagctgc gagaaggcgt tgaggcctcc ctcgttccag acgcggctgt 9000

agaccacgcc cccttcggca tcgcgggcgc gcatgaccac ctgcgcgaga ttgagctcca 9060

cgtgccgggc gaagacggcg tagtttcgca ggcgctgaaa gaggtagttg agggtggtgg 9120

cggtgtgttc tgccacgaag aagtacataa cccagcgtcg caacgtggat tcgttgatat 9180

cccccaaggc ctcaaggcgc tccatggcct cgtagaagtc cacggcgaag ttgaaaaact 9240

gggagttgcg cgccgacacg gttaactcct cctccagaag acggatgagc tcggcgacag 9300

tgtcgcgcac ctcgcgctca aaggctacag gggcctcttc ttcttcttca atctcctctt 9360

ccataagggc ctccccttct tcttcttctg gcggcggtgg gggagggggg acacggcggc 9420

gacgacggcg caccgggagg cggtcgacaa agcgctcgat catctccccg cggcgacggc 9480

gcatggtctc ggtgacggcg cggccgttct cgcgggggcg cagttggaag acgccgcccg 9540

tcatgtcccg gttatgggtt ggcggggggc tgccatgcgg cagggatacg gcgctaacga 9600

tgcatctcaa caattgttgt gtaggtactc cgccgccgag ggacctgagc gagtccgcat 9660

cgaccggatc ggaaaacctc tcgagaaagg cgtctaacca gtcacagtcg caaggtaggc 9720

tgagcaccgt ggcgggcggc agcgggcggc ggtcggggtt gtttctggcg gaggtgctgc 9780

tgatgatgta attaaagtag gcggtcttga gacggcggat ggtcgacaga agcaccatgt 9840

ccttgggtcc ggcctgctga atgcgcaggc ggtcggccat gccccaggct tcgttttgac 9900

atcggcgcag gtctttgtag tagtcttgca tgagcctttc taccggcact tcttcttctc 9960

cttcctcttg tcctgcatct cttgcatcta tcgctgcggc ggcggcggag tttggccgta 10020

ggtggcgccc tcttcctccc atgcgtgtga ccccgaagcc cctcatcggc tgaagcaggg 10080

ctaggtcggc gacaacgcgc tcggctaata tggcctgctg cacctgcgtg agggtagact 10140

ggaagtcatc catgtccaca aagcggtggt atgcgcccgt gttgatggtg taagtgcagt 10200

tggccataac ggaccagtta acggtctggt gacccggctg cgagagctcg gtgtacctga 10260

gacgcgagta agccctcgag tcaaatacgt agtcgttgca agtccgcacc aggtactggt 10320

atcccaccaa aaagtgcggc ggcggctggc ggtagagggg ccagcgtagg gtggccgggg 10380

ctccgggggc gagatcttcc aacataaggc gatgatatcc gtagatgtac ctggacatcc 10440

aggtgatgcc ggcggcggtg gtggaggcgc gcggaaagtc gcggacgcgg ttccagatgt 10500

tgcgcagcgg caaaaagtgc tccatggtcg ggacgctctg gccggtcagg cgcgcgcaat 10560

cgttgacgct ctagaccgtg caaaaggaga gcctgtaagc gggcactctt ccgtggtctg 10620

gtggataaat tcgcaagggt atcatggcgg acgaccgggg ttcgagcccc gtatccggcc 10680

gtccgccgtg atccatgcgg ttaccgcccg cgtgtcgaac ccaggtgtgc gacgtcagac 10740

aacgggggag tgctcctttt ggcttccttc caggcgcggc ggctgctgcg ctagcttttt 10800

tggccactgg ccgcgcgcag cgtaagcggt taggctggaa agcgaaagca ttaagtggct 10860

cgctccctgt agccggaggg ttattttcca agggttgagt cgcgggaccc ccggttcgag 10920

tctcggaccg gccggactgc ggcgaacggg ggtttgcctc cccgtcatgc aagaccccgc 10980

ttgcaaattc ctccggaaac agggacgagc cccttttttg cttttcccag atgcatccgg 11040

tgctgcggca gatgcgcccc cctcctcagc agcggcaaga gcaagagcag cggcagacat 11100

gcagggcacc ctcccctcct cctaccgcgt caggaggggc gacatccgcg gttgacgcgg 11160

cagcagatgg tgattacgaa cccccgcggc gccgggcccg gcactacctg gacttggagg 11220

agggcgaggg cctggcgcgg ctaggagcgc cctctcctga gcggcaccca agggtgcagc 11280

tgaagcgtga tacgcgtgag gcgtacgtgc cgcggcagaa cctgtttcgc gaccgcgagg 11340

gagaggagcc cgaggagatg cgggatcgaa agttccacgc agggcgcgag ctgcggcatg 11400

gcctgaatcg cgagcggttg ctgcgcgagg aggactttga gcccgacgcg cgaaccggga 11460

ttagtcccgc gcgcgcacac gtggcggccg ccgacctggt aaccgcatac gagcagacgg 11520

tgaaccagga gattaacttt caaaaaagct ttaacaacca cgtgcgtacg cttgtggcgc 11580

gcgaggaggt ggctatagga ctgatgcatc tgtgggactt tgtaagcgcg ctggagcaaa 11640

acccaaatag caagccgctc atggcgcagc tgttccttat agtgcagcac agcagggaca 11700

acgaggcatt cagggatgcg ctgctaaaca tagtagagcc cgagggccgc tggctgctcg 11760

atttgataaa catcctgcag agcatagtgg tgcaggagcg cagcttgagc ctggctgaca 11820

aggtggccgc catcaactat tccatgctta gcctgggcaa gttttacgcc cgcaagatat 11880

accatacccc ttacgttccc atagacaagg aggtaaagat cgaggggttc tacatgcgca 11940

tggcgctgaa ggtgcttacc ttgagcgacg acctgggcgt ttatcgcaac gagcgcatcc 12000

acaaggccgt gagcgtgagc cggcggcgcg agctcagcga ccgcgagctg atgcacagcc 12060

tgcaaagggc cctggctggc acgggcagcg gcgatagaga ggccgagtcc tactttgacg 12120

cgggcgctga cctgcgctgg gccccaagcc gacgcgccct ggaggcagct ggggccggac 12180

ctgggctggc ggtggcaccc gcgcgcgctg gcaacgtcgg cggcgtggag gaatatgacg 12240

aggacgatga gtacgagcca gaggacggcg agtactaagc ggtgatgttt ctgatcagat 12300

gatgcaagac gcaacggacc cggcggtgcg ggcggcgctg cagagccagc cgtccggcct 12360

taactccacg gacgactggc gccaggtcat ggaccgcatc atgtcgctga ctgcgcgcaa 12420

tcctgacgcg ttccggcagc agccgcaggc caaccggctc tccgcaattc tggaagcggt 12480

ggtcccggcg cgcgcaaacc ccacgcacga gaaggtgctg gcgatcgtaa acgcgctggc 12540

cgaaaacagg gccatccggc ccgacgaggc cggcctggtc tacgacgcgc tgcttcagcg 12600

cgtggctcgt tacaacagcg gcaacgtgca gaccaacctg gaccggctgg tgggggatgt 12660

gcgcgaggcc gtggcgcagc gtgagcgcgc gcagcagcag ggcaacctgg gctccatggt 12720

tgcactaaac gccttcctga gtacacagcc cgccaacgtg ccgcggggac aggaggacta 12780

caccaacttt gtgagcgcac tgcggctaat ggtgactgag acaccgcaaa gtgaggtgta 12840

ccagtctggg ccagactatt ttttccagac cagtagacaa ggcctgcaga ccgtaaacct 12900

gagccaggct ttcaaaaact tgcaggggct gtggggggtg cgggctccca caggcgaccg 12960

cgcgaccgtg tctagcttgc tgacgcccaa ctcgcgcctg ttgctgctgc taatagcgcc 13020

cttcacggac agtggcagcg tgtcccggga cacataccta ggtcacttgc tgacactgta 13080

ccgcgaggcc ataggtcagg cgcatgtgga cgagcatact ttccaggaga ttacaagtgt 13140

cagccgcgcg ctggggcagg aggacacggg cagcctggag gcaaccctaa actacctgct 13200

gaccaaccgg cggcagaaga tcccctcgtt gcacagttta aacagcgagg aggagcgcat 13260

tttgcgctac gtgcagcaga gcgtgagcct taacctgatg cgcgacgggg taacgcccag 13320

cgtggcgctg gacatgaccg cgcgcaacat ggaaccgggc atgtatgcct caaaccggcc 13380

gtttatcaac cgcctaatgg actacttgca tcgcgcggcc gccgtgaacc ccgagtattt 13440

caccaatgcc atcttgaacc cgcactggct accgccccct ggtttctaca ccgggggatt 13500

cgaggtgccc gagggtaacg atggattcct ctgggacgac atagacgaca gcgtgttttc 13560

cccgcaaccg cagaccctgc tagagttgca acagcgcgag caggcagagg cggcgctgcg 13620

aaaggaaagc ttccgcaggc caagcagctt gtccgatcta ggcgctgcgg ccccgcggtc 13680

agatgctagt agcccatttc caagcttgat agggtctctt accagcactc gcaccacccg 13740

cccgcgcctg ctgggcgagg aggagtacct aaacaactcg ctgctgcagc cgcagcgcga 13800

aaaaaacctg cctccggcat ttcccaacaa cgggatagag agcctagtgg acaagatgag 13860

tagatggaag acgtacgcgc aggagcacag ggacgtgcca ggcccgcgcc cgcccacccg 13920

tcgtcaaagg cacgaccgtc agcggggtct ggtgtgggag gacgatgact cggcagacga 13980

cagcagcgtc ctggatttgg gagggagtgg caacccgttt gcgcaccttc gccccaggct 14040

ggggagaatg ttttaaaaaa aaaaaaagca tgatgcaaaa taaaaaactc accaaggcca 14100

tggcaccgag cgttggtttt cttgtattcc ccttagtatg cggcgcgcgg cgatgtatga 14160

ggaaggtcct cctccctcct acgagagtgt ggtgagcgcg gcgccagtgg cggcggcgct 14220

gggttctccc ttcgatgctc ccctggaccc gccgtttgtg cctccgcggt acctgcggcc 14280

taccgggggg agaaacagca tccgttactc tgagttggca cccctattcg acaccacccg 14340

tgtgtacctg gtggacaaca agtcaacgga tgtggcatcc ctgaactacc agaacgacca 14400

cagcaacttt ctgaccacgg tcattcaaaa caatgactac agcccggggg aggcaagcac 14460

acagaccatc aatcttgacg accggtcgca ctggggcggc gacctgaaaa ccatcctgca 14520

taccaacatg ccaaatgtga acgagttcat gtttaccaat aagtttaagg cgcgggtgat 14580

ggtgtcgcgc ttgcctacta aggacaatca ggtggagctg aaatacgagt gggtggagtt 14640

cacgctgccc gagggcaact actccgagac catgaccata gaccttatga acaacgcgat 14700

cgtggagcac tacttgaaag tgggcagaca gaacggggtt ctggaaagcg acatcggggt 14760

aaagtttgac acccgcaact tcagactggg gtttgacccc gtcactggtc ttgtcatgcc 14820

tggggtatat acaaacgaag ccttccatcc agacatcatt ttgctgccag gatgcggggt 14880

ggacttcacc cacagccgcc tgagcaactt gttgggcatc cgcaagcggc aacccttcca 14940

ggagggcttt aggatcacct acgatgatct ggagggtggt aacattcccg cactgttgga 15000

tgtggacgcc taccaggcga gcttgaaaga tgacaccgaa cagggcgggg gtggcgcagg 15060

cggcagcaac agcagtggca gcggcgcgga agagaactcc aacgcggcag ccgcggcaat 15120

gcagccggtg gaggacatga acgatcatgc cattcgcggc gacacctttg ccacacgggc 15180

tgaggagaag cgcgctgagg ccgaagcagc ggccgaagct gccgcccccg ctgcgcaacc 15240

cgaggtcgag aagcctcaga agaaaccggt gatcaaaccc ctgacagagg acagcaagaa 15300

acgcagttac aacctaataa gcaatgacag caccttcacc cagtaccgca gctggtacct 15360

tgcatacaac tacggcgacc ctcagaccgg aatccgctca tggaccctgc tttgcactcc 15420

tgacgtaacc tgcggctcgg agcaggtcta ctggtcgttg ccagacatga tgcaagaccc 15480

cgtgaccttc cgctccacgc gccagatcag caactttccg gtggtgggcg ccgagctgtt 15540

gcccgtgcac tccaagagct tctacaacga ccaggccgtc tactcccaac tcatccgcca 15600

gtttacctct ctgacccacg tgttcaatcg ctttcccgag aaccagattt tggcgcgccc 15660

gccagccccc accatcacca ccgtcagtga aaacgttcct gctctcacag atcacgggac 15720

gctaccgctg cgcaacagca tcggaggagt ccagcgagtg accattactg acgccagacg 15780

ccgcacctgc ccctacgttt acaaggccct gggcatagtc tcgccgcgcg tcctatcgag 15840

ccgcactttt tgagcaagca tgtccatcct tatatcgccc agcaataaca caggctgggg 15900

cctgcgcttc ccaagcaaga tgtttggcgg ggccaagaag cgctccgacc aacacccagt 15960

gcgcgtgcgc gggcactacc gcgcgccctg gggcgcgcac aaacgcggcc gcactgggcg 16020

caccaccgtc gatgacgcca tcgacgcggt ggtggaggag gcgcgcaact acacgcccac 16080

gccgccacca gtgtccacag tggacgcggc cattcagacc gtggtgcgcg gagcccggcg 16140

ctatgctaaa atgaagagac ggcggaggcg cgtagcacgt cgccaccgcc gccgacccgg 16200

cactgccgcc caacgcgcgg cggcggccct gcttaaccgc gcacgtcgca ccggccgacg 16260

ggcggccatg cgggccgctc gaaggctggc cgcgggtatt gtcactgtgc cccccaggtc 16320

caggcgacga gcggccgccg cagcagccgc ggccattagt gctatgactc agggtcgcag 16380

gggcaacgtg tattgggtgc gcgactcggt tagcggcctg cgcgtgcccg tgcgcacccg 16440

ccccccgcgc aactagattg caagaaaaaa ctacttagac tcgtactgtt gtatgtatcc 16500

agcggcggcg gcgcgcaacg aagctatgtc caagcgcaaa atcaaagaag agatgctcca 16560

ggtcatcgcg ccggagatct atggcccccc gaagaaggaa gagcaggatt acaagccccg 16620

aaagctaaag cgggtcaaaa agaaaaagaa agatgatgat gatgaacttg acgacgaggt 16680

ggaactgctg cacgctaccg cgcccaggcg acgggtacag tggaaaggtc gacgcgtaaa 16740

acgtgttttg cgacccggca ccaccgtagt ctttacgccc ggtgagcgct ccacccgcac 16800

ctacaagcgc gtgtatgatg aggtgtacgg cgacgaggac ctgcttgagc aggccaacga 16860

gcgcctcggg gagtttgcct acggaaagcg gcataaggac atgctggcgt tgccgctgga 16920

cgagggcaac ccaacaccta gcctaaagcc cgtaacactg cagcaggtgc tgcccgcgct 16980

tgcaccgtcc gaagaaaagc gcggcctaaa gcgcgagtct ggtgacttgg cacccaccgt 17040

gcagctgatg gtacccaagc gccagcgact ggaagatgtc ttggaaaaaa tgaccgtgga 17100

acctgggctg gagcccgagg tccgcgtgcg gccaatcaag caggtggcgc cgggactggg 17160

cgtgcagacc gtggacgttc agatacccac taccagtagc accagtattg ccaccgccac 17220

agagggcatg gagacacaaa cgtccccggt tgcctcagcg gtggcggatg ccgcggtgca 17280

ggcggtcgct gcggccgcgt ccaagacctc tacggaggtg caaacggacc cgtggatgtt 17340

tcgcgtttca gccccccggc gcccgcgccg ttcgaggaag tacggcgccg ccagcgcgct 17400

actgcccgaa tatgccctac atccttccat tgcgcctacc cccggctatc gtggctacac 17460

ctaccgcccc agaagacgag caactacccg acgccgaacc accactggaa cccgccgccg 17520

ccgtcgccgt cgccagcccg tgctggcccc gatttccgtg cgcagggtgg ctcgcgaagg 17580

aggcaggacc ctggtgctgc caacagcgcg ctaccacccc agcatcgttt aaaagccggt 17640

ctttgtggtt cttgcagata tggccctcac ctgccgcctc cgtttcccgg tgccgggatt 17700

ccgaggaaga atgcaccgta ggaggggcat ggccggccac ggcctgacgg gcggcatgcg 17760

tcgtgcgcac caccggcggc ggcgcgcgtc gcaccgtcgc atgcgcggcg gtatcctgcc 17820

cctccttatt ccactgatcg ccgcggcgat tggcgccgtg cccggaattg catccgtggc 17880

cttgcaggcg cagagacact gattaaaaac aagttgcatg tggaaaaatc aaaataaaaa 17940

gtctggactc tcacgctcgc ttggtcctgt aactattttg tagaatggaa gacatcaact 18000

ttgcgtctct ggccccgcga cacggctcgc gcccgttcat gggaaactgg caagatatcg 18060

gcaccagcaa tatgagcggt ggcgccttca gctggggctc gctgtggagc ggcattaaaa 18120

atttcggttc caccgttaag aactatggca gcaaggcctg gaacagcagc acaggccaga 18180

tgctgaggga taagttgaaa gagcaaaatt tccaacaaaa ggtggtagat ggcctggcct 18240

ctggcattag cggggtggtg gacctggcca accaggcagt gcaaaataag attaacagta 18300

agcttgatcc ccgccctccc gtagaggagc ctccaccggc cgtggagaca gtgtctccag 18360

aggggcgtgg cgaaaagcgt ccgcgccccg acagggaaga aactctggtg acgcaaatag 18420

acgagcctcc ctcgtacgag gaggcactaa agcaaggcct gcccaccacc cgtcccatcg 18480

cgcccatggc taccggagtg ctgggccagc acacacccgt aacgctggac ctgcctcccc 18540

ccgccgacac ccagcagaaa cctgtgctgc caggcccgac cgccgttgtt gtaacccgtc 18600

ctagccgcgc gtccctgcgc cgcgccgcca gcggtccgcg atcgttgcgg cccgtagcca 18660

gtggcaactg gcaaagcaca ctgaacagca tcgtgggtct gggggtgcaa tccctgaagc 18720

gccgacgatg cttctgatag ctaacgtgtc gtatgtgtgt catgtatgcg tccatgtcgc 18780

cgccagagga gctgctgagc cgccgcgcgc ccgctttcca agatggctac cccttcgatg 18840

atgccgcagt ggtcttacat gcacatctcg ggccaggacg cctcggagta cctgagcccc 18900

gggctggtgc agtttgcccg cgccaccgag acgtacttca gcctgaataa caagtttaga 18960

aaccccacgg tggcgcctac gcacgacgtg accacagacc ggtcccagcg tttgacgctg 19020

cggttcatcc ctgtggaccg tgaggatact gcgtactcgt acaaggcgcg gttcacccta 19080

gctgtgggtg ataaccgtgt gctggacatg gcttccacgt actttgacat ccgcggcgtg 19140

ctggacaggg gccctacttt taagccctac tctggcactg cctacaacgc cctggctccc 19200

aagggtgccc caaatccttg cgaatgggat gaagctgcta ctgctcttga aataaaccta 19260

gaagaagagg acgatgacaa cgaagacgaa gtagacgagc aagctgagca gcaaaaaact 19320

cacgtatttg ggcaggcgcc ttattctggt ataaatatta caaaggaggg tattcaaata 19380

ggtgtcgaag gtcaaacacc taaatatgcc gataaaacat ttcaacctga acctcaaata 19440

ggagaatctc agtggtacga aacagaaatt aatcatgcag ctgggagagt cctaaaaaag 19500

actaccccaa tgaaaccatg ttacggttca tatgcaaaac ccacaaatga aaatggaggg 19560

caaggcattc ttgtaaagca acaaaatgga aagctagaaa gtcaagtgga aatgcaattt 19620

ttctcaacta ctgaggcagc cgcaggcaat ggtgataact tgactcctaa agtggtattg 19680

tacagtgaag atgtagatat agaaacccca gacactcata tttcttacat gcccactatt 19740

aaggaaggta actcacgaga actaatgggc caacaatcta tgcccaacag gcctaattac 19800

attgctttta gggacaattt tattggtcta atgtattaca acagcacggg taatatgggt 19860

gttctggcgg gccaagcatc gcagttgaat gctgttgtag atttgcaaga cagaaacaca 19920

gagctttcat accagctttt gcttgattcc attggtgata gaaccaggta cttttctatg 19980

tggaatcagg ctgttgacag ctatgatcca gatgttagaa ttattgaaaa tcatggaact 20040

gaagatgaac ttccaaatta ctgctttcca ctgggaggtg tgattaatac agagactctt 20100

accaaggtaa aacctaaaac aggtcaggaa aatggatggg aaaaagatgc tacagaattt 20160

tcagataaaa atgaaataag agttggaaat aattttgcca tggaaatcaa tctaaatgcc 20220

aacctgtgga gaaatttcct gtactccaac atagcgctgt atttgcccga caagctaaag 20280

tacagtcctt ccaacgtaaa aatttctgat aacccaaaca cctacgacta catgaacaag 20340

cgagtggtgg ctcccgggct agtggactgc tacattaacc ttggagcacg ctggtccctt 20400

gactatatgg acaacgtcaa cccatttaac caccaccgca atgctggcct gcgctaccgc 20460

tcaatgttgc tgggcaatgg tcgctatgtg cccttccaca tccaggtgcc tcagaagttc 20520

tttgccatta aaaacctcct tctcctgccg ggctcataca cctacgagtg gaacttcagg 20580

aaggatgtta acatggttct gcagagctcc ctaggaaatg acctaagggt tgacggagcc 20640

agcattaagt ttgatagcat ttgcctttac gccaccttct tccccatggc ccacaacacc 20700

gcctccacgc ttgaggccat gcttagaaac gacaccaacg accagtcctt taacgactat 20760

ctctccgccg ccaacatgct ctaccctata cccgccaacg ctaccaacgt gcccatatcc 20820

atcccctccc gcaactgggc ggctttccgc ggctgggcct tcacgcgcct taagactaag 20880

gaaaccccat cactgggctc gggctacgac ccttattaca cctactctgg ctctataccc 20940

tacctagatg gaacctttta cctcaaccac acctttaaga aggtggccat tacctttgac 21000

tcttctgtca gctggcctgg caatgaccgc ctgcttaccc ccaacgagtt tgaaattaag 21060

cgctcagttg acggggaggg ttacaacgtt gcccagtgta acatgaccaa agactggttc 21120

ctggtacaaa tgctagctaa ctataacatt ggctaccagg gcttctatat cccagagagc 21180

tacaaggacc gcatgtactc cttctttaga aacttccagc ccatgagccg tcaggtggtg 21240

gatgatacta aatacaagga ctaccaacag gtgggcatcc tacaccaaca caacaactct 21300

ggatttgttg gctaccttgc ccccaccatg cgcgaaggac aggcctaccc tgctaacttc 21360

ccctatccgc ttataggcaa gaccgcagtt gacagcatta cccagaaaaa gtttctttgc 21420

gatcgcaccc tttggcgcat cccattctcc agtaacttta tgtccatggg cgcactcaca 21480

gacctgggcc aaaaccttct ctacgccaac tccgcccacg cgctagacat gacttttgag 21540

gtggatccca tggacgagcc cacccttctt tatgttttgt ttgaagtctt tgacgtggtc 21600

cgtgtgcacc agccgcaccg cggcgtcatc gaaaccgtgt acctgcgcac gcccttctcg 21660
gccggcaacg ccacaacata aagaagcaag caacatcaac aacagctgcc gccatgggct 21720
ccagtgagca ggaactgaaa gccattgtca aagatcttgg ttgtgggcca tattttttgg 21780
gcacctatga caagcgcttt ccaggctttg tttctccaca caagctcgcc tgcgccatag 21840
tcaatacggc cggtcgcgag actgggggcg tacactggat ggcctttgcc tggaacccgc 21900
actcaaaaac atgctacctc tttgagccct ttggcttttc tgaccagcga ctcaagcagg 21960
tttaccagtt tgagtacgag tcactcctgc gccgtagcgc cattgcttct tcccccgacc 22020
gctgtataac gctggaaaag tccacccaaa gcgtacaggg gcccaactcg gccgcctgtg 22080
gactattctg ctgcatgttt ctccacgcct ttgccaactg gccccaaact cccatggatc 22140
acaaccccac catgaacctt attaccgggg tacccaactc catgctcaac agtccccagg 22200
tacagcccac cctgcgtcgc aaccaggaac agctctacag cttcctggag cgccactcgc 22260
cctacttccg cagccacagt gcgcagatta ggagcgccac ttctttttgt cacttgaaaa 22320
acatgtaaaa ataatgtact agagacactt tcaataaagg caaatgcttt tatttgtaca 22380
ctctcgggtg attatttacc cccacccttg ccgtctgcgc cgtttaaaaa tcaaaggggt 22440
tctgccgcgc atcgctatgc gccactggca gggacacgtt gcgatactgg tgtttagtgc 22500
tccacttaaa ctcaggcaca accatccgcg gcagctcggt gaagttttca ctccacaggc 22560
tgcgcaccat caccaacgcg tttagcaggt cgggcgccga tatcttgaag tcgcagttgg 22620
ggcctccgcc ctgcgcgcgc gagttgcgat acacagggtt gcagcactgg aacactatca 22680
gcgccgggtg gtgcacgctg gccagcacgc tcttgtcgga gatcagatcc gcgtccaggt 22740
cctccgcgtt gctcagggcg aacggagtca actttggtag ctgccttccc aaaaagggcg 22800
cgtgcccagg ctttgagttg cactcgcacc gtagtggcat caaaaggtga ccgtgcccgg 22860
tctgggcgtt aggatacagc gcctgcataa aagccttgat ctgcttaaaa gccacctgag 22920
cctttgcgcc ttcagagaag aacatgccgc aagacttgcc ggaaaactga ttggccggac 22980
aggccgcgtc gtgcacgcag caccttgcgt cggtgttgga gatctgcacc acatttcggc 23040
cccaccggtt cttcacgatc ttggccttgc tagactgctc cttcagcgcg cgctgcccgt 23100
tttcgctcgt cacatccatt tcaatcacgt gctccttatt tatcataatg cttccgtgta 23160
gacacttaag ctcgccttcg atctcagcgc agcggtgcag ccacaacgcg cagcccgtgg 23220
gctcgtgatg cttgtaggtc acctctgcaa acgactgcag gtacgcctgc aggaatcgcc 23280
ccatcatcgt cacaaaggtc ttgttgctgg tgaaggtcag ctgcaacccg cggtgctcct 23340
cgttcagcca ggtcttgcat acggccgcca gagcttccac ttggtcaggc agtagtttga 23400
agttcgcctt tagatcgtta tccacgtggt acttgtccat cagcgcgcgc gcagcctcca 23460
tgcccttctc ccacgcagac acgatcggca cactcagcgg gttcatcacc gtaatttcac 23520

tttccgcttc gctgggctct tcctcttcct cttgcgtccg cataccacgc gccactgggt 23580

cgtcttcatt cagccgccgc actgtgcgct tacctccttt gccatgcttg attagcaccg 23640

gtgggttgct gaaacccacc atttgtagcg ccacatcttc tctttcttcc tcgctgtcca 23700

cgattacctc tggtgatggc gggcgctcgg gcttgggaga agggcgcttc tttttcttct 23760

tgggcgcaat ggccaaatcc gccgccgagg tcgatggccg cgggctgggt gtgcgcggca 23820

ccagcgcgtc ttgtgatgag tcttcctcgt cctcggactc gatacgccgc ctcatccgct 23880

tttttggggg cgcccgggga ggcggcggcg acggggacgg ggacgacacg tcctccatgg 23940

ttgggggacg tcgcgccgca ccgcgtccgc gctcgggggt ggtttcgcgc tgctcctctt 24000

cccgactggc catttccttc tcctataggc agaaaaagat catggagtca gtcgagaaga 24060

aggacagcct aaccgccccc tctgagttcg ccaccaccgc ctccaccgat gccgccaacg 24120

cgcctaccac cttccccgtc gaggcacccc cgcttgagga ggaggaagtg attatcgagc 24180

aggacccagg ttttgtaagc gaagacgacg aggaccgctc agtaccaaca gaggataaaa 24240

agcaagacca ggacaacgca gaggcaaacg aggaacaagt cgggcggggg gacgaaaggc 24300

atggcgacta cctagatgtg ggagacgacg tgctgttgaa gcatctgcag cgccagtgcg 24360

ccattatctg cgacgcgttg caagagcgca gcgatgtgcc cctcgccata gcggatgtca 24420

gccttgccta cgaacgccac ctattctcac cgcgcgtacc ccccaaacgc caagaaaacg 24480

gcacatgcga gcccaacccg cgcctcaact tctaccccgt atttgccgtg ccagaggtgc 24540

ttgccaccta tcacatcttt ttccaaaact gcaagatacc cctatcctgc cgtgccaacc 24600

gcagccgagc ggacaagcag ctggccttgc ggcagggcgc tgtcatacct gatatcgcct 24660

cgctcaacga agtgccaaaa atctttgagg gtcttggacg cgacgagaag cgcgcggcaa 24720

acgctctgca acaggaaaac agcgaaaatg aaagtcactc tggagtgttg gtggaactcg 24780

agggtgacaa cgcgcgccta gccgtactaa aacgcagcat cgaggtcacc cactttgcct 24840

acccggcact taacctaccc cccaaggtca tgagcacagt catgagtgag ctgatcgtgc 24900

gccgtgcgca gcccctggag agggatgcaa atttgcaaga acaaacagag gagggcctac 24960

ccgcagttgg cgacgagcag ctagcgcgct ggcttcaaac gcgcgagcct gccgacttgg 25020

aggagcgacg caaactaatg atggccgcag tgctcgttac cgtggagctt gagtgcatgc 25080

agcggttctt tgctgacccg gagatgcagc gcaagctaga ggaaacattg cactacacct 25140

ttcgacaggg ctacgtacgc caggcctgca agatctccaa cgtggagctc tgcaacctgg 25200

tctcctacct tggaattttg cacgaaaacc gccttgggca aaacgtgctt cattccacgc 25260

tcaagggcga ggcgcgccgc gactacgtcc gcgactgcgt ttacttattt ctatgctaca 25320

cctggcagac ggccatgggc gtttggcagc agtgcttgga ggagtgcaac ctcaaggagc 25380

tgcagaaact gctaaagcaa aacttgaagg acctatggac ggccttcaac gagcgctccg 25440

tggccgcgca cctggcggac atcattttcc ccgaacgcct gcttaaaacc ctgcaacagg 25500

gtctgccaga cttcaccagt caaagcatgt tgcagaactt taggaacttt atcctagagc 25560

gctcaggaat cttgcccgcc acctgctgtg cacttcctag cgactttgtg cccattaagt 25620

accgcgaatg ccctccgccg ctttggggcc actgctacct tctgcagcta gccaactacc 25680

ttgcctacca ctctgacata atggaagacg tgagcggtga cggtctactg gagtgtcact 25740

gtcgctgcaa cctatgcacc ccgcaccgct ccctggtttg caattcgcag ctgcttaacg 25800

aaagtcaaat tatcggtacc tttgagctgc agggtccctc gcctgacgaa aagtccgcgg 25860

ctccggggtt gaaactcact ccggggctgt ggacgtcggc ttaccttcgc aaatttgtac 25920

ctgaggacta ccacgcccac gagattaggt tctacgaaga ccaatcccgc ccgcctaatg 25980

cggagcttac cgcctgcgtc attacccagg gccacattct tggccaattg caagccatca 26040

acaaagcccg ccaagagttt ctgctacgaa agggacgggg ggtttacttg gacccccagt 26100

ccggcgagga gctcaaccca atccccccgc cgccgcagcc ctatcagcag cagccgcggg 26160

cccttgcttc ccaggatggc acccaaaaag aagctgcagc tgccgccgcc acccacggac 26220

gaggaggaat actgggacag tcaggcagag gaggttttgg acgaggagga ggaggacatg 26280

atggaagact gggagagcct agacgaggaa gcttccgagg tcgaagaggt gtcagacgaa 26340

acaccgtcac cctcggtcgc attcccctcg ccggcgcccc agaaatcggc aaccggttcc 26400

agcatggcta caacctccgc tcctcaggcg ccgccggcac tgcccgttcg ccgacccaac 26460

cgtagatggg acaccactgg aaccagggcc ggtaagtcca agcagccgcc gccgttagcc 26520

caagagcaac aacagcgcca aggctaccgc tcatggcgcg ggcacaagaa cgccatagtt 26580

gcttgcttgc aagactgtgg gggcaacatc tccttcgccc gccgctttct tctctaccat 26640

cacggcgtgg ccttcccccg taacatcctg cattactacc gtcatctcta cagcccatac 26700

tgcaccggcg gcagcggcag caacagcagc ggccacacag aagcaaaggc gaccggatag 26760

caagactctg acaaagccca agaaatccac agcggcggca gcagcaggag gaggagcgct 26820

gcgtctggcg cccaacgaac ccgtatcgac ccgcgagctt agaaacagga tttttcccac 26880

tctgtatgct atatttcaac agagcagggg ccaagaacaa gagctgaaaa taaaaaacag 26940

gtctctgcga tccctcaccc gcagctgcct gtatcacaaa agcgaagatc agcttcggcg 27000

cacgctggaa gacgcggagg ctctcttcag taaatactgc gcgctgactc ttaaggacta 27060

gtttcgcgcc ctttctcaaa tttaagcgcg aaaactacgt catctccagc ggccacaccc 27120

ggcgccagca cctgttgtca gcgccattat gagcaaggaa attcccacgc cctacatgtg 27180

gagttaccag ccacaaatgg gacttgcggc tggagctgcc caagactact caacccgaat 27240

aaactacatg agcgcgggac cccacatgat atcccgggtc aacggaatac gcgcccaccg 27300

aaaccgaatt ctcctggaac aggcggctat taccaccaca cctcgtaata accttaatcc 27360

ccgtagttgg cccgctgccc tggtgtacca ggaaagtccc gctcccacca ctgtggtact 27420

tcccagagac gcccaggccg aagttcagat gactaactca ggggcgcagc ttgcgggcgg 27480

ctttcgtcac agggtgcggt cgcccgggca gggtataact cacctgacaa tcagagggcg 27540

aggtattcag ctcaacgacg agtcggtgag ctcctcgctt ggtctccgtc cggacgggac 27600

atttcagatc ggcggcgccg gccgctcttc attcacgcct cgtcaggcaa tcctaactct 27660

gcagacctcg tcctctgagc cgcgctctgg aggcattgga actctgcaat ttattgagga 27720

gtttgtgcca tcggtctact ttaacccctt ctcgggacct cccggccact atccggatca 27780

atttattcct aactttgacg cggtaaagga ctcggcggac ggctacgact gaatgttaag 27840

tggagaggca gagcaactgc gcctgaaaca cctggtccac tgtcgccgcc acaagtgctt 27900

tgcccgcgac tccggtgagt tttgctactt tgaattgccc gaggatcata tcgagggccc 27960

ggcgcacggc gtccggctta ccgcccaggg agagcttgcc cgtagcctga ttcgggagtt 28020

tacccagcgc cccctgctag ttgagcggga caggggaccc tgtgttctca ctgtgatttg 28080

caactgtcct aaccctggat tacatcaaga tctttgttgc catctctgtg ctgagtataa 28140

taaatacaga aattaaaata tactggggct cctatcgcca tcctgtaaac gccaccgtct 28200

tcacccgccc aagcaaacca aggcgaacct tacctggtac ttttaacatc tctccctctg 28260

tgatttacaa cagtttcaac ccagacggag tgagtctacg agagaacctc tccgagctca 28320

gctactccat cagaaaaaac accaccctcc ttacctgccg ggaacgtacg atgcggctgg 28380

gcagtcctgg actgctcttc ctgctcttca gcagccttcg agctgatact caggagaagg 28440

aagtcagagc gatggtaggc agcgacgtgg agctcagctg cgcttgccct gaaggaagcc 28500

gttttgactt aaatgatgtt tacgtatatt ggcaaaccag tgagtcgaaa accgtggtga 28560

cctaccacat cccacagaac agctccttgg aaaacgtgga cagccgctac cggaaccgag 28620

ccctgatgtc accggccggc atgctgcggg gcgacttctc cctgcgcttg ttcaacgtca 28680

ccccccagga cgagcagaag tttcactgcc tggtgttgag ccaatccctg ggattccagg 28740

aggttttgag cgttgaggtt acactgcatg tggcagcaaa cttcagcgtg cccgtcgtca 28800

gcgcccccca cagcccctcc caggatgagc tcaccttcac gtgtacatcc ataaacggct 28860

accccaggcc caacgtgtac tggatcaata agacggacaa cagcctgctg gaccaggctc 28920

tgcagaatga caccgtcttc ttgaacatgc ggggcttgta tgacgtggtc agcgtgctga 28980

ggatcgcacg gacccccagc gtgaacattg gctgctgcat agagaacgtg cttctgcagc 29040

agaacctgac tgtcggcagc cagacaggaa atgacatcgg agagagagac aagatcacag 29100

agaatccagt cagtaccggc gagaaaaacg cggccacgtg gagcatcctg gctgtcctgt 29160

gcctgcttgt ggtcgtggcg gtggccatag gctgggtgtg cagggaccga tgcctccaac 29220

acagctatgc aggtgcctgg gctgtgagtc cggagacaga gctcactggc cacgtttgac 29280

aattgactct atgtgggata tgctccagcg ctacaacctt gaagtcaggc ttcctggatg 29340

tcagcatctg actttggcca gcacctgtcc cgcggatttg ttccagtcca actacagcga 29400

cccaccctaa cagagatgac caacacaacc aacgcggccg ccgctaccgg acttacatct 29460

accacaaata caccccaagt ttctgccttt gtcaataact gggataactt gggcatgtgg 29520

tggttctcca tagcgcttat gtttgtatgc cttattatta tgtggctcat ctgctgccta 29580

aagcgcaaac gcgcccgacc acccatctat agtcccatca ttgtgctaca cccaaacaat 29640

gatggaatcc atagattgga cggactgaaa cacatgttct tttctcttac agtatgatta 29700

aatgagacat gattcctcga gtttttatat tactgaccct tgttgcgctt ttttgtgcgt 29760

gctccacatt ggctgcggtt tctcacatcg aagtagactg cattccagcc ttcacagtct 29820

atttgcttta cggatttgtc accctcacgc tcatctgcag cctcatcact gtggtcatcg 29880

cctttatcca gtgcattgac tgggtctgtg tgcgctttgc atatctcaga caccatcccc 29940

agtacaggga caggactata gctgagcttc ttagaattct ttaattatga aatttactgt 30000

gacttttctg ctgattattt gcaccctatc tgcgttttgt tccccgacct ccaagcctca 30060

aagacatata tcatgcagat tcactcgtat atggaatatt ccaagttgct acaatgaaaa 30120

aagcgatctt tccgaagcct ggttatatgc aatcatctct gttatggtgt tctgcagtac 30180

catcttagcc ctagctatat atccctacct tgacattggc tggaacgcaa tagatgccat 30240

gaaccaccca actttccccg cgcccgctat gcttccactg caacaagttg ttgccggcgg 30300

ctttgtccca gccaatcagc ctcgcccacc ttctcccacc cccactgaaa tcagctactt 30360

taatctaaca ggaggagatg actgacaccc tagatctaga aatggacgga attattacag 30420

agcagcgcct gctagaaaga cgcagggcag cggccgagca acagcgcatg aatcaagagc 30480

tccaagacat ggttaacttg caccagtgca aaaggggtat cttttgtctc gtaaagcagg 30540

ccaaagtcac ctacgacagt aataccaccg gacaccgcct tagctacaag ttgccaacca 30600

agcgtcagaa attggtggtc atggtgggag aaaagcccat taccataact cagcactcgg 30660

tagaaaccga aggctgcatt cactcacctt gtcaaggacc tgaggatctc tgcaccctta 30720

ttaagaccct gtgcggtctc aaagatctta ttccctttaa ctaataaaaa aaaataataa 30780

agcatcactt acttaaaatc agttagcaaa tttctgtcca gtttattcag cagcacctcc 30840

ttgccctcct cccagctctg gtattgcagc ttcctcctgg ctgcaaactt tctccacaat 30900

ctaaatggaa tgtcagtttc ctcctgttcc tgtccatccg cacccactat cttcatgttg 30960

ttgcagatga agcgcgcaag accgtctgaa gataccttca accccgtgta tccatatgac 31020

acggaaaccg gtcctccaac tgtgcctttt cttactcctc cctttgtatc ccccaatggg 31080

tttcaagaga gtccccctgg ggtactctct ttgcgcctat ccgaacctct agttacctcc 31140

aatggcatgc ttgcgctcaa aatgggcaac ggcctctctc tggacgaggc cggcaacctt 31200

acctcccaaa atgtaaccac tgtgagccca cctctcaaaa aaaccaagtc aaacataaac 31260

ctggaaatat ctgcacccct cacagttacc tcagaagccc taactgtggc tgccgccgca 31320

cctctaatgg tcgcgggcaa cacactcacc atgcaatcac aggccccgct aaccgtgcac 31380

gactccaaac ttagcattgc cacccaagga cccctcacag tgtcagaagg aaagctagcc 31440

ctgcaaacat caggccccct caccaccacc gatagcagta cccttactat cactgcctca 31500

ccccctctaa ctactgccac tggtagcttg ggcattgact tgaaagagcc catttataca 31560

caaaatggaa aactaggact aaagtacggg gctcctttgc atgtaacaga cgacctaaac 31620

actttgaccg tagcaactgg tccaggtgtg actattaata atacttcctt gcaaactaaa 31680

gttactggag ccttgggttt tgattcacaa ggcaatatgc aacttaatgt agcaggagga 31740

ctaaggattg attctcaaaa cagacgcctt atacttgatg ttagttatcc gtttgatgct 31800

caaaaccaac taaatctaag actaggacag ggccctcttt ttataaactc agcccacaac 31860

ttggatatta actacaacaa aggcctttac ttgtttacag cttcaaacaa ttccaaaaag 31920

cttgaggtta acctaagcac tgccaagggg ttgatgtttg acgctacagc catagccatt 31980

aatgcaggag atgggcttga atttggttca cctaatgcac caaacacaaa tcccctcaaa 32040

acaaaaattg gccatggcct agaatttgat tcaaacaagg ctatggttcc taaactagga 32100

actggcctta gttttgacag cacaggtgcc attacagtag gaaacaaaaa taatgataag 32160

ctaaccctat ggacaggtcc aaaaccagaa gccaactgca taattgaata cgggaaacaa 32220

aacccagata gcaaactaac tttaatcctt gtaaaaaatg gaggaattgt taatggatat 32280

gtaacgctaa tgggagcctc agactacgtt aacaccttat ttaaaaacaa aaatgtctcc 32340

attaatgtag aactatactt tgatgccact ggtcatatat taccagactc atcttctctt 32400

aaaacagatc tagaactaaa atacaagcaa accgctgact ttagtgcaag aggttttatg 32460

ccaagtacta cagcgtatcc atttgtcctt cctaatgcgg gaacacataa tgaaaattat 32520

atttttggtc aatgctacta caaagcaagc gatggtgccc tttttccgtt ggaagttact 32580

gttatgctta ataaacgcct gccagatagt cgcacatcct atgttatgac ttttttatgg 32640

tccttgaatg ctggtctagc tccagaaact actcaggcaa ccctcataac ctccccattt 32700

accttttcct atattagaga agatgactaa taaactctaa agaatcgttt gtgttatgtt 32760

tcaacgtgtt tatttttcaa ttgcagaaaa tttcaagtca tttttcattc agtagtatag 32820

ccccaccacc acatagctta tacagatcac cgtaccttaa tcaaactcac agaaccctag 32880

tattcaacct gccacctccc tcccaacaca cagagtacac agtcctttct ccccggctgg 32940

ccttaaaaag catcatatca tgggtaacag acatattctt aggtgttata ttccacacgg 33000

tttcctgtcg agccaaacgc tcatcagtga tattaataaa ctccccgggc agctcactta 33060

agttcatgtc gctgtccagc tgctgagcca caggctgctg tccaacttgc ggttgcttaa 33120

cgggcggcga aggagaagtc cacgcctaca tgggggtaga gtcataatcg tgcatcagga 33180

tagggcggtg gtgctgcagc agcgcgcgaa taaactgctg ccgccgccgc tccgtcctgc 33240

aggaatacaa catggcagtg gtctcctcag cgatgattcg caccgcccgc agcataaggc 33300

gccttgtcct ccgggcacag cagcgcaccc tgatctcact taaatcagca cagtaactgc 33360

agcacagcac cacaatattg ttcaaaatcc cacagtgcaa ggcgctgtat ccaaagctca 33420

tggcggggac cacagaaccc acgtggccat cataccacaa gcgcaggtag attaagtggc 33480

gacccctcat aaacacgctg gacataaaca ttacctcttt tggcatgttg taattcacca 33540

cctcccggta ccatataaac ctctgattaa acatggcgcc atccaccacc atcctaaacc 33600

agctggccaa aacctgcccg ccggctatac actgcaggga accgggactg gaacaatgac 33660

agtggagagc ccaggactcg taaccatgga tcatcatgct cgtcatgata tcaatgttgg 33720

cacaacacag gcacacgtgc atacacttcc tcaggattac aagctcctcc cgcgttagaa 33780

ccatatccca gggaacaacc cattcctgaa tcagcgtaaa tcccacactg cagggaagac 33840

ctcgcacgta actcacgttg tgcattgtca aagtgttaca ttcgggcagc agcggatgat 33900

cctccagtat ggtagcgcgg gtttctgtct caaaaggagg tagacgatcc ctactgtacg 33960

gagtgcgccg agacaaccga gatcgtgttg gtcgtagtgt catgccaaat ggaacgccgg 34020

acgtagtcat atttcctgaa gcaaaaccag gtgcgggcgt gacaaacaga tctgcgtctc 34080

cggtctcgcc gcttagatcg ctctgtgtag tagttgtagt atatccactc tctcaaagca 34140

tccaggcgcc ccctggcttc gggttctatg taaactcctt catgcgccgc tgccctgata 34200

acatccacca ccgcagaata agccacaccc agccaaccta cacattcgtt ctgcgagtca 34260

cacacgggag gagcgggaag agctggaaga accatgtttt tttttttatt ccaaaagatt 34320

atccaaaacc tcaaaatgaa gatctattaa gtgaacgcgc tcccctccgg tggcgtggtc 34380

aaactctaca gccaaagaac agataatggc atttgtaaga tgttgcacaa tggcttccaa 34440

aaggcaaacg gccctcacgt ccaagtggac gtaaaggcta aacccttcag ggtgaatctc 34500

ctctataaac attccagcac cttcaaccat gcccaaataa ttctcatctc gccaccttct 34560

caatatatct ctaagcaaat cccgaatatt aagtccggcc attgtaaaaa tctgctccag 34620

agcgccctcc accttcagcc tcaagcagcg aatcatgatt gcaaaaattc aggttcctca 34680

cagacctgta taagattcaa aagcggaaca ttaacaaaaa taccgcgatc ccgtaggtcc 34740

cttcgcaggg ccagctgaac ataatcgtgc aggtctgcac ggaccagcgc ggccacttcc 34800

ccgccaggaa ccatgacaaa agaacccaca ctgattatga cacgcatact cggagctatg 34860

ctaaccagcg tagccccgat gtaagcttgt tgcatgggcg gcgatataaa atgcaaggtg 34920

ctgctcaaaa aatcaggcaa agcctcgcgc aaaaaagaaa gcacatcgta gtcatgctca 34980

tgcagataaa ggcaggtaag ctccggaacc accacagaaa aagacaccat ttttctctca 35040

aacatgtctg cgggtttctg cataaacaca aaataaaata acaaaaaaac atttaaacat 35100

tagaagcctg tcttacaaca ggaaaaacaa cccttataag cataagacgg actacggcca 35160

tgccggcgtg accgtaaaaa aactggtcac cgtgattaaa aagcaccacc gacagctcct 35220

cggtcatgtc cggagtcata atgtaagact cggtaaacac atcaggttga ttcacatcgg 35280

tcagtgctaa aaagcgaccg aaatagcccg ggggaataca tacccgcagg cgtagagaca 35340

acattacagc ccccatagga ggtataacaa aattaatagg agagaaaaac acataaacac 35400

ctgaaaaacc ctcctgccta ggcaaaatag caccctcccg ctccagaaca acatacagcg 35460

cttccacagc ggcagccata acagtcagcc ttaccagtaa aaaagaaaac ctattaaaaa 35520

aacaccactc gacacggcac cagctcaatc agtcacagtg taaaaaaggg ccaagtgcag 35580

agcgagtata tataggacta aaaaatgacg taacggttaa agtccacaaa aaacacccag 35640

aaaaccgcac gcgaacctac gcccagaaac gaaagccaaa aaacccacaa cttcctcaaa 35700

tcgtcacttc cgttttccca cgttacgtca cttcccattt taagaaaact acaattccca 35760

acacatacaa gttactccgc cctaaaacct acgtcacccg ccccgttccc acgccccgcg 35820

ccacgtcaca aactccaccc cctcattatc atattggctt caatccaaaa taaggtatat 35880

tattgatgat gttaat 35896

<210> 3
<211> 37093
<212> DNA
<213> Artificial Sequence

<220>
<223> AdV5/3-D24-ADA (TGX-05)

<400> 3
attaacatca tcaataatat accttatttt ggattgaagc caatatgata atgagggggt 60

ggagtttgtg acgtggcgcg gggcgtggga acggggcggg tgacgtagta gtgtggcgga 120

agtgtgatgt tgcaagtgtg gcggaacaca tgtaagcgac ggatgtggca aaagtgacgt 180

ttttggtgtg cgccggtgta cacaggaagt gacaattttc gcgcggtttt aggcggatgt 240

tgtagtaaat ttgggcgtaa ccgagtaaga tttggccatt ttcgcgggaa aactgaataa 300

gaggaagtga aatctgaata attttgtgtt actcatagcg cgtaatattt gtctagggcc 360

gcggggactt tgaccgttta cgtggagact cgcccaggtg tttttctcag gtgttttccg 420

cgttccgggt caaagttggc gttttattat tatagtcagc tgacgtgtag tgtatttata 480

cccggtgagt tcctcaagag gccactcttg agtgccagcg agtagagttt tctcctccga 540
gccgctccga caccgggact gaaaatgaga catattatct gccacggagg tgttattacc 600
gaagaaatgg ccgccagtct tttggaccag ctgatcgaag aggtactggc tgataatctt 660
ccacctccta gccattttga accacctacc cttcacgaac tgtatgattt agacgtgacg 720
gcccccgaag atcccaacga ggaggcggtt tcgcagattt ttcccgactc tgtaatgttg 780
gcggtgcagg aagggattga cttactcact tttccgccgg cgcccggttc tccggagccg 840
cctcaccttt cccggcagcc cgagcagccg gagcagagag ccttgggtcc ggtttctatg 900
ccaaaccttg taccggaggt gatcgatcca cccagtgacg acgaggatga agagggtgag 960
gagtttgtgt tagattatgt ggagcacccc gggcacggtt gcaggtcttg tcattatcac 1020
cggaggaata cgggggaccc agatattatg tgttcgcttt gctatatgag gacctgtggc 1080
atgtttgtct acagtaagtg aaaattatgg gcagtgggtg atagagtggt gggtttggtg 1140
tggtaatttt ttttttaatt tttacagttt tgtggtttaa agaattttgt attgtgattt 1200
ttttaaaagg tcctgtgtct gaacctgagc ctgagcccga gccagaaccg gagcctgcaa 1260
gacctacccg ccgtcctaaa atggcgcctg ctatcctgag acgcccgaca tcacctgtgt 1320
ctagagaatg caatagtagt acggatagct gtgactccgg tccttctaac acacctcctg 1380
agatacaccc ggtggtcccg ctgtgcccca ttaaaccagt tgccgtgaga gttggtgggc 1440
gtcgccaggc tgtggaatgt atcgaggact tgcttaacga gcctgggcaa cctttggact 1500
tgagctgtaa acgccccagg ccataaggtg taaacctgtg attgcgtgtg tggttaacgc 1560
ctttgtttgc tgaatgagtt gatgtaagtt taataaaggg tgagataatg tttaacttgc 1620
atggcgtgtt aaatggggcg gggcttaaag ggtatataat gcgccgtggg ctaatcttgg 1680
ttacatctga cctcatggag gcttgggagt gtttggaaga tttttctgct gtgcgtaact 1740
tgctggaaca gagctctaac agtacctctt ggttttggag gtttctgtgg ggctcatccc 1800
aggcaaagtt agtctgcaga attaaggagg attacaagtg ggaatttgaa gagcttttga 1860
aatcctgtgg tgagctgttt gattctttga atctgggtca ccaggcgctt ttccaagaga 1920
aggtcatcaa gactttggat ttttccacac cggggcgcgc tgcggctgct gttgcttttt 1980
tgagttttat aaaggataaa tggagcgaag aaacccatct gagcgggggg tacctgctgg 2040
attttctggc catgcatctg tggagagcgg ttgtgagaca caagaatcgc ctgctactgt 2100
tgtcttccgt ccgcccggcg ataataccga cggaggagca gcagcagcag caggaggaag 2160
ccaggcggcg gcggcaggag cagagcccat ggaacccgag agccggcctg gaccctcggg 2220
aatgaatgtt gtacaggtgg ctgaactgta tccagaactg agacgcattt tgacaattac 2280
agaggatggg caggggctaa agggggtaaa gagggagcgg ggggcttgtg aggctacaga 2340

ggaggctagg aatctagctt ttagcttaat gaccagacac cgtcctgagt gtattacttt 2400

tcaacagatc aaggataatt gcgctaatga gcttgatctg ctggcgcaga agtattccat 2460

agagcagctg accacttact ggctgcagcc aggggatgat tttgaggagg ctattagggt 2520

atatgcaaag gtggcactta ggccagattg caagtacaag atcagcaaac ttgtaaatat 2580

caggaattgt tgctacattt ctgggaacgg ggccgaggtg gagatagata cggaggatag 2640

ggtggccttt agatgtagca tgataaatat gtggccgggg gtgcttggca tggacggggt 2700

ggttattatg aatgtaaggt ttactggccc caattttagc ggtacggttt tcctggccaa 2760

taccaacctt atcctacacg gtgtaagctt ctatgggttt aacaatacct gtgtggaagc 2820

ctggaccgat gtaagggttc ggggctgtgc cttttactgc tgctggaagg gggtggtgtg 2880

tcgccccaaa agcagggctt caattaagaa atgcctcttt gaaaggtgta ccttgggtat 2940

cctgtctgag ggtaactcca gggtgcgcca caatgtggcc tccgactgtg gttgcttcat 3000

gctagtgaaa agcgtggctg tgattaagca taacatggta tgtggcaact gcgaggacag 3060

ggcctctcag atgctgacct gctcggacgg caactgtcac ctgctgaaga ccattcacgt 3120

agccagccac tctcgcaagg cctggccagt gtttgagcat aacatactga cccgctgttc 3180

cttgcatttg ggtaacagga ggggggtgtt cctaccttac caatgcaatt tgagtcacac 3240

taagatattg cttgagcccg agagcatgtc caaggtgaac ctgaacgggg tgtttgacat 3300

gaccatgaag atctggaagg tgctgaggta cgatgagacc cgcaccaggt gcagaccctg 3360

cgagtgtggc ggtaaacata ttaggaacca gcctgtgatg ctggatgtga ccgaggagct 3420

gaggcccgat cacttggtgc tggcctgcac ccgcgctgag tttggctcta gcgatgaaga 3480

tacagattga ggtactgaaa tgtgtgggcg tggcttaagg gtgggaaaga atatataagg 3540

tgggggtctt atgtagtttt gtatctgttt tgcagcagcc gccgccgcca tgagcaccaa 3600

ctcgtttgat ggaagcattg tgagctcata tttgacaacg cgcatgcccc catgggccgg 3660

ggtgcgtcag aatgtgatgg gctccagcat tgatggtcgc cccgtcctgc ccgcaaactc 3720

tactaccttg acctacgaga ccgtgtctgg aacgccgttg gagactgcag cctccgccgc 3780

cgcttcagcc gctgcagcca ccgcccgcgg gattgtgact gactttgctt tcctgagccc 3840

gcttgcaagc agtgcagctt cccgttcatc cgcccgcgat gacaagttga cggctctttt 3900

ggcacaattg gattctttga cccgggaact taatgtcgtt tctcagcagc tgttggatct 3960

gcgccagcag gtttctgccc tgaaggcttc ctcccctccc aatgcggttt aaaacataaa 4020

taaaaaacca gactctgttt ggatttggat caagcaagtg tcttgctgtc tttatttagg 4080

ggttttgcgc gcgcggtagg cccgggacca gcggtctcgg tcgttgaggg tcctgtgtat 4140

tttttccagg acgtggtaaa ggtgactctg gatgttcaga tacatgggca taagcccgtc 4200

tctggggtgg aggtagcacc actgcagagc ttcatgctgc ggggtggtgt tgtagatgat 4260

ccagtcgtag caggagcgct gggcgtggtg cctaaaaatg tctttcagta gcaagctgat 4320

tgccaggggc aggcccttgg tgtaagtgtt tacaaagcgg ttaagctggg atgggtgcat 4380

acgtggggat atgagatgca tcttggactg tatttttagg ttggctatgt tcccagccat 4440

atccctccgg ggattcatgt tgtgcagaac caccagcaca gtgtatccgg tgcacttggg 4500

aaatttgtca tgtagcttag aaggaaatgc gtggaagaac ttggagacgc ccttgtgacc 4560

tccaagattt tccatgcatt cgtccataat gatggcaatg ggcccacggg cggcggcctg 4620

ggcgaagata tttctgggat cactaacgtc atagttgtgt tccaggatga gatcgtcata 4680

ggccattttt acaaagcgcg ggcggagggt gccagactgc ggtataatgg ttccatccgg 4740

cccaggggcg tagttaccct cacagatttg catttcccac gctttgagtt cagatggggg 4800

gatcatgtct acctgcgggg cgatgaagaa aacggtttcc ggggtagggg agatcagctg 4860

ggaagaaagc aggttcctga gcagctgcga cttaccgcag ccggtgggcc cgtaaatcac 4920

acctattacc ggctgcaact ggtagttaag agagctgcag ctgccgtcat ccctgagcag 4980

gggggccact tcgttaagca tgtccctgac tcgcatgttt tccctgacca aatccgccag 5040

aaggcgctcg ccgcccagcg atagcagttc ttgcaaggaa gcaaagtttt tcaacggttt 5100

gagaccgtcc gccgtaggca tgcttttgag cgtttgacca agcagttcca ggcggtccca 5160

cagctcggtc acctgctcta cggcatctcg atccagcata tctcctcgtt tcgcgggttg 5220

gggcggcttt cgctgtacgg cagtagtcgg tgctcgtcca gacgggccag ggtcatgtct 5280

ttccacgggc gcagggtcct cgtcagcgta gtctgggtca cggtgaaggg gtgcgctccg 5340

ggctgcgcgc tggccagggt gcgcttgagg ctggtcctgc tggtgctgaa gcgctgccgg 5400

tcttcgccct gcgcgtcggc caggtagcat ttgaccatgg tgtcatagtc cagcccctcc 5460

gcggcgtggc ccttggcgcg cagcttgccc ttggaggagg cgccgcacga ggggcagtgc 5520

agacttttga gggcgtagag cttgggcgcg agaaataccg attccgggga gtaggcatcc 5580

gcgccgcagg ccccgcagac ggtctcgcat tccacgagcc aggtgagctc tggccgttcg 5640

gggtcaaaaa ccaggtttcc cccatgcttt ttgatgcgtt tcttacctct ggtttccatg 5700

agccggtgtc cacgctcggt gacgaaaagg ctgtccgtgt ccccgtatac agacttgaga 5760

ggcctgtcct cgagcggtgt tccgcggtcc tcctcgtata gaaactcgga ccactctgag 5820

acaaaggctc gcgtccaggc cagcacgaag gaggctaagt gggaggggta gcggtcgttg 5880

tccactaggg ggtccactcg ctccagggtg tgaagacaca tgtcgccctc ttcggcatca 5940

aggaaggtga ttggtttgta ggtgtaggcc acgtgaccgg gtgttcctga aggggggcta 6000

taaaaggggg tgggggcgcg ttcgtcctca ctctcttccg catcgctgtc tgcgagggcc 6060

agctgttggg gtgagtactc cctctgaaaa gcgggcatga cttctgcgct aagattgtca 6120

gtttccaaaa acgaggagga tttgatattc acctggcccg cggtgatgcc tttgagggtg 6180

gccgcatcca tctggtcaga aaagacaatc tttttgttgt caagcttggt ggcaaacgac 6240

ccgtagaggg cgttggacag caacttggcg atggagcgca gggtttggtt tttgtcgcga 6300

tcggcgcgct ccttggccgc gatgtttagc tgcacgtatt cgcgcgcaac gcaccgccat 6360

tcgggaaaga cggtggtgcg ctcgtcgggc accaggtgca cgcgccaacc gcggttgtgc 6420

agggtgacaa ggtcaacgct ggtggctacc tctccgcgta ggcgctcgtt ggtccagcag 6480

aggcggccgc ccttgcgcga gcagaatggc ggtagggggt ctagctgcgt ctcgtccggg 6540

gggtctgcgt ccacggtaaa gaccccgggc agcaggcgcg cgtcgaagta gtctatcttg 6600

catccttgca agtctagcgc ctgctgccat gcgcgggcgg caagcgcgcg ctcgtatggg 6660

ttgagtgggg gaccccatgg catggggtgg gtgagcgcgg aggcgtacat gccgcaaatg 6720

tcgtaaacgt agaggggctc tctgagtatt ccaagatatg tagggtagca tcttccaccg 6780

cggatgctgg cgcgcacgta atcgtatagt tcgtgcgagg gagcgaggag gtcgggaccg 6840

aggttgctac gggcgggctg ctctgctcgg aagactatct gcctgaagat ggcatgtgag 6900

ttggatgata tggttggacg ctggaagacg ttgaagctgg cgtctgtgag acctaccgcg 6960

tcacgcacga aggaggcgta ggagtcgcgc agcttgttga ccagctcggc ggtgacctgc 7020

acgtctaggg cgcagtagtc cagggtttcc ttgatgatgt catacttatc ctgtcccttt 7080

tttttccaca gctcgcggtt gaggacaaac tcttcgcggt ctttccagta ctcttggatc 7140

ggaaacccgt cggcctccga acggtaagag cctagcatgt agaactggtt gacggcctgg 7200

taggcgcagc atcccttttc tacgggtagc gcgtatgcct gcgcggcctt ccggagcgag 7260

gtgtgggtga gcgcaaaggt gtccctgacc atgactttga ggtactggta tttgaagtca 7320

gtgtcgtcgc atccgccctg ctcccagagc aaaaagtccg tgcgcttttt ggaacgcgga 7380

tttggcaggg cgaaggtgac atcgttgaag agtatctttc ccgcgcgagg cataaagttg 7440

cgtgtgatgc ggaagggtcc cggcacctcg gaacggttgt taattacctg ggcggcgagc 7500

acgatctcgt caaagccgtt gatgttgtgg cccacaatgt aaagttccaa gaagcgcggg 7560

atgcccttga tggaaggcaa ttttttaagt tcctcgtagg tgagctcttc aggggagctg 7620

agcccgtgct ctgaaagggc ccagtctgca agatgagggt tggaagcgac gaatgagctc 7680

cacaggtcac gggccattag catttgcagg tggtcgcgaa aggtcctaaa ctggcgacct 7740

atggccattt tttctggggt gatgcagtag aaggtaagcg ggtcttgttc ccagcggtcc 7800

catccaaggt tcgcggctag gtctcgcgcg gcagtcacta gaggctcatc tccgccgaac 7860

ttcatgacca gcatgaaggg cacgagctgc ttcccaaagg cccccatcca agtataggtc 7920

tctacatcgt aggtgacaaa gagacgctcg gtgcgaggat gcgagccgat cgggaagaac 7980

tggatctccc gccaccaatt ggaggagtgg ctattgatgt ggtgaaagta gaagtccctg 8040

cgacgggccg aacactcgtg ctggcttttg taaaaacgtg cgcagtactg gcagcggtgc 8100

acgggctgta catcctgcac gaggttgacc tgacgaccgc gcacaaggaa gcagagtggg 8160

aatttgagcc cctcgcctgg cgggtttggc tggtggtctt ctacttcggc tgcttgtcct 8220

tgaccgtctg gctgctcgag gggagttacg gtggatcgga ccaccacgcc gcgcgagccc 8280

aaagtccaga tgtccgcgcg cggcggtcgg agcttgatga caacatcgcg cagatgggag 8340

ctgtccatgg tctggagctc ccgcggcgtc aggtcaggcg ggagctcctg caggtttacc 8400

tcgcatagac gggtcagggc gcgggctaga tccaggtgat acctaatttc caggggctgg 8460

ttggtggcgg cgtcgatggc ttgcaagagg ccgcatcccc gcggcgcgac tacggtaccg 8520

cgcggcgggc ggtgggccgc gggggtgtcc ttggatgatg catctaaaag cggtgacgcg 8580

ggcgagcccc cggaggtagg gggggctccg gacccgccgg gagagggggc aggggcacgt 8640

cggcgccgcg cgcgggcagg agctggtgct gcgcgcgtag gttgctggcg aacgcgacga 8700

cgcggcggtt gatctcctga atctggcgcc tctgcgtgaa gacgacgggc ccggtgagct 8760

tgaacctgaa agagagttcg acagaatcaa tttcggtgtc gttgacggcg gcctggcgca 8820

aaatctcctg cacgtctcct gagttgtctt gataggcgat ctcggccatg aactgctcga 8880

tctcttcctc ctggagatct ccgcgtccgg ctcgctccac ggtggcggcg aggtcgttgg 8940

aaatgcgggc catgagctgc gagaaggcgt tgaggcctcc ctcgttccag acgcggctgt 9000

agaccacgcc cccttcggca tcgcgggcgc gcatgaccac ctgcgcgaga ttgagctcca 9060

cgtgccgggc gaagacggcg tagtttcgca ggcgctgaaa gaggtagttg agggtggtgg 9120

cggtgtgttc tgccacgaag aagtacataa cccagcgtcg caacgtggat tcgttgatat 9180

cccccaaggc ctcaaggcgc tccatggcct cgtagaagtc cacggcgaag ttgaaaaact 9240

gggagttgcg cgccgacacg gttaactcct cctccagaag acggatgagc tcggcgacag 9300

tgtcgcgcac ctcgcgctca aaggctacag gggcctcttc ttcttcttca atctcctctt 9360

ccataagggc ctccccttct tcttcttctg gcggcggtgg gggagggggg acacggcggc 9420

gacgacggcg caccgggagg cggtcgacaa agcgctcgat catctccccg cggcgacggc 9480

gcatggtctc ggtgacggcg cggccgttct cgcgggggcg cagttggaag acgccgcccg 9540

tcatgtcccg gttatgggtt ggcggggggc tgccatgcgg cagggatacg gcgctaacga 9600

tgcatctcaa caattgttgt gtaggtactc cgccgccgag ggacctgagc gagtccgcat 9660

cgaccggatc ggaaaacctc tcgagaaagg cgtctaacca gtcacagtcg caaggtaggc 9720

tgagcaccgt ggcgggcggc agcgggcggc ggtcggggtt gtttctggcg gaggtgctgc 9780

tgatgatgta attaaagtag gcggtcttga gacggcggat ggtcgacaga agcaccatgt 9840

ccttgggtcc ggcctgctga atgcgcaggc ggtcggccat gccccaggct tcgttttgac 9900

atcggcgcag gtctttgtag tagtcttgca tgagcctttc taccggcact tcttcttctc 9960

cttcctcttg tcctgcatct cttgcatcta tcgctgcggc ggcggcggag tttggccgta 10020

ggtggcgccc tcttcctccc atgcgtgtga ccccgaagcc cctcatcggc tgaagcaggg 10080

ctaggtcggc gacaacgcgc tcggctaata tggcctgctg cacctgcgtg agggtagact 10140

ggaagtcatc catgtccaca aagcggtggt atgcgcccgt gttgatggtg taagtgcagt 10200

tggccataac ggaccagtta acggtctggt gacccggctg cgagagctcg gtgtacctga 10260

gacgcgagta agccctcgag tcaaatacgt agtcgttgca agtccgcacc aggtactggt 10320

atcccaccaa aaagtgcggc ggcggctggc ggtagagggg ccagcgtagg gtggccgggg 10380

ctccgggggc gagatcttcc aacataaggc gatgatatcc gtagatgtac ctggacatcc 10440

aggtgatgcc ggcggcggtg gtggaggcgc gcggaaagtc gcggacgcgg ttccagatgt 10500

tgcgcagcgg caaaaagtgc tccatggtcg ggacgctctg gccggtcagg cgcgcgcaat 10560

cgttgacgct ctagaccgtg caaaaggaga gcctgtaagc gggcactctt ccgtggtctg 10620

gtggataaat tcgcaagggt atcatggcgg acgaccgggg ttcgagcccc gtatccggcc 10680

gtccgccgtg atccatgcgg ttaccgcccg cgtgtcgaac ccaggtgtgc gacgtcagac 10740

aacgggggag tgctcctttt ggcttccttc caggcgcggc ggctgctgcg ctagcttttt 10800

tggccactgg ccgcgcgcag cgtaagcggt taggctggaa agcgaaagca ttaagtggct 10860

cgctccctgt agccggaggg ttattttcca agggttgagt cgcgggaccc ccggttcgag 10920

tctcggaccg gccggactgc ggcgaacggg ggtttgcctc cccgtcatgc aagaccccgc 10980

ttgcaaattc ctccggaaac agggacgagc cccttttttg cttttcccag atgcatccgg 11040

tgctgcggca gatgcgcccc cctcctcagc agcggcaaga gcaagagcag cggcagacat 11100

gcagggcacc ctcccctcct cctaccgcgt caggaggggc gacatccgcg gttgacgcgg 11160

cagcagatgg tgattacgaa cccccgcggc gccgggcccg gcactacctg gacttggagg 11220

agggcgaggg cctggcgcgg ctaggagcgc cctctcctga gcggcaccca agggtgcagc 11280

tgaagcgtga tacgcgtgag gcgtacgtgc cgcggcagaa cctgtttcgc gaccgcgagg 11340

gagaggagcc cgaggagatg cgggatcgaa agttccacgc agggcgcgag ctgcggcatg 11400

gcctgaatcg cgagcggttg ctgcgcgagg aggactttga gcccgacgcg cgaaccggga 11460

ttagtcccgc gcgcgcacac gtggcggccg ccgacctggt aaccgcatac gagcagacgg 11520

tgaaccagga gattaacttt caaaaaagct ttaacaacca cgtgcgtacg cttgtggcgc 11580

gcgaggaggt ggctatagga ctgatgcatc tgtgggactt tgtaagcgcg ctggagcaaa 11640

acccaaatag caagccgctc atggcgcagc tgttccttat agtgcagcac agcagggaca 11700

acgaggcatt cagggatgcg ctgctaaaca tagtagagcc cgagggccgc tggctgctcg 11760

atttgataaa catcctgcag agcatagtgg tgcaggagcg cagcttgagc ctggctgaca 11820

aggtggccgc catcaactat tccatgctta gcctgggcaa gttttacgcc cgcaagatat 11880

accatacccc ttacgttccc atagacaagg aggtaaagat cgaggggttc tacatgcgca 11940

tggcgctgaa ggtgcttacc ttgagcgacg acctgggcgt ttatcgcaac gagcgcatcc 12000

acaaggccgt gagcgtgagc cggcggcgcg agctcagcga ccgcgagctg atgcacagcc 12060

tgcaaagggc cctggctggc acgggcagcg gcgatagaga ggccgagtcc tactttgacg 12120

cgggcgctga cctgcgctgg gccccaagcc gacgcgccct ggaggcagct ggggccggac 12180

ctgggctggc ggtggcaccc gcgcgcgctg gcaacgtcgg cggcgtggag gaatatgacg 12240

aggacgatga gtacgagcca gaggacggcg agtactaagc ggtgatgttt ctgatcagat 12300

gatgcaagac gcaacggacc cggcggtgcg ggcggcgctg cagagccagc cgtccggcct 12360

taactccacg gacgactggc gccaggtcat ggaccgcatc atgtcgctga ctgcgcgcaa 12420

tcctgacgcg ttccggcagc agccgcaggc caaccggctc tccgcaattc tggaagcggt 12480

ggtcccggcg cgcgcaaacc ccacgcacga gaaggtgctg gcgatcgtaa acgcgctggc 12540

cgaaaacagg gccatccggc ccgacgaggc cggcctggtc tacgacgcgc tgcttcagcg 12600

cgtggctcgt tacaacagcg gcaacgtgca gaccaacctg gaccggctgg tgggggatgt 12660

gcgcgaggcc gtggcgcagc gtgagcgcgc gcagcagcag ggcaacctgg gctccatggt 12720

tgcactaaac gccttcctga gtacacagcc cgccaacgtg ccgcggggac aggaggacta 12780

caccaacttt gtgagcgcac tgcggctaat ggtgactgag acaccgcaaa gtgaggtgta 12840

ccagtctggg ccagactatt ttttccagac cagtagacaa ggcctgcaga ccgtaaacct 12900

gagccaggct ttcaaaaact tgcaggggct gtggggggtg cgggctccca caggcgaccg 12960

cgcgaccgtg tctagcttgc tgacgcccaa ctcgcgcctg ttgctgctgc taatagcgcc 13020

cttcacggac agtggcagcg tgtcccggga cacataccta ggtcacttgc tgacactgta 13080

ccgcgaggcc ataggtcagg cgcatgtgga cgagcatact ttccaggaga ttacaagtgt 13140

cagccgcgcg ctggggcagg aggacacggg cagcctggag gcaaccctaa actacctgct 13200

gaccaaccgg cggcagaaga tcccctcgtt gcacagttta aacagcgagg aggagcgcat 13260

tttgcgctac gtgcagcaga gcgtgagcct taacctgatg cgcgacgggg taacgcccag 13320

cgtggcgctg gacatgaccg cgcgcaacat ggaaccgggc atgtatgcct caaaccggcc 13380

gtttatcaac cgcctaatgg actacttgca tcgcgcggcc gccgtgaacc ccgagtattt 13440

caccaatgcc atcttgaacc cgcactggct accgccccct ggtttctaca ccgggggatt 13500

cgaggtgccc gagggtaacg atggattcct ctgggacgac atagacgaca gcgtgttttc 13560

cccgcaaccg cagaccctgc tagagttgca acagcgcgag caggcagagg cggcgctgcg 13620

aaaggaaagc ttccgcaggc caagcagctt gtccgatcta ggcgctgcgg ccccgcggtc 13680

agatgctagt agcccatttc caagcttgat agggtctctt accagcactc gcaccacccg 13740

cccgcgcctg ctgggcgagg aggagtacct aaacaactcg ctgctgcagc cgcagcgcga 13800

aaaaaacctg cctccggcat ttcccaacaa cgggatagag agcctagtgg acaagatgag 13860

tagatggaag acgtacgcgc aggagcacag ggacgtgcca ggcccgcgcc cgcccacccg 13920

tcgtcaaagg cacgaccgtc agcggggtct ggtgtgggag gacgatgact cggcagacga 13980

cagcagcgtc ctggatttgg gagggagtgg caacccgttt gcgcaccttc gccccaggct 14040

ggggagaatg ttttaaaaaa aaaaaaagca tgatgcaaaa taaaaaactc accaaggcca 14100

tggcaccgag cgttggtttt cttgtattcc ccttagtatg cggcgcgcgg cgatgtatga 14160

ggaaggtcct cctccctcct acgagagtgt ggtgagcgcg gcgccagtgg cggcggcgct 14220

gggttctccc ttcgatgctc ccctggaccc gccgtttgtg cctccgcggt acctgcggcc 14280

taccgggggg agaaacagca tccgttactc tgagttggca cccctattcg acaccacccg 14340

tgtgtacctg gtggacaaca agtcaacgga tgtggcatcc ctgaactacc agaacgacca 14400

cagcaacttt ctgaccacgg tcattcaaaa caatgactac agcccggggg aggcaagcac 14460

acagaccatc aatcttgacg accggtcgca ctggggcggc gacctgaaaa ccatcctgca 14520

taccaacatg ccaaatgtga acgagttcat gtttaccaat aagtttaagg cgcgggtgat 14580

ggtgtcgcgc ttgcctacta aggacaatca ggtggagctg aaatacgagt gggtggagtt 14640

cacgctgccc gagggcaact actccgagac catgaccata gaccttatga acaacgcgat 14700

cgtggagcac tacttgaaag tgggcagaca gaacggggtt ctggaaagcg acatcggggt 14760

aaagtttgac acccgcaact tcagactggg gtttgacccc gtcactggtc ttgtcatgcc 14820

tggggtatat acaaacgaag ccttccatcc agacatcatt ttgctgccag gatgcggggt 14880

ggacttcacc cacagccgcc tgagcaactt gttgggcatc cgcaagcggc aacccttcca 14940

ggagggcttt aggatcacct acgatgatct ggagggtggt aacattcccg cactgttgga 15000

tgtggacgcc taccaggcga gcttgaaaga tgacaccgaa cagggcgggg gtggcgcagg 15060

cggcagcaac agcagtggca gcggcgcgga agagaactcc aacgcggcag ccgcggcaat 15120

gcagccggtg gaggacatga acgatcatgc cattcgcggc gacacctttg ccacacgggc 15180

tgaggagaag cgcgctgagg ccgaagcagc ggccgaagct gccgcccccg ctgcgcaacc 15240

cgaggtcgag aagcctcaga agaaaccggt gatcaaaccc ctgacagagg acagcaagaa 15300

acgcagttac aacctaataa gcaatgacag caccttcacc cagtaccgca gctggtacct 15360

tgcatacaac tacggcgacc ctcagaccgg aatccgctca tggaccctgc tttgcactcc 15420

tgacgtaacc tgcggctcgg agcaggtcta ctggtcgttg ccagacatga tgcaagaccc 15480

cgtgaccttc cgctccacgc gccagatcag caactttccg gtggtgggcg ccgagctgtt 15540

gcccgtgcac tccaagagct tctacaacga ccaggccgtc tactcccaac tcatccgcca 15600

gtttacctct ctgacccacg tgttcaatcg ctttcccgag aaccagattt tggcgcgccc 15660

gccagccccc accatcacca ccgtcagtga aaacgttcct gctctcacag atcacgggac 15720

gctaccgctg cgcaacagca tcggaggagt ccagcgagtg accattactg acgccagacg 15780

ccgcacctgc ccctacgttt acaaggccct gggcatagtc tcgccgcgcg tcctatcgag 15840

ccgcactttt tgagcaagca tgtccatcct tatatcgccc agcaataaca caggctgggg 15900

cctgcgcttc ccaagcaaga tgtttggcgg ggccaagaag cgctccgacc aacacccagt 15960

gcgcgtgcgc gggcactacc gcgcgccctg gggcgcgcac aaacgcggcc gcactgggcg 16020

caccaccgtc gatgacgcca tcgacgcggt ggtggaggag gcgcgcaact acacgcccac 16080

gccgccacca gtgtccacag tggacgcggc cattcagacc gtggtgcgcg gagcccggcg 16140

ctatgctaaa atgaagagac ggcggaggcg cgtagcacgt cgccaccgcc gccgacccgg 16200

cactgccgcc caacgcgcgg cggcggccct gcttaaccgc gcacgtcgca ccggccgacg 16260

ggcggccatg cgggccgctc gaaggctggc cgcgggtatt gtcactgtgc cccccaggtc 16320

caggcgacga gcggccgccg cagcagccgc ggccattagt gctatgactc agggtcgcag 16380

gggcaacgtg tattgggtgc gcgactcggt tagcggcctg cgcgtgcccg tgcgcacccg 16440

ccccccgcgc aactagattg caagaaaaaa ctacttagac tcgtactgtt gtatgtatcc 16500

agcggcggcg gcgcgcaacg aagctatgtc caagcgcaaa atcaaagaag agatgctcca 16560

ggtcatcgcg ccggagatct atggcccccc gaagaaggaa gagcaggatt acaagccccg 16620

aaagctaaag cgggtcaaaa agaaaaagaa agatgatgat gatgaacttg acgacgaggt 16680

ggaactgctg cacgctaccg cgcccaggcg acgggtacag tggaaaggtc gacgcgtaaa 16740

acgtgttttg cgacccggca ccaccgtagt ctttacgccc ggtgagcgct ccacccgcac 16800

ctacaagcgc gtgtatgatg aggtgtacgg cgacgaggac ctgcttgagc aggccaacga 16860

gcgcctcggg gagtttgcct acggaaagcg gcataaggac atgctggcgt tgccgctgga 16920

cgagggcaac ccaacaccta gcctaaagcc cgtaacactg cagcaggtgc tgcccgcgct 16980

tgcaccgtcc gaagaaaagc gcggcctaaa gcgcgagtct ggtgacttgg cacccaccgt 17040

gcagctgatg gtacccaagc gccagcgact ggaagatgtc ttggaaaaaa tgaccgtgga 17100

acctgggctg gagcccgagg tccgcgtgcg gccaatcaag caggtggcgc cgggactggg 17160

cgtgcagacc gtggacgttc agatacccac taccagtagc accagtattg ccaccgccac 17220

agagggcatg gagacacaaa cgtccccggt tgcctcagcg gtggcggatg ccgcggtgca 17280

ggcggtcgct gcggccgcgt ccaagacctc tacggaggtg caaacggacc cgtggatgtt 17340

tcgcgtttca gccccccggc gcccgcgccg ttcgaggaag tacggcgccg ccagcgcgct 17400

actgcccgaa tatgccctac atccttccat tgcgcctacc cccggctatc gtggctacac 17460

ctaccgcccc agaagacgag caactacccg acgccgaacc accactggaa cccgccgccg 17520

ccgtcgccgt cgccagcccg tgctggcccc gatttccgtg cgcagggtgg ctcgcgaagg 17580

aggcaggacc ctggtgctgc caacagcgcg ctaccacccc agcatcgttt aaaagccggt 17640

ctttgtggtt cttgcagata tggccctcac ctgccgcctc cgtttcccgg tgccgggatt 17700

ccgaggaaga atgcaccgta ggaggggcat ggccggccac ggcctgacgg gcggcatgcg 17760

tcgtgcgcac caccggcggc ggcgcgcgtc gcaccgtcgc atgcgcggcg gtatcctgcc 17820

cctccttatt ccactgatcg ccgcggcgat tggcgccgtg cccggaattg catccgtggc 17880

cttgcaggcg cagagacact gattaaaaac aagttgcatg tggaaaaatc aaaataaaaa 17940

gtctggactc tcacgctcgc ttggtcctgt aactattttg tagaatggaa gacatcaact 18000

ttgcgtctct ggccccgcga cacggctcgc gcccgttcat gggaaactgg caagatatcg 18060

gcaccagcaa tatgagcggt ggcgccttca gctggggctc gctgtggagc ggcattaaaa 18120

atttcggttc caccgttaag aactatggca gcaaggcctg gaacagcagc acaggccaga 18180

tgctgaggga taagttgaaa gagcaaaatt tccaacaaaa ggtggtagat ggcctggcct 18240

ctggcattag cggggtggtg gacctggcca accaggcagt gcaaaataag attaacagta 18300

agcttgatcc ccgccctccc gtagaggagc ctccaccggc cgtggagaca gtgtctccag 18360

aggggcgtgg cgaaaagcgt ccgcgccccg acagggaaga aactctggtg acgcaaatag 18420

acgagcctcc ctcgtacgag gaggcactaa agcaaggcct gcccaccacc cgtcccatcg 18480

cgcccatggc taccggagtg ctgggccagc acacacccgt aacgctggac ctgcctcccc 18540

ccgccgacac ccagcagaaa cctgtgctgc caggcccgac cgccgttgtt gtaacccgtc 18600

ctagccgcgc gtccctgcgc cgcgccgcca gcggtccgcg atcgttgcgg cccgtagcca 18660

gtggcaactg gcaaagcaca ctgaacagca tcgtgggtct gggggtgcaa tccctgaagc 18720

gccgacgatg cttctgatag ctaacgtgtc gtatgtgtgt catgtatgcg tccatgtcgc 18780

cgccagagga gctgctgagc cgccgcgcgc ccgctttcca agatggctac cccttcgatg 18840

atgccgcagt ggtcttacat gcacatctcg ggccaggacg cctcggagta cctgagcccc 18900

gggctggtgc agtttgcccg cgccaccgag acgtacttca gcctgaataa caagtttaga 18960

aaccccacgg tggcgcctac gcacgacgtg accacagacc ggtcccagcg tttgacgctg 19020

cggttcatcc ctgtggaccg tgaggatact gcgtactcgt acaaggcgcg gttcacccta 19080

gctgtgggtg ataaccgtgt gctggacatg gcttccacgt actttgacat ccgcggcgtg 19140

ctggacaggg gccctacttt taagccctac tctggcactg cctacaacgc cctggctccc 19200

aagggtgccc caaatccttg cgaatgggat gaagctgcta ctgctcttga aataaaccta 19260

gaagaagagg acgatgacaa cgaagacgaa gtagacgagc aagctgagca gcaaaaaact 19320

cacgtatttg ggcaggcgcc ttattctggt ataaatatta caaaggaggg tattcaaata 19380

ggtgtcgaag gtcaaacacc taaatatgcc gataaaacat ttcaacctga acctcaaata 19440

ggagaatctc agtggtacga aacagaaatt aatcatgcag ctgggagagt cctaaaaaag 19500

actaccccaa tgaaaccatg ttacggttca tatgcaaaac ccacaaatga aaatggaggg 19560

caaggcattc ttgtaaagca acaaaatgga aagctagaaa gtcaagtgga aatgcaattt 19620

ttctcaacta ctgaggcagc cgcaggcaat ggtgataact tgactcctaa agtggtattg 19680

tacagtgaag atgtagatat agaaacccca gacactcata tttcttacat gcccactatt 19740

aaggaaggta actcacgaga actaatgggc caacaatcta tgcccaacag gcctaattac 19800

attgctttta gggacaattt tattggtcta atgtattaca acagcacggg taatatgggt 19860

gttctggcgg gccaagcatc gcagttgaat gctgttgtag atttgcaaga cagaaacaca 19920

gagctttcat accagctttt gcttgattcc attggtgata gaaccaggta cttttctatg 19980

tggaatcagg ctgttgacag ctatgatcca gatgttagaa ttattgaaaa tcatggaact 20040

gaagatgaac ttccaaatta ctgctttcca ctgggaggtg tgattaatac agagactctt 20100

accaaggtaa aacctaaaac aggtcaggaa aatggatggg aaaaagatgc tacagaattt 20160

tcagataaaa atgaaataag agttggaaat aattttgcca tggaaatcaa tctaaatgcc 20220

aacctgtgga gaaatttcct gtactccaac atagcgctgt atttgcccga caagctaaag 20280

tacagtcctt ccaacgtaaa aatttctgat aacccaaaca cctacgacta catgaacaag 20340

cgagtggtgg ctcccgggct agtggactgc tacattaacc ttggagcacg ctggtccctt 20400

gactatatgg acaacgtcaa cccatttaac caccaccgca atgctggcct gcgctaccgc 20460

tcaatgttgc tgggcaatgg tcgctatgtg cccttccaca tccaggtgcc tcagaagttc 20520

tttgccatta aaaacctcct tctcctgccg ggctcataca cctacgagtg gaacttcagg 20580

aaggatgtta acatggttct gcagagctcc ctaggaaatg acctaagggt tgacggagcc 20640

agcattaagt ttgatagcat ttgcctttac gccaccttct tccccatggc ccacaacacc 20700

gcctccacgc ttgaggccat gcttagaaac gacaccaacg accagtcctt taacgactat 20760

ctctccgccg ccaacatgct ctaccctata cccgccaacg ctaccaacgt gcccatatcc 20820

atcccctccc gcaactgggc ggctttccgc ggctgggcct tcacgcgcct taagactaag 20880

gaaaccccat cactgggctc gggctacgac ccttattaca cctactctgg ctctataccc 20940

tacctagatg gaacctttta cctcaaccac acctttaaga aggtggccat tacctttgac 21000

tcttctgtca gctggcctgg caatgaccgc ctgcttaccc ccaacgagtt tgaaattaag 21060

cgctcagttg acggggaggg ttacaacgtt gcccagtgta acatgaccaa agactggttc 21120

ctggtacaaa tgctagctaa ctataacatt ggctaccagg gcttctatat cccagagagc 21180

tacaaggacc gcatgtactc cttctttaga aacttccagc ccatgagccg tcaggtggtg 21240

gatgatacta aatacaagga ctaccaacag gtgggcatcc tacaccaaca caacaactct 21300

ggatttgttg gctaccttgc ccccaccatg cgcgaaggac aggcctaccc tgctaacttc 21360

ccctatccgc ttataggcaa gaccgcagtt gacagcatta cccagaaaaa gtttctttgc 21420

gatcgcaccc tttggcgcat cccattctcc agtaacttta tgtccatggg cgcactcaca 21480

gacctgggcc aaaaccttct ctacgccaac tccgcccacg cgctagacat gacttttgag 21540

gtggatccca tggacgagcc cacccttctt tatgttttgt ttgaagtctt tgacgtggtc 21600

cgtgtgcacc agccgcaccg cggcgtcatc gaaaccgtgt acctgcgcac gcccttctcg 21660

gccggcaacg ccacaacata aagaagcaag caacatcaac aacagctgcc gccatgggct 21720

ccagtgagca ggaactgaaa gccattgtca aagatcttgg ttgtgggcca tattttttgg 21780

gcacctatga caagcgcttt ccaggctttg tttctccaca caagctcgcc tgcgccatag 21840

tcaatacggc cggtcgcgag actgggggcg tacactggat ggcctttgcc tggaacccgc 21900

actcaaaaac atgctacctc tttgagccct ttggcttttc tgaccagcga ctcaagcagg 21960

tttaccagtt tgagtacgag tcactcctgc gccgtagcgc cattgcttct tcccccgacc 22020

gctgtataac gctggaaaag tccacccaaa gcgtacaggg gcccaactcg gccgcctgtg 22080

gactattctg ctgcatgttt ctccacgcct ttgccaactg gccccaaact cccatggatc 22140

acaaccccac catgaacctt attaccgggg tacccaactc catgctcaac agtccccagg 22200

tacagcccac cctgcgtcgc aaccaggaac agctctacag cttcctggag cgccactcgc 22260

cctacttccg cagccacagt gcgcagatta ggagcgccac ttctttttgt cacttgaaaa 22320

acatgtaaaa ataatgtact agagacactt tcaataaagg caaatgcttt tatttgtaca 22380

ctctcgggtg attatttacc cccacccttg ccgtctgcgc cgtttaaaaa tcaaaggggt 22440

tctgccgcgc atcgctatgc gccactggca gggacacgtt gcgatactgg tgtttagtgc 22500

tccacttaaa ctcaggcaca accatccgcg gcagctcggt gaagttttca ctccacaggc 22560

tgcgcaccat caccaacgcg tttagcaggt cgggcgccga tatcttgaag tcgcagttgg 22620

ggcctccgcc ctgcgcgcgc gagttgcgat acacagggtt gcagcactgg aacactatca 22680

gcgccgggtg gtgcacgctg gccagcacgc tcttgtcgga gatcagatcc gcgtccaggt 22740

cctccgcgtt gctcagggcg aacggagtca actttggtag ctgccttccc aaaaagggcg 22800

cgtgcccagg ctttgagttg cactcgcacc gtagtggcat caaaaggtga ccgtgcccgg 22860

tctgggcgtt aggatacagc gcctgcataa aagccttgat ctgcttaaaa gccacctgag 22920

cctttgcgcc ttcagagaag aacatgccgc aagacttgcc ggaaaactga ttggccggac 22980

aggccgcgtc gtgcacgcag caccttgcgt cggtgttgga gatctgcacc acatttcggc 23040

cccaccggtt cttcacgatc ttggccttgc tagactgctc cttcagcgcg cgctgcccgt 23100

tttcgctcgt cacatccatt tcaatcacgt gctccttatt tatcataatg cttccgtgta 23160

gacacttaag ctcgccttcg atctcagcgc agcggtgcag ccacaacgcg cagcccgtgg 23220

gctcgtgatg cttgtaggtc acctctgcaa acgactgcag gtacgcctgc aggaatcgcc 23280

ccatcatcgt cacaaaggtc ttgttgctgg tgaaggtcag ctgcaacccg cggtgctcct 23340

cgttcagcca ggtcttgcat acggccgcca gagcttccac ttggtcaggc agtagtttga 23400

agttcgcctt tagatcgtta tccacgtggt acttgtccat cagcgcgcgc gcagcctcca 23460

tgcccttctc ccacgcagac acgatcggca cactcagcgg gttcatcacc gtaatttcac 23520

tttccgcttc gctgggctct tcctcttcct cttgcgtccg cataccacgc gccactgggt 23580

cgtcttcatt cagccgccgc actgtgcgct tacctccttt gccatgcttg attagcaccg 23640

gtgggttgct gaaacccacc atttgtagcg ccacatcttc tctttcttcc tcgctgtcca 23700

cgattacctc tggtgatggc gggcgctcgg gcttgggaga agggcgcttc tttttcttct 23760

tgggcgcaat ggccaaatcc gccgccgagg tcgatggccg cgggctgggt gtgcgcggca 23820

ccagcgcgtc ttgtgatgag tcttcctcgt cctcggactc gatacgccgc ctcatccgct 23880

tttttggggg cgcccgggga ggcggcggcg acggggacgg ggacgacacg tcctccatgg 23940

ttgggggacg tcgcgccgca ccgcgtccgc gctcgggggt ggtttcgcgc tgctcctctt 24000

cccgactggc catttccttc tcctataggc agaaaaagat catggagtca gtcgagaaga 24060

aggacagcct aaccgccccc tctgagttcg ccaccaccgc ctccaccgat gccgccaacg 24120

cgcctaccac cttccccgtc gaggcacccc cgcttgagga ggaggaagtg attatcgagc 24180

aggacccagg ttttgtaagc gaagacgacg aggaccgctc agtaccaaca gaggataaaa 24240

agcaagacca ggacaacgca gaggcaaacg aggaacaagt cgggcggggg gacgaaaggc 24300

atggcgacta cctagatgtg ggagacgacg tgctgttgaa gcatctgcag cgccagtgcg 24360

ccattatctg cgacgcgttg caagagcgca gcgatgtgcc cctcgccata gcggatgtca 24420

gccttgccta cgaacgccac ctattctcac cgcgcgtacc ccccaaacgc caagaaaacg 24480

gcacatgcga gcccaacccg cgcctcaact tctaccccgt atttgccgtg ccagaggtgc 24540

ttgccaccta tcacatcttt ttccaaaact gcaagatacc cctatcctgc cgtgccaacc 24600

gcagccgagc ggacaagcag ctggccttgc ggcagggcgc tgtcatacct gatatcgcct 24660

cgctcaacga agtgccaaaa atctttgagg gtcttggacg cgacgagaag cgcgcggcaa 24720

acgctctgca acaggaaaac agcgaaaatg aaagtcactc tggagtgttg gtggaactcg 24780

agggtgacaa cgcgcgccta gccgtactaa aacgcagcat cgaggtcacc cactttgcct 24840

acccggcact taacctaccc cccaaggtca tgagcacagt catgagtgag ctgatcgtgc 24900

gccgtgcgca gcccctggag agggatgcaa atttgcaaga acaaacagag gagggcctac 24960

ccgcagttgg cgacgagcag ctagcgcgct ggcttcaaac gcgcgagcct gccgacttgg 25020

aggagcgacg caaactaatg atggccgcag tgctcgttac cgtggagctt gagtgcatgc 25080

agcggttctt tgctgacccg gagatgcagc gcaagctaga ggaaacattg cactacacct 25140

ttcgacaggg ctacgtacgc caggcctgca agatctccaa cgtggagctc tgcaacctgg 25200

tctcctacct tggaattttg cacgaaaacc gccttgggca aaacgtgctt cattccacgc 25260

tcaagggcga ggcgcgccgc gactacgtcc gcgactgcgt ttacttattt ctatgctaca 25320

cctggcagac ggccatgggc gtttggcagc agtgcttgga ggagtgcaac ctcaaggagc 25380

tgcagaaact gctaaagcaa aacttgaagg acctatggac ggccttcaac gagcgctccg 25440

tggccgcgca cctggcggac atcattttcc ccgaacgcct gcttaaaacc ctgcaacagg 25500

gtctgccaga cttcaccagt caaagcatgt tgcagaactt taggaacttt atcctagagc 25560

gctcaggaat cttgcccgcc acctgctgtg cacttcctag cgactttgtg cccattaagt 25620

accgcgaatg ccctccgccg ctttggggcc actgctacct tctgcagcta gccaactacc 25680

ttgcctacca ctctgacata atggaagacg tgagcggtga cggtctactg gagtgtcact 25740

gtcgctgcaa cctatgcacc ccgcaccgct ccctggtttg caattcgcag ctgcttaacg 25800

aaagtcaaat tatcggtacc tttgagctgc agggtccctc gcctgacgaa aagtccgcgg 25860

ctccggggtt gaaactcact ccggggctgt ggacgtcggc ttaccttcgc aaatttgtac 25920

ctgaggacta ccacgcccac gagattaggt tctacgaaga ccaatcccgc ccgcctaatg 25980

cggagcttac cgcctgcgtc attacccagg gccacattct tggccaattg caagccatca 26040

acaaagcccg ccaagagttt ctgctacgaa agggacgggg ggtttacttg gacccccagt 26100

ccggcgagga gctcaaccca atccccccgc cgccgcagcc ctatcagcag cagccgcggg 26160

cccttgcttc ccaggatggc acccaaaaag aagctgcagc tgccgccgcc acccacggac 26220

gaggaggaat actgggacag tcaggcagag gaggttttgg acgaggagga ggaggacatg 26280

atggaagact gggagagcct agacgaggaa gcttccgagg tcgaagaggt gtcagacgaa 26340

acaccgtcac cctcggtcgc attcccctcg ccggcgcccc agaaatcggc aaccggttcc 26400

agcatggcta caacctccgc tcctcaggcg ccgccggcac tgcccgttcg ccgacccaac 26460

cgtagatggg acaccactgg aaccagggcc ggtaagtcca agcagccgcc gccgttagcc 26520

caagagcaac aacagcgcca aggctaccgc tcatggcgcg ggcacaagaa cgccatagtt 26580

gcttgcttgc aagactgtgg gggcaacatc tccttcgccc gccgctttct tctctaccat 26640

cacggcgtgg ccttcccccg taacatcctg cattactacc gtcatctcta cagcccatac 26700

tgcaccggcg gcagcggcag caacagcagc ggccacacag aagcaaaggc gaccggatag 26760

caagactctg acaaagccca agaaatccac agcggcggca gcagcaggag gaggagcgct 26820

gcgtctggcg cccaacgaac ccgtatcgac ccgcgagctt agaaacagga tttttcccac 26880

tctgtatgct atatttcaac agagcagggg ccaagaacaa gagctgaaaa taaaaaacag 26940

gtctctgcga tccctcaccc gcagctgcct gtatcacaaa agcgaagatc agcttcggcg 27000

cacgctggaa gacgcggagg ctctcttcag taaatactgc gcgctgactc ttaaggacta 27060

gtttcgcgcc ctttctcaaa tttaagcgcg aaaactacgt catctccagc ggccacaccc 27120

ggcgccagca cctgttgtca gcgccattat gagcaaggaa attcccacgc cctacatgtg 27180

gagttaccag ccacaaatgg gacttgcggc tggagctgcc caagactact caacccgaat 27240

aaactacatg agcgcgggac cccacatgat atcccgggtc aacggaatac gcgcccaccg 27300

aaaccgaatt ctcctggaac aggcggctat taccaccaca cctcgtaata accttaatcc 27360

ccgtagttgg cccgctgccc tggtgtacca ggaaagtccc gctcccacca ctgtggtact 27420

tcccagagac gcccaggccg aagttcagat gactaactca ggggcgcagc ttgcgggcgg 27480

ctttcgtcac agggtgcggt cgcccgggca gggtataact cacctgacaa tcagagggcg 27540

aggtattcag ctcaacgacg agtcggtgag ctcctcgctt ggtctccgtc cggacgggac 27600

atttcagatc ggcggcgccg gccgctcttc attcacgcct cgtcaggcaa tcctaactct 27660

gcagacctcg tcctctgagc cgcgctctgg aggcattgga actctgcaat ttattgagga 27720

gtttgtgcca tcggtctact ttaacccctt ctcgggacct cccggccact atccggatca 27780

atttattcct aactttgacg cggtaaagga ctcggcggac ggctacgact gaatgttaag 27840

tggagaggca gagcaactgc gcctgaaaca cctggtccac tgtcgccgcc acaagtgctt 27900

tgcccgcgac tccggtgagt tttgctactt tgaattgccc gaggatcata tcgagggccc 27960

ggcgcacggc gtccggctta ccgcccaggg agagcttgcc cgtagcctga ttcgggagtt 28020

tacccagcgc cccctgctag ttgagcggga caggggaccc tgtgttctca ctgtgatttg 28080

caactgtcct aaccctggat tacatcaaga tctttgttgc catctctgtg ctgagtataa 28140

taaatacaga aattaaaata tactggggct cctatcgcca tcctgtaaac gccaccgtct 28200

tcacccgccc aagcaaacca aggcgaacct tacctggtac ttttaacatc tctccctctg 28260

tgatttacaa cagtttcaac ccagacggag tgagtctacg agagaacctc tccgagctca 28320

gctactccat cagaaaaaac accaccctcc ttacctgccg ggaacgtacg atggatatag 28380

aagatgaaga aaacatgagt tccagcagca ctgatgtgaa ggaaaaccgc aatctggaca 28440

acgtgtcccc caaggatggc agcacacctg ggcctggcga gggctctcag ctctccaatg 28500

ggggtggtgg tggccccggc agaaagcggc ccctggagga gggcagcaat ggccactcca 28560

agtaccgcct gaagaaaagg aggaaaacac cagggcccgt cctccccaag aacgccctga 28620

tgcagctgaa tgagatcaag cctggtttgc agtacacact cctgtcccag actgggcccg 28680

tgcacgcgcc tttgtttgtc atgtctgtgg aggtgaatgg ccaggttttt gaggctctgg 28740

gtcccacaaa gaaaaaggca aaactccatg ctgctgagaa ggccttgagg tctttcgttc 28800

agtttcctaa tgcctctgag gcccacctgg ccatggggag gaccctgtct gtcaacacgg 28860

acttcacatc tgaccaggcc gacttccctg acacgctctt caatggtttt gaaactcctg 28920

acaaggcgga gcctcccttt tacgtgggct ccaatgggga tgactccttc agttccagcg 28980

gggacctcag cttgtctgct tccccggtgc ctgccagcct agcccagcct cctctccctg 29040

tcttaccacc attcccaccc ccgagtggga agaatcccgt gatgatcttg aacgaactgc 29100

gcccaggact caagtatgac ttcctctccg agagcgggga gagccatgcc aagagcttcg 29160

tcatgtctgt ggtcgtggat ggtcagttct ttgaaggctc ggggagaaac aagaagcttg 29220

ccaaggcccg ggctgcgcag tctgccctgg ccgccatttt taacttgcac ttggatcaga 29280

cgccatctcg ccagcctatt cccagtgagg gtcttcagct gcatttaccg caggttttag 29340

ctgacgctgt ctcacgcctg gtcctgggta agtttggtga cctgaccgac aacttctcct 29400

cccctcacgc tcgcagaaaa gtgctggctg gagtcgtcat gacaacaggc acagatgtta 29460

aagatgccaa ggtgataagt gtttctacag gaacaaaatg tattaatggt gaatacatga 29520

gtgatcgtgg ccttgcatta aatgactgcc atgcagaaat aatatctcgg agatccttgc 29580

tcagatttct ttatacacaa cttgagcttt acttaaataa caaagatgat caaaaaagat 29640

ccatctttca gaaatcagag cgaggggggt ttaggctgaa ggagaatgtc cagtttcatc 29700

tgtacatcag cacctctccc tgtggagatg ccagaatctt ctcaccacat gagccaatcc 29760

tggaagaacc agcagataga cacccaaatc gtaaagcaag aggacagcta cggaccaaaa 29820

tagagtctgg tgaggggacg attccagtgc gctccaatgc gagcatccaa acgtgggacg 29880

gggtgctgca aggggagcgg ctgctcacca tgtcctgcag tgacaagatt gcacgctgga 29940

acgtggtggg catccaggga tccctgctca gcattttcgt ggagcccatt tacttctcga 30000

gcatcatcct gggcagcctt taccacgggg accacctttc cagggccatg taccagcgga 30060

tctccaacat agaggacctg ccacctctct acaccctcaa caagcctttg ctcagtggca 30120

tcagcaatgc agaagcacgg cagccaggga aggcccccaa cttcagtgtc aactggacgg 30180

taggcgactc cgctattgag gtcatcaacg ccacgactgg gaaggatgag ctgggccgcg 30240

cgtcccgcct gtgtaagcac gcgttgtact gtcgctggat gcgtgtgcac ggcaaggttc 30300

cctcccactt actacgctcc aagattacca agcccaacgt gtaccatgag tccaagctgg 30360

cggcaaagga gtaccaggcc gccaaggcgc gtctgttcac agccttcatc aaggcggggc 30420

tgggggcctg ggtggagaag cccaccgagc aggaccagtt ctcactcacg ccctgacaat 30480

tgactctatg tgggatatgc tccagcgcta caaccttgaa gtcaggcttc ctggatgtca 30540

gcatctgact ttggccagca cctgtcccgc ggatttgttc cagtccaact acagcgaccc 30600

accctaacag agatgaccaa cacaaccaac gcggccgccg ctaccggact tacatctacc 30660

acaaatacac cccaagtttc tgcctttgtc aataactggg ataacttggg catgtggtgg 30720

ttctccatag cgcttatgtt tgtatgcctt attattatgt ggctcatctg ctgcctaaag 30780

cgcaaacgcg cccgaccacc catctatagt cccatcattg tgctacaccc aaacaatgat 30840

ggaatccata gattggacgg actgaaacac atgttctttt ctcttacagt atgattaaat 30900

gagacatgat tcctcgagtt tttatattac tgacccttgt tgcgcttttt tgtgcgtgct 30960

ccacattggc tgcggtttct cacatcgaag tagactgcat tccagccttc acagtctatt 31020

tgctttacgg atttgtcacc ctcacgctca tctgcagcct catcactgtg gtcatcgcct 31080

ttatccagtg cattgactgg gtctgtgtgc gctttgcata tctcagacac catccccagt 31140

acagggacag gactatagct gagcttctta gaattcttta attatgaaat ttactgtgac 31200

ttttctgctg attatttgca ccctatctgc gttttgttcc ccgacctcca agcctcaaag 31260

acatatatca tgcagattca ctcgtatatg gaatattcca agttgctaca atgaaaaaag 31320

cgatctttcc gaagcctggt tatatgcaat catctctgtt atggtgttct gcagtaccat 31380

cttagcccta gctatatatc cctaccttga cattggctgg aacgcaatag atgccatgaa 31440

ccacccaact ttccccgcgc ccgctatgct tccactgcaa caagttgttg ccggcggctt 31500

tgtcccagcc aatcagcctc gcccaccttc tcccaccccc actgaaatca gctactttaa 31560

tctaacagga ggagatgact gacaccctag atctagaaat ggacggaatt attacagagc 31620

agcgcctgct agaaagacgc agggcagcgg ccgagcaaca gcgcatgaat caagagctcc 31680

aagacatggt taacttgcac cagtgcaaaa ggggtatctt ttgtctcgta aagcaggcca 31740

aagtcaccta cgacagtaat accaccggac accgccttag ctacaagttg ccaaccaagc 31800

gtcagaaatt ggtggtcatg gtgggagaaa agcccattac cataactcag cactcggtag 31860

aaaccgaagg ctgcattcac tcaccttgtc aaggacctga ggatctctgc acccttatta 31920

agaccctgtg cggtctcaaa gatcttattc cctttaacta ataaaaaaaa ataataaagc 31980

atcacttact taaaatcagt tagcaaattt ctgtccagtt tattcagcag cacctccttg 32040

ccctcctccc agctctggta ttgcagcttc ctcctggctg caaactttct ccacaatcta 32100

aatggaatgt cagtttcctc ctgttcctgt ccatccgcac ccactatctt catgttgttg 32160

cagatgaagc gcgcaagacc gtctgaagat accttcaacc ccgtgtatcc atatgacacg 32220

gaaaccggtc ctccaactgt gccttttctt actcctccct ttgtatcccc caatgggttt 32280

caagagagtc cccctggggt actctctttg cgcctatccg aacctctagt tacctccaat 32340

ggcatgcttg cgctcaaaat gggcaacggc ctctctctgg acgaggccgg caaccttacc 32400

tcccaaaatg taaccactgt gagcccacct ctcaaaaaaa ccaagtcaaa cataaacctg 32460

gaaatatctg cacccctcac agttacctca gaagccctaa ctgtggctgc cgccgcacct 32520

ctaatggtcg cgggcaacac actcaccatg caatcacagg ccccgctaac cgtgcacgac 32580

tccaaactta gcattgccac ccaaggaccc ctcacagtgt cagaaggaaa gctagccctg 32640

caaacatcag gccccctcac caccaccgat agcagtaccc ttactatcac tgcctcaccc 32700

cctctaacta ctgccactgg tagcttgggc attgacttga aagagcccat ttatacacaa 32760

aatggaaaac taggactaaa gtacggggct cctttgcatg taacagacga cctaaacact 32820

ttgaccgtag caactggtcc aggtgtgact attaataata cttccttgca aactaaagtt 32880

actggagcct tgggttttga ttcacaaggc aatatgcaac ttaatgtagc aggaggacta 32940

aggattgatt ctcaaaacag acgccttata cttgatgtta gttatccgtt tgatgctcaa 33000

aaccaactaa atctaagact aggacagggc cctcttttta taaactcagc ccacaacttg 33060

gatattaact acaacaaagg cctttacttg tttacagctt caaacaattc caaaaagctt 33120

gaggttaacc taagcactgc caaggggttg atgtttgacg ctacagccat agccattaat 33180

gcaggagatg ggcttgaatt tggttcacct aatgcaccaa acacaaatcc cctcaaaaca 33240

aaaattggcc atggcctaga atttgattca aacaaggcta tggttcctaa actaggaact 33300

ggccttagtt ttgacagcac aggtgccatt acagtaggaa acaaaaataa tgataagcta 33360

accctatgga caggtccaaa accagaagcc aactgcataa ttgaatacgg gaaacaaaac 33420

ccagatagca aactaacttt aatccttgta aaaaatggag gaattgttaa tggatatgta 33480

acgctaatgg gagcctcaga ctacgttaac accttattta aaaacaaaaa tgtctccatt 33540

aatgtagaac tatactttga tgccactggt catatattac cagactcatc ttctcttaaa 33600

acagatctag aactaaaata caagcaaacc gctgacttta gtgcaagagg ttttatgcca 33660

agtactacag cgtatccatt tgtccttcct aatgcgggaa cacataatga aaattatatt 33720

tttggtcaat gctactacaa agcaagcgat ggtgcccttt ttccgttgga agttactgtt 33780

atgcttaata aacgcctgcc agatagtcgc acatcctatg ttatgacttt tttatggtcc 33840

ttgaatgctg gtctagctcc agaaactact caggcaaccc tcataacctc cccatttacc 33900

ttttcctata ttagagaaga tgactaataa actctaaaga atcgtttgtg ttatgtttca 33960

acgtgtttat ttttcaattg cagaaaattt caagtcattt ttcattcagt agtatagccc 34020

caccaccaca tagcttatac agatcaccgt accttaatca aactcacaga accctagtat 34080

tcaacctgcc acctccctcc caacacacag agtacacagt cctttctccc cggctggcct 34140

taaaaagcat catatcatgg gtaacagaca tattcttagg tgttatattc cacacggttt 34200

cctgtcgagc caaacgctca tcagtgatat taataaactc cccgggcagc tcacttaagt 34260

tcatgtcgct gtccagctgc tgagccacag gctgctgtcc aacttgcggt tgcttaacgg 34320

gcggcgaagg agaagtccac gcctacatgg gggtagagtc ataatcgtgc atcaggatag 34380

ggcggtggtg ctgcagcagc gcgcgaataa actgctgccg ccgccgctcc gtcctgcagg 34440

aatacaacat ggcagtggtc tcctcagcga tgattcgcac cgcccgcagc ataaggcgcc 34500

ttgtcctccg ggcacagcag cgcaccctga tctcacttaa atcagcacag taactgcagc 34560

acagcaccac aatattgttc aaaatcccac agtgcaaggc gctgtatcca aagctcatgg 34620

cggggaccac agaacccacg tggccatcat accacaagcg caggtagatt aagtggcgac 34680

ccctcataaa cacgctggac ataaacatta cctcttttgg catgttgtaa ttcaccacct 34740

cccggtacca tataaacctc tgattaaaca tggcgccatc caccaccatc ctaaaccagc 34800

tggccaaaac ctgcccgccg gctatacact gcagggaacc gggactggaa caatgacagt 34860

ggagagccca ggactcgtaa ccatggatca tcatgctcgt catgatatca atgttggcac 34920

aacacaggca cacgtgcata cacttcctca ggattacaag ctcctcccgc gttagaacca 34980

tatcccaggg aacaacccat tcctgaatca gcgtaaatcc cacactgcag ggaagacctc 35040

gcacgtaact cacgttgtgc attgtcaaag tgttacattc gggcagcagc ggatgatcct 35100

ccagtatggt agcgcgggtt tctgtctcaa aaggaggtag acgatcccta ctgtacggag 35160

tgcgccgaga caaccgagat cgtgttggtc gtagtgtcat gccaaatgga acgccggacg 35220

tagtcatatt tcctgaagca aaaccaggtg cgggcgtgac aaacagatct gcgtctccgg 35280

tctcgccgct tagatcgctc tgtgtagtag ttgtagtata tccactctct caaagcatcc 35340

aggcgccccc tggcttcggg ttctatgtaa actccttcat gcgccgctgc cctgataaca 35400

tccaccaccg cagaataagc cacacccagc caacctacac attcgttctg cgagtcacac 35460

acgggaggag cgggaagagc tggaagaacc atgttttttt ttttattcca aaagattatc 35520

caaaacctca aaatgaagat ctattaagtg aacgcgctcc cctccggtgg cgtggtcaaa 35580

ctctacagcc aaagaacaga taatggcatt tgtaagatgt tgcacaatgg cttccaaaag 35640

gcaaacggcc ctcacgtcca agtggacgta aaggctaaac ccttcagggt gaatctcctc 35700

tataaacatt ccagcacctt caaccatgcc caaataattc tcatctcgcc accttctcaa 35760

tatatctcta agcaaatccc gaatattaag tccggccatt gtaaaaatct gctccagagc 35820

gccctccacc ttcagcctca agcagcgaat catgattgca aaaattcagg ttcctcacag 35880

acctgtataa gattcaaaag cggaacatta acaaaaatac cgcgatcccg taggtccctt 35940

cgcagggcca gctgaacata atcgtgcagg tctgcacgga ccagcgcggc cacttccccg 36000

ccaggaacca tgacaaaaga acccacactg attatgacac gcatactcgg agctatgcta 36060

accagcgtag ccccgatgta agcttgttgc atgggcggcg atataaaatg caaggtgctg 36120

ctcaaaaaat caggcaaagc ctcgcgcaaa aaagaaagca catcgtagtc atgctcatgc 36180

agataaaggc aggtaagctc cggaaccacc acagaaaaag acaccatttt tctctcaaac 36240

atgtctgcgg gtttctgcat aaacacaaaa taaaataaca aaaaaacatt taaacattag 36300

aagcctgtct tacaacagga aaaacaaccc ttataagcat aagacggact acggccatgc 36360

cggcgtgacc gtaaaaaaac tggtcaccgt gattaaaaag caccaccgac agctcctcgg 36420

tcatgtccgg agtcataatg taagactcgg taaacacatc aggttgattc acatcggtca 36480

gtgctaaaaa gcgaccgaaa tagcccgggg gaatacatac ccgcaggcgt agagacaaca 36540

ttacagcccc cataggaggt ataacaaaat taataggaga gaaaaacaca taaacacctg 36600

aaaaaccctc ctgcctaggc aaaatagcac cctcccgctc cagaacaaca tacagcgctt 36660

ccacagcggc agccataaca gtcagcctta ccagtaaaaa agaaaaccta ttaaaaaaac 36720

accactcgac acggcaccag ctcaatcagt cacagtgtaa aaaagggcca agtgcagagc 36780

gagtatatat aggactaaaa aatgacgtaa cggttaaagt ccacaaaaaa cacccagaaa 36840

accgcacgcg aacctacgcc cagaaacgaa agccaaaaaa cccacaactt cctcaaatcg 36900

tcacttccgt tttcccacgt tacgtcactt cccattttaa gaaaactaca attcccaaca 36960

catacaagtt actccgccct aaaacctacg tcacccgccc cgttcccacg ccccgcgcca 37020

cgtcacaaac tccaccccct cattatcata ttggcttcaa tccaaaataa ggtatattat 37080

tgatgatgtt aat 37093

<210> 4
<211> 8
<212> PRT
<213> Artificial Sequence

<220>
<223> A binding site for Rb protein in E1A

<400> 4
Leu Thr Cys His Glu Ala Gly Phe
1               5

<210> 5
<211> 45
<212> DNA
<213> Artificial Sequence

<220>
<223> CR2-D24

<400> 5
cttgtaccgg aggtgatcga tccacccagt gacgacgagg atgaa 45

<210> 6
<211> 1764
<212> DNA
<213> Artificial Sequence

<220>
<223> Ad5/3 fiber

<400> 6

atgaagcgcg caagaccgtc tgaagatacc ttcaaccccg tgtatccata tgacacggaa 60

accggtcctc caactgtgcc ttttcttact cctccctttg tatcccccaa tgggtttcaa 120

gagagtcccc ctggggtact ctctttgcgc ctatccgaac ctctagttac ctccaatggc 180

atgcttgcgc tcaaaatggg caacggcctc tctctggacg aggccggcaa ccttacctcc 240

caaaatgtaa ccactgtgag cccacctctc aaaaaaacca agtcaaacat aaacctggaa 300

atatctgcac ccctcacagt tacctcagaa gccctaactg tggctgccgc cgcacctcta 360

atggtcgcgg gcaacacact caccatgcaa tcacaggccc cgctaaccgt gcacgactcc 420

aaacttagca ttgccaccca aggacccctc acagtgtcag aaggaaagct agccctgcaa 480

acatcaggcc ccctcaccac caccgatagc agtaccctta ctatcactgc ctcaccccct 540

ctaactactg ccactggtag cttgggcatt gacttgaaag agcccattta tacacaaaat 600

ggaaaactag gactaaagta cggggctcct ttgcatgtaa cagacgacct aaacactttg 660

accgtagcaa ctggtccagg tgtgactatt aataatactt ccttgcaaac taaagttact 720

ggagccttgg gttttgattc acaaggcaat atgcaactta atgtagcagg aggactaagg 780

attgattctc aaaacagacg ccttatactt gatgttagtt atccgtttga tgctcaaaac 840

caactaaatc taagactagg acagggccct ctttttataa actcagccca caacttggat 900

attaactaca acaaaggcct ttacttgttt acagcttcaa acaattccaa aaagcttgag 960

gttaacctaa gcactgccaa ggggttgatg tttgacgcta cagccatagc cattaatgca 1020

ggagatgggc ttgaatttgg ttcacctaat gcaccaaaca caaatcccct caaaacaaaa 1080

attggccatg gcctagaatt tgattcaaac aaggctatgg ttcctaaact aggaactggc 1140

cttagttttg acagcacagg tgccattaca gtaggaaaca aaaataatga taagctaacc 1200

ctatggacag gtccaaaacc agaagccaac tgcataattg aatacgggaa acaaaaccca 1260

gatagcaaac taactttaat ccttgtaaaa aatggaggaa ttgttaatgg atatgtaacg 1320

ctaatgggag cctcagacta cgttaacacc ttatttaaaa acaaaaatgt ctccattaat 1380

gtagaactat actttgatgc cactggtcat atattaccag actcatcttc tcttaaaaca 1440

gatctagaac taaaatacaa gcaaaccgct gactttagtg caagaggttt tatgccaagt 1500

actacagcgt atccatttgt ccttcctaat gcgggaacac ataatgaaaa ttatattttt 1560

ggtcaatgct actacaaagc aagcgatggt gccctttttc cgttggaagt tactgttatg 1620

cttaataaac gcctgccaga tagtcgcaca tcctatgtta tgactttttt atggtccttg 1680

aatgctggtc tagctccaga aactactcag gcaaccctca taacctcccc atttaccttt 1740

tcctatatta gagaagatga ctaa 1764

<210> 7
<211> 909

<212> DNA
<213> Artificial Sequence

<220>
<223> ICOSL

<400> 7
atgcggctgg gcagtcctgg actgctcttc ctgctcttca gcagccttcg agctgatact 60

caggagaagg aagtcagagc gatggtaggc agcgacgtgg agctcagctg cgcttgccct 120

gaaggaagcc gttttgactt aaatgatgtt tacgtatatt ggcaaaccag tgagtcgaaa 180

accgtggtga cctaccacat cccacagaac agctccttgg aaaacgtgga cagccgctac 240

cggaaccgag ccctgatgtc accggccggc atgctgcggg gcgacttctc cctgcgcttg 300

ttcaacgtca ccccccagga cgagcagaag tttcactgcc tggtgttgag ccaatccctg 360

ggattccagg aggttttgag cgttgaggtt acactgcatg tggcagcaaa cttcagcgtg 420

cccgtcgtca gcgcccccca cagcccctcc caggatgagc tcaccttcac gtgtacatcc 480

ataaacggct accccaggcc caacgtgtac tggatcaata agacggacaa cagcctgctg 540

gaccaggctc tgcagaatga caccgtcttc ttgaacatgc ggggcttgta tgacgtggtc 600

agcgtgctga ggatcgcacg gacccccagc gtgaacattg gctgctgcat agagaacgtg 660

cttctgcagc agaacctgac tgtcggcagc cagacaggaa atgacatcgg agagagagac 720

aagatcacag agaatccagt cagtaccggc gagaaaaacg cggccacgtg gagcatcctg 780

gctgtcctgt gcctgcttgt ggtcgtggcg gtggccatag gctgggtgtg cagggaccga 840

tgcctccaac acagctatgc aggtgcctgg gctgtgagtc cggagacaga gctcactggc 900

cacgtttga 909

<210> 8
<211> 45
<212> DNA
<213> Artificial Sequence

<220>
<223> CR2-D24

<400> 8
cttgtaccgg aggtgatcga tccacccagt gacgacgagg atgaa 45

<210> 9
<211> 1764
<212> DNA
<213> Artificial Sequence

<220>
<223> Ad5/3 fiber

<400> 9
atgaagcgcg caagaccgtc tgaagatacc ttcaaccccg tgtatccata tgacacggaa 60

accggtcctc caactgtgcc ttttcttact cctccctttg tatcccccaa tgggtttcaa 120

gagagtcccc ctggggtact ctctttgcgc ctatccgaac ctctagttac ctccaatggc 180

atgcttgcgc tcaaaatggg caacggcctc tctctggacg aggccggcaa ccttacctcc 240

caaaatgtaa ccactgtgag cccacctctc aaaaaaacca agtcaaacat aaacctggaa 300

atatctgcac ccctcacagt tacctcagaa gccctaactg tggctgccgc cgcacctcta 360

atggtcgcgg gcaacacact caccatgcaa tcacaggccc cgctaaccgt gcacgactcc 420

aaacttagca ttgccaccca aggacccctc acagtgtcag aaggaaagct agccctgcaa 480

acatcaggcc ccctcaccac caccgatagc agtaccctta ctatcactgc ctcaccccct 540

ctaactactg ccactggtag cttgggcatt gacttgaaag agcccattta tacacaaaat 600

ggaaaactag gactaaagta cggggctcct ttgcatgtaa cagacgacct aaacactttg 660

accgtagcaa ctggtccagg tgtgactatt aataatactt ccttgcaaac taaagttact 720

ggagccttgg gttttgattc acaaggcaat atgcaactta atgtagcagg aggactaagg 780

attgattctc aaaacagacg ccttatactt gatgttagtt atccgtttga tgctcaaaac 840

caactaaatc taagactagg acagggccct ctttttataa actcagccca caacttggat 900

attaactaca acaaaggcct ttacttgttt acagcttcaa acaattccaa aaagcttgag 960

gttaacctaa gcactgccaa ggggttgatg tttgacgcta cagccatagc cattaatgca 1020

ggagatgggc ttgaatttgg ttcacctaat gcaccaaaca caaatcccct caaaacaaaa 1080

attggccatg gcctagaatt tgattcaaac aaggctatgg ttcctaaact aggaactggc 1140

cttagttttg acagcacagg tgccattaca gtaggaaaca aaaataatga taagctaacc 1200

ctatggacag gtccaaaacc agaagccaac tgcataattg aatacgggaa acaaaaccca 1260

gatagcaaac taactttaat ccttgtaaaa aatggaggaa ttgttaatgg atatgtaacg 1320

ctaatgggag cctcagacta cgttaacacc ttatttaaaa acaaaaatgt ctccattaat 1380

gtagaactat actttgatgc cactggtcat atattaccag actcatcttc tcttaaaaca 1440

gatctagaac taaaatacaa gcaaaccgct gactttagtg caagaggttt tatgccaagt 1500

actacagcgt atccatttgt ccttcctaat gcgggaacac ataatgaaaa ttatattttt 1560

ggtcaatgct actacaaagc aagcgatggt gccctttttc cgttggaagt tactgttatg 1620

cttaataaac gcctgccaga tagtcgcaca tcctatgtta tgactttttt atggtccttg 1680

aatgctggtc tagctccaga aactactcag gcaaccctca taacctcccc atttaccttt 1740

tcctatatta gagaagatga ctaa 1764

<210> 10

<211> 2106
<212> DNA
<213> Artificial Sequence

<220>
<223> ADA

<400> 10
atggatatag aagatgaaga aaacatgagt tccagcagca ctgatgtgaa ggaaaaccgc 60

aatctggaca acgtgtcccc caaggatggc agcacacctg ggcctggcga gggctctcag 120

ctctccaatg ggggtggtgg tggccccggc agaaagcggc ccctggagga gggcagcaat 180

ggccactcca agtaccgcct gaagaaaagg aggaaaacac cagggcccgt cctccccaag 240

aacgccctga tgcagctgaa tgagatcaag cctggtttgc agtacacact cctgtcccag 300

actgggcccg tgcacgcgcc tttgtttgtc atgtctgtgg aggtgaatgg ccaggttttt 360

gagggctctg gtcccacaaa gaaaaaggca aaactccatg ctgctgagaa ggccttgagg 420

tctttcgttc agtttcctaa tgcctctgag gcccacctgg ccatggggag gaccctgtct 480

gtcaacacgg acttcacatc tgaccaggcc gacttccctg acacgctctt caatggtttt 540

gaaactcctg acaaggcgga gcctcccttt tacgtgggct ccaatgggga tgactccttc 600

agttccagcg gggacctcag cttgtctgct tccccggtgc ctgccagcct agcccagcct 660

cctctccctg tcttaccacc attcccaccc ccgagtggga agaatcccgt gatgatcttg 720

aacgaactgc gcccaggact caagtatgac ttcctctccg agagcgggga gagccatgcc 780

aagagcttcg tcatgtctgt ggtcgtggat ggtcagttct ttgaaggctc ggggagaaac 840

aagaagcttg ccaaggcccg ggctgcgcag tctgccctgg ccgccatttt taacttgcac 900

ttggatcaga cgccatctcg ccagcctatt cccagtgagg gtcttcagct gcatttaccg 960

caggttttag ctgacgctgt ctcacgcctg gtcctgggta agtttggtga cctgaccgac 1020

aacttctcct cccctcacgc tcgcagaaaa gtgctggctg gagtcgtcat gacaacaggc 1080

acagatgtta aagatgccaa ggtgataagt gtttctacag gaacaaaatg tattaatggt 1140

gaatacatga gtgatcgtgg ccttgcatta aatgactgcc atgcagaaat aatatctcgg 1200

agatccttgc tcagatttct ttatacacaa cttgagcttt acttaaataa caaagatgat 1260

caaaaaagat ccatctttca gaaatcagag cgaggggggt ttaggctgaa ggagaatgtc 1320

cagtttcatc tgtacatcag cacctctccc tgtggagatg ccagaatctt ctcaccacat 1380

gagccaatcc tggaagaacc agcagataga cacccaaatc gtaaagcaag aggacagcta 1440

cggaccaaaa tagagtctgg tgaggggacg attccagtgc gctccaatgc gagcatccaa 1500

acgtgggacg gggtgctgca aggggagcgg ctgctcacca tgtcctgcag tgacaagatt 1560

gcacgctgga acgtggtggg catccaggga tccctgctca gcattttcgt ggagcccatt 1620

tacttctcga gcatcatcct gggcagcctt taccacgggg accacctttc cagggccatg 1680

taccagcgga tctccaacat agaggacctg ccacctctct acaccctcaa caagcctttg 1740

ctcagtggca tcagcaatgc agaagcacgg cagccaggga aggcccccaa cttcagtgtc 1800

aactggacgg taggcgactc cgctattgag gtcatcaacg ccacgactgg gaaggatgag 1860

ctgggccgcg cgtcccgcct gtgtaagcac gcgttgtact gtcgctggat gcgtgtgcac 1920

ggcaaggttc cctcccactt actacgctcc aagattacca agcccaacgt gtaccatgag 1980

tccaagctgg cggcaaagga gtaccaggcc gccaaggcgc gtctgttcac agccttcatc 2040

aaggcggggc tgggggcctg ggtggagaag cccaccgagc aggaccagtt ctcactcacg 2100

ccctga 2106

<210> 11
<211> 45
<212> DNA
<213> Artificial Sequence

<220>
<223> CR2-D24

<400> 11
cttgtaccgg aggtgatcga tccacccagt gacgacgagg atgaa 45

<210> 12
<211> 1764
<212> DNA
<213> Artificial Sequence

<220>
<223> Ad5/3 fiber

<400> 12
atgaagcgcg caagaccgtc tgaagatacc ttcaaccccg tgtatccata tgacacggaa 60

accggtcctc caactgtgcc ttttcttact cctccctttg tatcccccaa tgggtttcaa 120

gagagtcccc ctggggtact ctctttgcgc ctatccgaac ctctagttac ctccaatggc 180

atgcttgcgc tcaaaatggg caacggcctc tctctggacg aggccggcaa ccttacctcc 240

caaaatgtaa ccactgtgag cccacctctc aaaaaaacca agtcaaacat aaacctggaa 300

atatctgcac ccctcacagt tacctcagaa gccctaactg tggctgccgc cgcacctcta 360

atggtcgcgg gcaacacact caccatgcaa tcacaggccc cgctaaccgt gcacgactcc 420

aaacttagca ttgccaccca aggacccctc acagtgtcag aaggaaagct agccctgcaa 480

acatcaggcc ccctcaccac caccgatagc agtaccctta ctatcactgc ctcaccccct 540

ctaactactg ccactggtag cttgggcatt gacttgaaag agcccattta tacacaaaat 600

ggaaaactag gactaaagta cggggctcct ttgcatgtaa cagacgacct aaacactttg 660

accgtagcaa ctggtccagg tgtgactatt aataatactt ccttgcaaac taaagttact 720

ggagccttgg gttttgattc acaaggcaat atgcaactta atgtagcagg aggactaagg 780

attgattctc aaaacagacg ccttatactt gatgttagtt atccgtttga tgctcaaaac 840

caactaaatc taagactagg acagggccct ctttttataa actcagccca caacttggat 900

attaactaca acaaaggcct ttacttgttt acagcttcaa acaattccaa aaagcttgag 960

gttaacctaa gcactgccaa ggggttgatg tttgacgcta cagccatagc cattaatgca 1020

ggagatgggc ttgaatttgg ttcacctaat gcaccaaaca caaatcccct caaaacaaaa 1080

attggccatg gcctagaatt tgattcaaac aaggctatgg ttcctaaact aggaactggc 1140

cttagttttg acagcacagg tgccattaca gtaggaaaca aaaataatga taagctaacc 1200

ctatggacag gtccaaaacc agaagccaac tgcataattg aatacgggaa acaaaaccca 1260

gatagcaaac taactttaat ccttgtaaaa aatggaggaa ttgttaatgg atatgtaacg 1320

ctaatgggag cctcagacta cgttaacacc ttatttaaaa acaaaaatgt ctccattaat 1380

gtagaactat actttgatgc cactggtcat atattaccag actcatcttc tcttaaaaca 1440

gatctagaac taaaatacaa gcaaaccgct gactttagtg caagaggttt tatgccaagt 1500

actacagcgt atccatttgt ccttcctaat gcgggaacac ataatgaaaa ttatattttt 1560

ggtcaatgct actacaaagc aagcgatggt gccctttttc cgttggaagt tactgttatg 1620

cttaataaac gcctgccaga tagtcgcaca tcctatgtta tgactttttt atggtccttg 1680

aatgctggtc tagctccaga aactactcag gcaaccctca taacctcccc atttaccttt 1740

tcctatatta gagaagatga ctaa 1764

<210> 13
<211> 534
<212> DNA
<213> Artificial Sequence

<220>
<223> CMV promoter/enhancer

<400> 13
ctagctagtt attaatagta atcaattacg gggtcattag ttcatagccc atatatggag 60

ttccgcgtta cataacttac ggtaaatggc ccgcctggct gaccgcccaa cgacccccgc 120

ccattgacgt caataatgac gtatgttccc atagtaacgc caatagggac tttccattga 180

cgtcaatggg tggagtattt acggtaaact gcccacttgg cagtacatca agtgtatcat 240

atgccaagtc cgccccctat tgacgtcaat gacggtaaat ggcccgcctg gcattatgcc 300

cagtacatga ccttacggga ctttcctact tggcagtaca tctacgtatt agtcatcgct 360

attaccatgg tgatgcggtt ttggcagtac accaatgggc gtggatagcg gtttgactca 420

cggggatttc caagtctcca ccccattgac gtcaatggga gtttgttttg gcaccaaaat 480

caacgggact ttccaaaatg tcgtaatacc cccgccccgt tgacgcaaat gggc 534

<210> 14
<211> 26
<212> DNA
<213> Artificial Sequence

<220>
<223> CUO

<400> 14
caaacagacc agattgtctg tttgtt 26

<210> 15
<211> 909
<212> DNA
<213> Artificial Sequence

<220>
<223> ICOSL

<400> 15
atgcggctgg gcagtcctgg actgctcttc ctgctcttca gcagccttcg agctgatact 60

caggagaagg aagtcagagc gatggtaggc agcgacgtgg agctcagctg cgcttgccct 120

gaaggaagcc gttttgactt aaatgatgtt tacgtatatt ggcaaaccag tgagtcgaaa 180

accgtggtga cctaccacat cccacagaac agctccttgg aaaacgtgga cagccgctac 240

cggaaccgag ccctgatgtc accggccggc atgctgcggg gcgacttctc cctgcgcttg 300

ttcaacgtca ccccccagga cgagcagaag tttcactgcc tggtgttgag ccaatccctg 360

ggattccagg aggttttgag cgttgaggtt acactgcatg tggcagcaaa cttcagcgtg 420

cccgtcgtca gcgcccccca cagcccctcc caggatgagc tcaccttcac gtgtacatcc 480

ataaacggct accccaggcc caacgtgtac tggatcaata agacggacaa cagcctgctg 540

gaccaggctc tgcagaatga caccgtcttc ttgaacatgc ggggcttgta tgacgtggtc 600

agcgtgctga ggatcgcacg gacccccagc gtgaacattg gctgctgcat agagaacgtg 660

cttctgcagc agaacctgac tgtcggcagc cagacaggaa atgacatcgg agagagagac 720

aagatcacag agaatccagt cagtaccggc gagaaaaacg cggccacgtg gagcatcctg 780

gctgtcctgt gcctgcttgt ggtcgtggcg gtggccatag gctgggtgtg cagggaccga 840

tgcctccaac acagctatgc aggtgcctgg gctgtgagtc cggagacaga gctcactggc 900

cacgtttga 909

<210> 16

<211> 572
<212> DNA
<213> Artificial Sequence

<220>
<223> IRES

<400> 16
cccctctccc tcccccccct aacgttactg gccgaagccg cttggaataa ggccggtgtg 60

cgtttgtcta tatgttattt tccaccatat tgccgtcttt tggcaatgtg agggcccgga 120

aacctggccc tgtcttcttg acgagcattc ctaggggtct ttcccctctc gccaaaggaa 180

tgcaaggtct gttgaatgtc gtgaaggaag cagttcctct ggaagcttct tgaagacaaa 240

caacgtctgt agcgaccctt tgcaggcagc ggaacccccc acctggcgac aggtgcctct 300

gcggccaaaa gccacgtgta taagatacac ctgcaaaggc ggcacaaccc cagtgccacg 360

ttgtgagttg gatagttgtg gaaagagtca aatggctctc ctcaagcgta ttcaacaagg 420

ggctgaagga tgcccagaag gtaccccatt gtatgggatc tgatctgggg cctcggtgca 480

catgctttac atgtgtttag tcgaggttaa aaaaacgtct aggccccccg aaccacgggg 540

acgtggtttt cctttgaaaa acacgatgat aa 572

<210> 17
<211> 2101
<212> DNA
<213> Artificial Sequence

<220>
<223> ADARB1

<400> 17
tatagaagat gaagaaaaca tgagttccag cagcactgat gtgaaggaaa accgcaatct 60

ggacaacgtg tcccccaagg atggcagcac acctgggcct ggcgagggct ctcagctctc 120

caatgggggt ggtggtggcc ccggcagaaa gcggcccctg gaggagggca gcaatggcca 180

ctccaagtac cgcctgaaga aaaggaggaa aacaccaggg cccgtcctcc ccaagaacgc 240

cctgatgcag ctgaatgaga tcaagcctgg tttgcagtac acactcctgt cccagactgg 300

gcccgtgcac gcgcctttgt ttgtcatgtc tgtggaggtg aatggccagg tttttgaggg 360

ctctggtccc acaaagaaaa aggcaaaact ccatgctgct gagaaggcct tgaggtcttt 420

cgttcagttt cctaatgcct ctgaggccca cctggccatg gggaggaccc tgtctgtcaa 480

cacggacttc acatctgacc aggccgactt ccctgacacg ctcttcaatg gttttgaaac 540

tcctgacaag gcggagcctc ccttttacgt gggctccaat ggggatgact ccttcagttc 600

cagcggggac ctcagcttgt ctgcttcccc ggtgcctgcc agcctagccc agcctcctct 660

ccctgtctta ccaccattcc caccccccag tgggaagaat cccgtgatga tcttgaacga 720

actgcgccca ggactcaagt atgacttcct ctccgagagc ggggagagcc atgccaagag 780

cttcgtcatg tctgtggtcg tggatggtca gttctttgaa ggctcgggga gaaacaagaa 840

gcttgccaag gcccgggctg cgcagtctgc cctggccgcc atttttaact tgcacttgga 900

tcagacgcca tctcgccagc ctattcccag tgagggtctt cagctgcatt taccgcaggt 960

tttagctgac gctgtctcac gcctggtcct gggtaagttt ggtgacctga ccgacaactt 1020

ctcctcccct cacgctcgca gaaaagtgct ggctggagtc gtcatgacaa caggcacaga 1080

tgttaaagat gccaaggtga taagtgtttc tacaggaaca aaatgtatta atggtgaata 1140

catgagtgat cgtggccttg cattaaatga ctgccatgca gaaataatat ctcggagatc 1200

cttgctcaga tttctttata cacaacttga gctttactta aataacaaag atgatcaaaa 1260

aagatccatc tttcagaaat cagagcgagg ggggtttagg ctgaaggaga atgtccagtt 1320

tcatctgtac atcagcacct ctccctgtgg agatgccaga atcttctcac cacatgagcc 1380

aatcctggaa gaaccagcag atagacaccc aaatcgtaaa gcaagaggac agctacggac 1440

caaaatagag tctggtgagg ggacgattcc agtgcgctcc aatgcgagca tccaaacgtg 1500

ggacggggtg ctgcaagggg agcggctgct caccatgtcc tgcagtgaca agattgcacg 1560

ctggaacgtg gtgggcatcc agggatccct gctcagcatt ttcgtggagc ccatttactt 1620

ctcgagcatc atcctgggca gcctttacca cggggaccac ctttccaggg ccatgtacca 1680

gcggatctcc aacatagagg acctgccacc tctctacacc ctcaacaagc ctttgctcag 1740

tggcatcagc aatgcagaag cacggcagcc agggaaggcc cccaacttca gtgtcaactg 1800

gacggtaggc gactccgcta ttgaggtcat caacgccacg actgggaagg atgagctggg 1860

ccgcgcgtcc cgcctgtgta agcacgcgtt gtactgtcgc tggatgcgtg tgcacggcaa 1920

ggttccctcc cacttactac gctccaagat taccaagccc aacgtgtacc atgagtccaa 1980

gctggcggca aaggagtacc aggccgccaa ggcgcgtctg ttcacagcct tcatcaaggc 2040

ggggctgggg gcctgggtgg agaagcccac cgagcaggac cagttctcac tcacgccctg 2100

a 2101

<210> 18
<211> 122
<212> DNA
<213> Artificial Sequence

<220>
<223> SV40 poly(A) signal

<400> 18
aacttgttta ttgcagctta taatggttac aaataaagca atagcatcac aaatttcaca 60

aataaagcat ttttttcact gcattctagt tgtggtttgt ccaaactcat caatgtatct 120

ta                                                                 122

<210> 19
<211> 61
<212> DNA
<213> Artificial Sequence

<220>
<223> Primer 288-17

<400> 19
acaccgggac cgatccagcc tccgcgggcc gccaccatgc ggctgggcag tcctggactg      60

c                                                                   61

<210> 20
<211> 25
<212> DNA
<213> Artificial Sequence

<220>
<223> Primer 288-41

<400> 20
tcaaacgtgg ccagtgagct ctgtc                                         25

<210> 21
<211> 40
<212> DNA
<213> Artificial Sequence

<220>
<223> Primer 288-18

<400> 21
   agctcactgg ccacgtttga cccctctccc tcccccccct                          40

<210> 22
<211> 40
<212> DNA
<213> Artificial Sequence

<220>
<223> Primer 288-19

<400> 22
tcttcatctt ctatatccat gtcgactcta gaggatcccg                         40

<210> 23
<211> 40
<212> DNA
<213> Artificial Sequence

<220>
<223> Primer 288-20

<400> 23
agttctcact cacgccctga aacttgttta ttgcagctta                                40


<210> 24
<211> 26
<212> DNA
<213> Artificial Sequence


<220>
<223> Primer 288-21

<400> 24
tagttaaagg gaataagatc ggtacc                                              26

## Description

### FIELD OF THE DISCLOSURE

The present invention relates to cancer therapies. More specifically, the present invention relates to oncolytic adenoviral vectors and cells and pharmaceutical compositions comprising said vectors. The present invention also relates to a use of said vectors in the manufacture of a medicament for treating cancer in a subject and a method of treating cancer in a subject. Furthermore, the present invention relates to methods of producing a member of the B7 family of costimulatory ligands and adenosine deaminase (ADA) in a cell and increasing anti-tumor effect and induction of specific immune response in a subject, as well as uses of the oncolytic adenoviral vector of the invention for producing transgenes in a cell and increasing anti-tumor effect and generation of specific immune response in a subject.

### BACKGROUND OF THE DISCLOSURE

During the last twenty years gene transfer technology has been under intensive examination. The aim of cancer gene therapies is to introduce a therapeutic gene into a tumor cell. These therapeutic genes introduced to a target cell may for example correct mutated genes, suppress active oncogenes or generate additional properties to the cell. Suitable exogenous therapeutic genes include but are not limited to immunotherapeutic, anti-angiogenic, chemoprotective and "suicide" genes, and they can be introduced to a cell by utilizing modified virus vectors or non-viral methods including electroporation, gene gun and lipid or polymer coatings.

Requirements of optimal viral vectors include an efficient capability to find specific target cells and express the viral genome in the target cells. Furthermore, optimal vectors have to stay active in the target tissues or cells. All these properties of viral vectors have been developed during the last decades and for example retroviral, adenoviral and adeno-associated viral vectors have been widely studied in biomedicine.

To further improve tumor penetration and local amplification of the anti-tumor effect, selectively oncolytic agents, e.g. conditionally replicating adenoviruses, have been constructed. Oncolytic adenoviruses are a promising tool for treatment of cancers. Tumor cells are killed by oncolytic adenoviruses due to a replication of the virus in a tumor cell, the last phase of the replication resulting in a release of thousands of virions into the surrounding tumor tissues for effective tumor penetration and vascular re-infection. Tumor cells allow replication of the virus while normal cells are spared due to engineered changes in the virus genome, which prevent replication in non-tumor cells.

In addition to replication mediated cell killing, oncolytic adenoviruses can also be armed with different therapeutic transgenes. This approach combines the advantages of conventional gene delivery with the potency of replication competent agents. One goal of arming viruses is induction of an immune reaction towards the cells that allow virus replication. Virus replication alone, although immunogenic, is normally not enough to induce effective anti-tumor immunity. To strengthen induction of therapeutic immunity, viruses can be armed with stimulatory proteins such as cytokines for facilitation of the introduction of tumor antigens to antigen presenting cells such as dendritic cells, and their stimulation and/or maturation. Introduction of immunotherapeutic genes into tumor cells and furthermore, translation of the proteins, leads to activation of the immune response and efficient destruction of tumor cells. The most relevant immune cells in this regard are natural killer celis (NK), T helper cells and cytotoxic CD8+ T-cells.

More than 50 different serotypes of adenoviruses have been found in humans. Adenovirus serotype 5 (Ad5) is known to cause respiratory diseases and it is the most common serotype studied in the field of gene therapy. In the first Ad5 vectors E1 and/or E3 regions were deleted enabling insertion of foreign DNA to the vectors. Furthermore, deletions of other regions as well as further mutations have provided extra properties to viral vectors, indeed, various modifications of adenoviruses have been suggested for achieving efficient antitumor effects.

ICOS - Inducible T-cell co-stimulator is an immune checkpoint protein. Is a CD28-superfamily costimulatory molecule that is expressed on activated T cells. Plays an important role in cell-cell signalling, immune responses and regulation of cell proliferation (Hutloff et al., 1999).

The B7 family of immune-regulatory ligands comprises inducible costimulator ligand (ICOSL), programmed death-1 ligand (PD-L1), programmed death-2 ligand (PD-L2), B7-H3, and B7-H4). In humans, cell surface expression of ICOS-L has been described on B cells, dendritic cells, monocytes/macrophages, and T cells. Instead, ICOS-L binds to ICOS, a T cell-specific costimulatory molecule homologous to CD28 and CTLA-4. ICOS-ligand costimulates Th1 and Th2 cytokine secretion by memory CD4+ T cells (Khayyamian et al., 2002).

ADA (adenosine deaminase (also known as adenosine aminohydrolase, or ADA) is an enzyme involved in purine metabolism. It is needed for the breakdown of adenosine from food and for the turnover of nucleic acids in tissues; including 2 isoforms of ADA: ADA1 and ADA2.

ICOS/ICOSL pathway is necessary for the optimal therapeutic effect of anti-check point inhibitors, thus implicating this pathway as a target of future combinatorial strategies to improve the efficacy of anti-cancer therapies. It has been shown the ICOS/ICOSL pathway is critical for optimal anti-tumour activity of anti-CTLA-4 (Fu et al., 2011).

EP2379586B1 discloses an oncolytic adenoviral vector comprising an adenovirus serotype 5 (Ad5) nucleic acid backbone, a 24 bp deletion (D24) in the Rb binding constant region 2 of adenoviral E1 and a nucleic acid sequence encoding a granulocyte-macrophage colony-stimulating factor (GM-CSF) in the place of the deleted gp19k/6.7K in the adenoviral E3 region. WO2016126608A1 relates to adenoviruses. Exemplary oncolytic viruses include a conditionally replicating oncolytic serotype 5 adenovirus (Ad5) in which human E2F-1 promoter drives expression of the essential E1a viral genes, thereby restricting viral replication and cytotoxicity to Rb pathway-defective tumor cells and DNX-2401 (formerly named D24-RGD), which is an adenovirus that has been engineered to replicate selectively in retinoblastoma (Rb)-pathway deficient cells and to infect cells that express certain RGD-binding integrins more efficiently.

Still, more efficient and accurate gene transfer as well as increased specificity and sufficient tumor killing ability of gene therapies are warranted. Safety records of therapeutic vectors must also be excellent. The present invention provides a cancer therapeutic tool with these aforementioned properties by utilizing both oncolytic and immunotherapeutic properties of adenoviruses in a novel and inventive way.

### BRIEF DESCRIPTION OF THE DISCLOSURE

The object of the invention is to provide novel methods and means for achieving the above-mentioned properties of adenoviruses and thus, solving the problems of conventional cancer therapies. More specifically, the invention provides novel methods and means for gene therapy.

Adenosine - Derivatives of adenosine are widely found in nature and play an important role in biochemical processes (ATP, ADP). Multiple immunosuppressive mechanisms impede anti-tumour immunity. Among them, the accumulation of extracellular adenosine is a potent and widespread strategy exploited by tumours to escape immunosurveillance through the activation of purinergic receptors. In tumours, this pathway is hijacked and hinders anti-tumour immunity, promoting cancer progression (Allard et al., 2016). Adenosine signalling downregulates functions of most immune effector cells but enhances expansion and activity of immune cells responsible for suppression of anti-tumour immune responses. Adenosine facilitates tumour escape from the immune control. It can be considered for therapeutics targeting the adenosinergic pathway for benefiting cancer patients (Muller-Haegele et al., 2014).

Adenosine deaminase (ADA), is an enzyme involved in purine metabolism that can inhibit tumour progression by metabolizing adenosine (Kutryb-Zajac et al., 2018, Londoño-R et al., 2018). This indicates a potential use of the inhibitors of adenosine pathway for cancer immunotherapy (Ohta, 2016).

The present inventors hypothesized that the combined expression of a member of the B7 family of costimulatory ligands and ADA would have beneficial effects for tumor eradication. More specifically, the present inventors hypothesized that the double transgene construct (TGX-11) coding for ICOSL and ADA1 utilizes the properties of oncolytic viruses - oncolyses due to the fact of 24bp deletion in E1A region for cancer cell restricted replication (Kuryk et al., 2016) and also enhanced tropism due to fiber knob modifications (chimeric virus5/3 knob) (Kuryk et al., 2018). In addition to oncolytic properties, due to the presence of two transgenes the construct can exhibit enhanced anti-tumour effect by increasing T cell activation by to the interactions of ICOSL with its receptor on T cells and reduction of the suppressive tumour microenvironment by the inhibition of adenosine (by ADA), leading to enhance cross presentation of tumour antigens, priming of cancer specific T cells and modulation of tumour microenvironment. The construct can improve infiltration with activated T cells in both virus-injected and distant tumours. Also, immunomodulation with an oncolytic virus TGX-11 expressing ICOSL and ADA1 can be an effective strategy to drive systemic efficacy of immune checkpoint blockade.

Produced transgenes along with the oncolytic properties of the construct can result in synergistic anti-cancer effect and improved clinical outcome.

The double transgene virus was found to be a more efficient model compared to that where both transgenes were encoded by separate viruses but used together. The double transgene simplifies the production process especially in clinical studies over combined viruses expressing transgenes separately.

The present application describes construction of recombinant viral vectors, methods related to the vectors, and their use in tumor cells lines and animal models.

The present invention relates to an oncolytic adenoviral vector comprising an adenovirus serotype 5 (Ad5) nucleic acid backbone, a 24 bp deletion (D24) in the Rb binding constant region 2 of adenoviral E1 and a nucleic acid sequence encoding a member of the B7 family of costimulatory ligands and ADA transgenes. In addition, there may be an IRES in the place of the deleted 2.7 kbp in the adenoviral E3 region.

The present invention further relates to a cell comprising the adenoviral vector of the invention.

The present invention also relates to a pharmaceutical composition comprising the adenoviral vector of the invention.

The present invention also relates to a use of the adenoviral vector of the invention in the manufacture of a medicament for treating cancer in a subject.

The present invention also relates to a method of treating cancer in a subject, wherein the method comprises administration of the vector or pharmaceutical composition of the invention to a subject.

Furthermore, the present invention also relates to a method of producing a member of the B7 family of costimulatory ligands and ADA in a cell, wherein the method comprises: a) carrying a vehicle comprising an oncolytic adenoviral vector of the invention to a cell, and b) expressing a member of the B7 family of costimulatory ligands and ADA of said vector in the cell.

Furthermore, the present invention also relates to a method of increasing tumor specific immune response in a subject, wherein the method comprises: a) carrying a vehicle comprising an oncolytic adenoviral vector of the invention to a target cell or tissue, b) expressing a member of the B7 family of costimulatory ligands and ADA of said vector in the cell, and c) increasing amount of cytotoxic T cells and/or natural killer cells in said target cell or tissue.

Still, the present invention also relates to a use of the oncolytic adenoviral vector of the invention for producing a member of the B7 family of costimulatory ligands and ADA in a cell.

Still, the present invention also relates to a use of the oncolytic adenoviral vector of the invention for increasing tumor specific immune response in a subject.

The present invention provides a tool for treatment of cancers, some of which are refractory to current approaches. Also, restrictions regarding tumor types suitable for treatment remain few compared to many other treatments. In fact, all solid tumors may be treated with the proposed invention. Larger tumors by mass and more complex tumors can be treated by using the present invention. The treatment can be given intratumorally, intracavitary, intravenously and in a combination of these. The approach can give systemic efficacy despite local injection. The approach can also eradicate cells proposed as tumor initiating ("cancer stem cells").

### BRIEF DESCRIPTION OF THE DRAWINGS

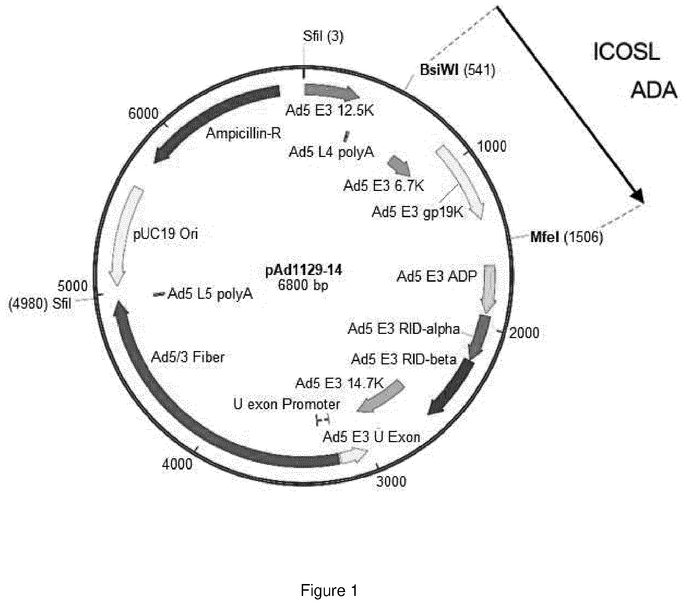

Figure 1 illustrates the shuttle plasmid constructions for the shuttle plasmids for pTGX-04/pTGX-05.

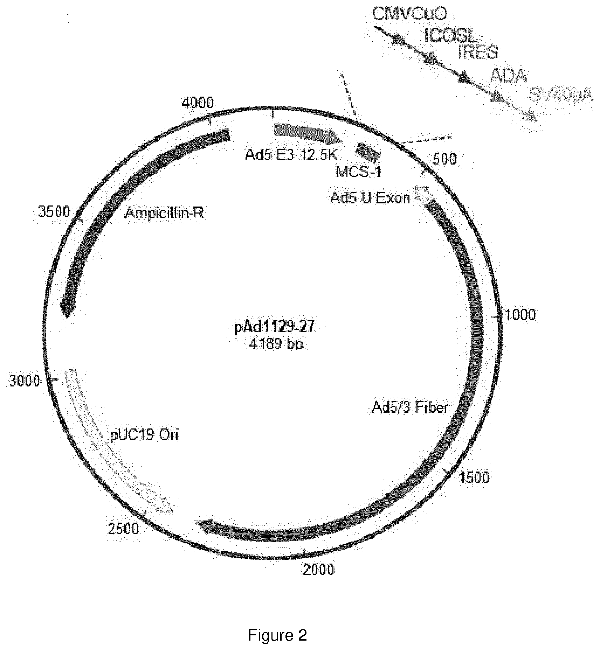

Figure 2 illustrates the shuttle plasmid constructions for the shuttle plasmid for pTGX-11 (CMV(CuO)-ICOSL-IRES-ADA-SV40pA).

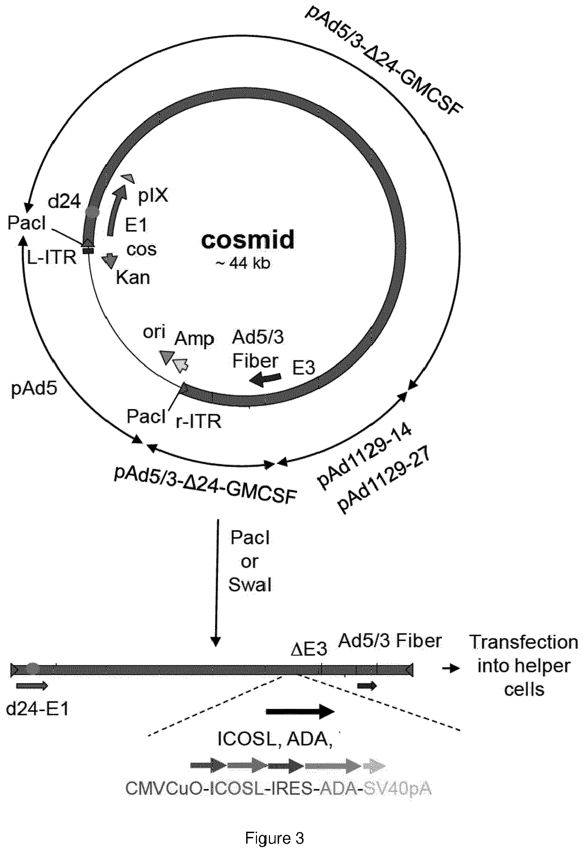

Figure 3 illustrates the cosmid constructions for TGX-04 (AdV5/3-D24-ICOSL), TGX-05 (AdV5/3-D24-ADA), TGX-11 (AdV5/3-D24-CMV(CuO)-ICOSL-IRES-ADA-SV40pA).

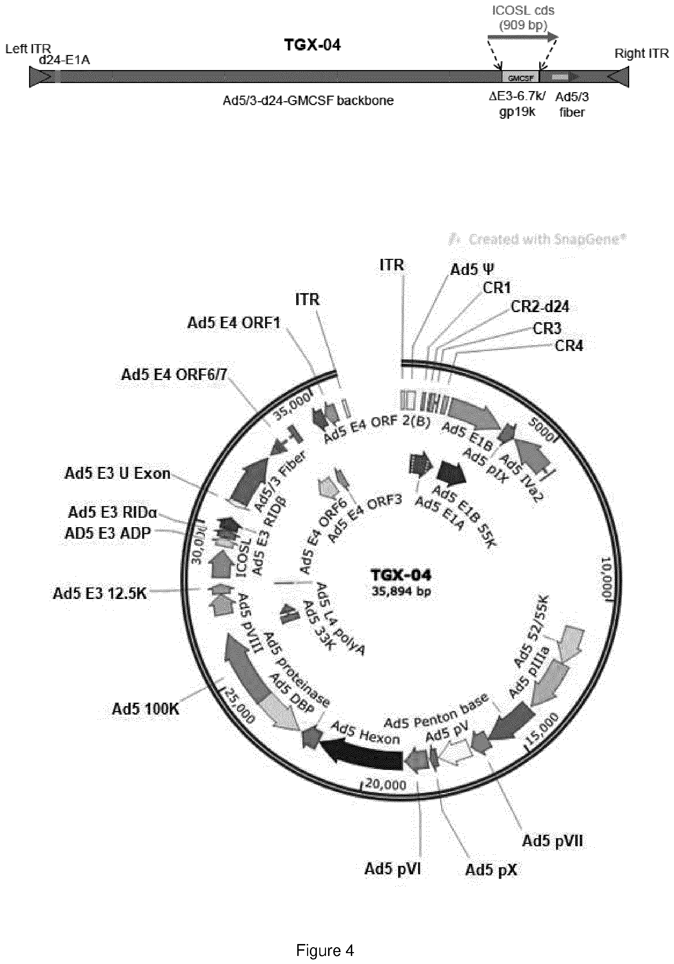

Figure 4 shows TGX-04 (AdV5/3-D24-ICOSL) virus structure. ICOSL (functional expression cassette, location: 28,371 - 29,279bp) inserted into E3 region. The virus contains the 24-bp deletion in E1A CR2 (E1A Conserved Region 2 (CR2) with a 24-bp deletion that removed the strongest of two binding sites in E1A for Rb protein (LTCHEAGF), 907 - 951bp), expresses the Ad5/3 hybrid fiber (location: 30,967 - 32,730bp). Nucleotide sequence enclosed in attachment (TGX-04).

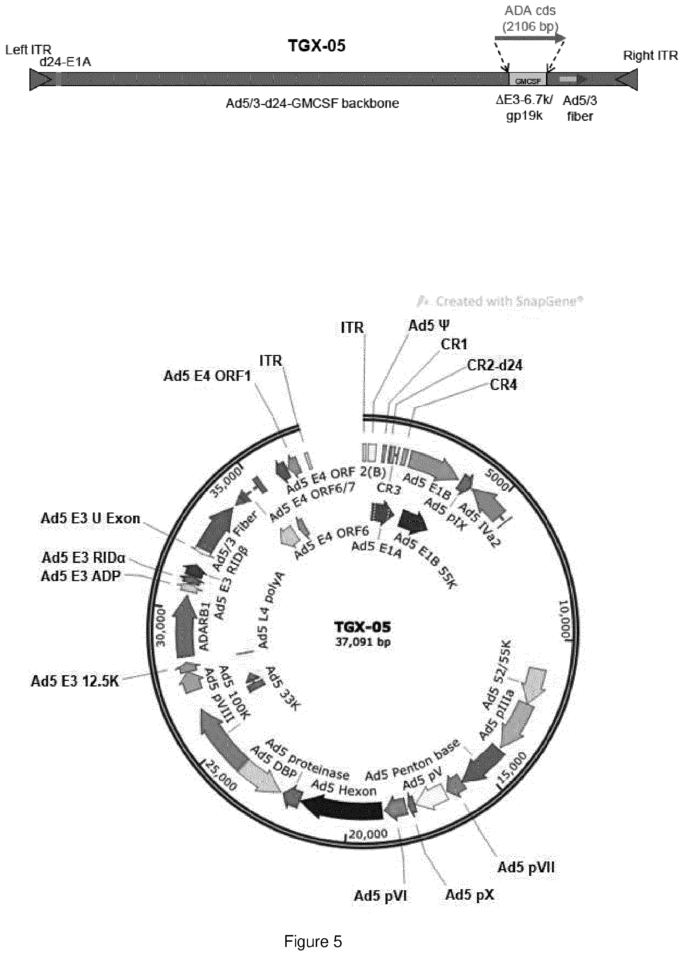

Figure 5 shows TGX-05 (AdV5/3-D24-ADA) virus structure. ADA (functional expression cassette, location: 28,371 - 30,476bp) inserted into E3 region. The virus contains the 24-bp deletion in E1A CR2 (E1A Conserved Region 2 (CR2) with a 24-bp deletion that removed the strongest of two binding sites in E1A for Rb protein (LTCHEAGF), location: 907-951bp), expresses the Ad5/3 hybrid fiber (location: 32,164 - 33,927bp). Nucleotide sequence enclosed in attachment (TGX-05).

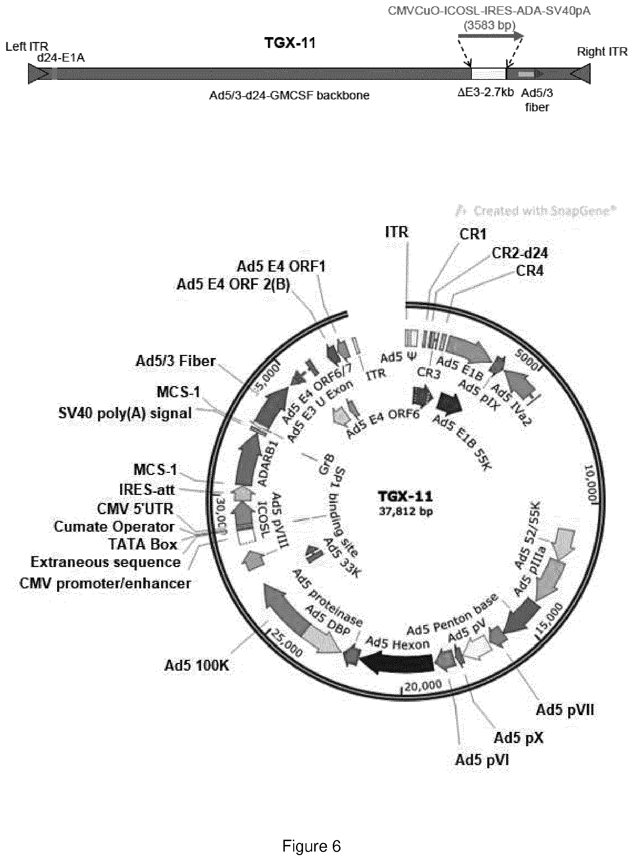

Figure 6 shows TGX-11 (AdV5/3-D24-CMV(CuO)-ICOSL-IRES-ADA-SV40pA) virus structure. ICOSL (functional expression cassette, location: 28,904 - 29,812bp) -IRES (29,813 - 30,384bp) -ADA (functional expression cassette, location: 31,207 - 31,950) - SV40pA expression under the control of CMV promoter (28,172 - 28,705) and CuO inserted into E3 region. The virus contains the 24-bp deletion in E1A CR2 (E1A Conserved Region 2 (CR2) with a 24-bp deletion that removed the strongest of two binding sites in E1A for Rb protein (LTCHEAGF), location: 907-951bp), expresses the Ad5/3 hybrid fiber (location: 32,885 - 34,648bp). Nucleotide sequence enclosed in attachment (TGX-13).

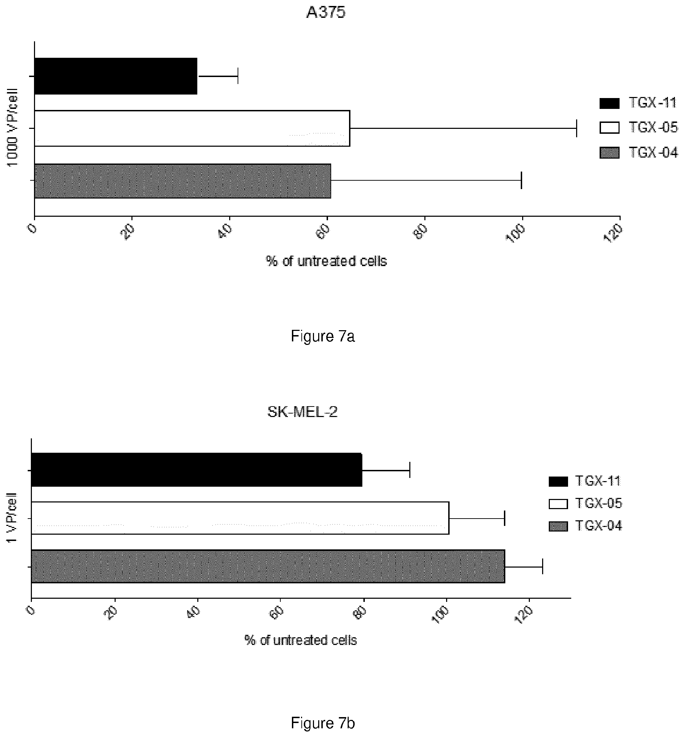

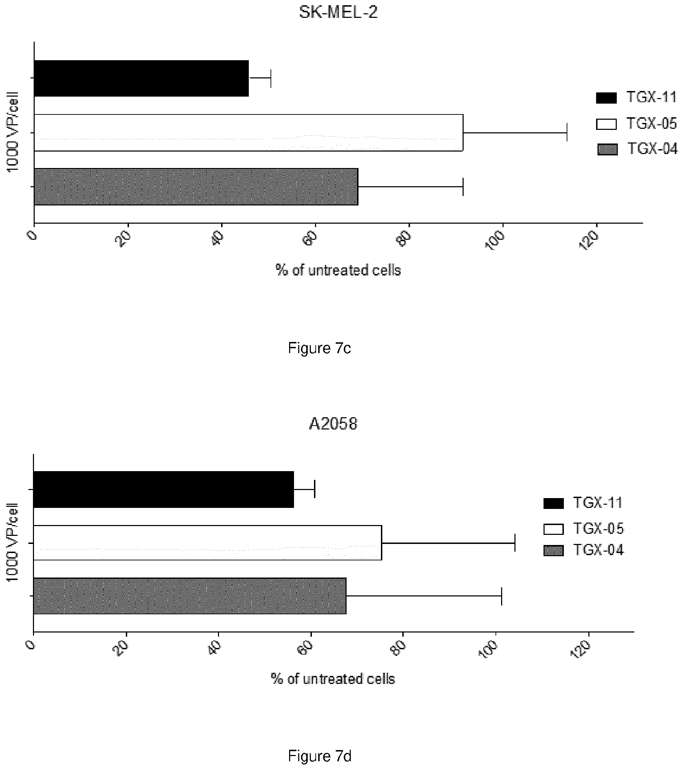

Figure 7. Figure 7 a) shows the effects of tested oncolytic adenoviruses on viability of human melanoma cell line A375 at the concentration of 1000 VP/cell after 72 hours post infection. Absorbance of untreated cells is expressed as 100% and absorbance of treated cells is expressed as a percentage of absorbance of untreated cells. Results are expressed as mean +/- SEM. Figure 7 b9 illustrates the effects tested oncolytic adenoviruses on viability of human melanoma cell line SK-MEL-2 at the concentration of 1 VP/cell after 72 hours post infection. Absorbance of untreated cells is expressed as 100% and absorbance of treated cells is expressed as a percentage of absorbance of untreated cells. Results are expressed as mean +/- SEM. Figure 7 c) shows the effects of tested oncolytic adenoviruses on viability of human melanoma cell line SK-MEL-2 at the concentration of 1000 VP/cell after 72 hours post infection. Absorbance of untreated cells is expressed as 100% and absorbance of treated cells is expressed as a percentage of absorbance of untreated cells. Results are expressed as mean +/- SEM. Figure 7 d) shows the effects of tested oncolytic adenoviruses on viability of human melanoma cell line A2058 at the concentration of 1000 VP/cell after 72 hours post infection. Absorbance of untreated cells is expressed as 100% and absorbance of treated cells is expressed as a percentage of absorbance of untreated cells. Results are expressed as mean +/- SEM.

### SEQUENCE LISTING

Sequence ID NO: 1. A nucleic acid sequence encoding an oncolytic adenoviral vector AdV5/3-D24-CMV(CuO)-ICOSL-IRES-ADA-SV40pA (TGX-11). ICOSL (functional expression cassette, location: 28,904 - 29,812bp) -IRES (29,813 - 30,384bp) -ADA (functional expression cassette, location: 31,207 - 31,950) -SV40pA expression under the control of CMV promoter (28,172 - 28,705) and CuO inserted into E3 region. The virus contains the 24-bp deletion in E1A CR2 (E1A Conserved Region 2 (CR2) with a 24-bp deletion that removed the strongest of two binding sites in E1A for Rb protein (LTCHEAGF), location: 907-951bp), expresses the Ad5/3 hybrid fiber (location: 32,885 - 34,648bp). Nucleotide sequence enclosed in attachment (TGX-13).
Sequence ID NO: 2. A nucleic acid sequence encoding an oncolytic adenoviral vector AdV5/3-D24-ICOSL (TGX-04). ICOSL (functional expression cassette, location: 28,371 - 29,279bp) inserted into E3 region. The virus contains the 24-bp deletion in E1A CR2 (E1A Conserved Region 2 (CR2) with a 24-bp deletion that removed the strongest of two binding sites in E1A for Rb protein (LTCHEAGF), 907 - 951bp), expresses the Ad5/3 hybrid fiber (location: 30,967 - 32,730bp). Nucleotide sequence enclosed in attachment (TGX-04).
Sequence ID NO: 3. A nucleic acid sequence encoding an oncolytic adenoviral vector AdV5/3-D24-ADA (TGX-05). ADA (functional expression cassette, location: 28,371 - 30,476bp) inserted into E3 region. The virus contains the 24-bp deletion in E1A CR2 (E1A Conserved Region 2 (CR2) with a 24-bp deletion that removed the strongest of two binding sites in E1A for Rb protein (LTCHEAGF), location: 907-951bp), expresses the Ad5/3 hybrid fiber (location: 32,164 - 33,927bp). Nucleotide sequence enclosed in attachment (TGX-05).
Sequence ID NO: 4. A binding site for Rb protein in E1A.
Sequence ID NO: 5. CR2-D24 (in construct TGX-04).
Sequence ID NO: 6. Ad5/3 fiber (TGX-04).
Sequence ID NO: 7. ICOSL (TGX-04).
Sequence ID NO: 8. CR2-D24 (TGX-05).
Sequence ID NO: 9. Ad5/3 fiber (TGX-05).
Sequence ID NO: 10. ADA (TGX-05).
Sequence ID NO: 11. CR2-D24 (TGX-11).
Sequence ID NO: 12. Ad5/3 fiber (TGX-11).
Sequence ID NO: 13. CMV promoter/enhancer (TGX-11).
Sequence ID NO: 14. CUO (TGX-11).
Sequence ID NO: 15. ICOSL (TGX-11).
Sequence ID NO: 16. IRES (TGX-11).
Sequence ID NO: 17. ADARB1 (ADA) (TGX-11).
Sequence ID NO: 18. SV40 poly(A) signal (TGX-11).
Sequence ID NO: 19. Primer 288-17.
Sequence ID NO: 20. Primer 288-41.
Sequence ID NO: 21. Primer 288-18.
Sequence ID NO: 22. Primer 288-19.
Sequence ID NO: 23. Primer 288-20.
Sequence ID NO: 24. Primer 288-21.

### DETAILED DESCRIPTION OF THE DISCLOSURE

The double transgene oncolytic adenoviral vector of the present invention is based on an adenovirus serotype 5 (Ad5) nucleic acid backbone with a 24 bp deletion (D24) in the Rb binding constant region 2 (CR2) of adenoviral E1. The vector comprises a nucleic acid sequence encoding a member of the B7 family of costimulatory ligands and a nucleic acid sequence encoding ADA in the place of the deleted 2.7kbp in the adenoviral E3 region. In a preferred embodiment of the invention, the adenoviral vector backbone is ONCOS-102 as disclosed in EP2379586B1, which comprises of a member of the B7 family of costimulatory ligands and ADA transgenes connected with IRES or P2A sequence in the place of GM-CSF/E3 region.

Preferably, the B7 family of immune-regulatory ligands is ICOSL. Instead of ICOSL also other ligands of the B7 family of immune-regulatory ligands, such as programmed death-1 ligand (PD-L1), programmed death-2 ligand (PD-L2), B7-H3, and B7-H4, can be use in the invention. Most preferably, a member of the B7 family of costimulatory ligands is human ICOSL.

Adenosine deaminase can be ADA1 or ADA2. Preferably ADA is human ADA1.

The results of the experiments showed surprising superior effect of double transgene virus. Double transgene virus coding for ICOSL and ADA1 (TGX-11) showed anti-cancer superiority over singe transgene viruses TGX-04 (coding for ICOSL) and TGX-05 (coding for ADA1) in several different cell types (Figures 7a-d).

Compared to a wild type adenovirus genome, the adenoviral vector of the invention lacks 24 base pairs from CR2 in E1 region, specifically in E1A region, and 2.7kbp in E3 region. 24 base pair deletion (D24) of E1 affects CR2 domain, which is responsible for binding the Rb tumor suppressor/cell cycle regulator protein and thus, allows the induction of the synthesis (S) phase i.e. DNA synthesis or replication phase. pRb and E1A interaction requires eight amino acids 121 to 127 of the E1A protein conserved region, which are deleted in the present invention. Viruses with the D24 are known to have a reduced ability to overcome the G1-S checkpoint and replicate efficiently in cells where this interaction is not necessary, e.g. in tumor cells defective in the Rb-p16 pathway.

The E3 region is nonessential for viral replication *in vitro,* but the E3 proteins have an important role in the regulation of host immune response i.e. in the inhibition of both innate and specific immune responses. The deletion in E3 refers to a deletion of 2.7k base pairs from the adenoviral E3A region. In a resulting adenoviral construct, where 2.7kbp along with gp19k and 6.7K genes are deleted. The gp19k gene product is known to bind and sequester major histocompatibility complex I (MHC1) molecules in the endoplasmic reticulum, and to prevent the recognition of infected cells by cytotoxic T-lymphocytes. Since many tumors are deficient in MHC1, deletion of gp19k increases tumor selectivity of viruses (virus is cleared faster than wild type virus from normal cells but there is no difference in tumor cells). 6.7K proteins are expressed on cellular surfaces and they take part in downregulating TNF-related apoptosis inducing ligand (TRAIL) receptor 2.

An internal ribosome entry site, abbreviated IRES, is an RNA element that allows for translation initiation in a cap-independent manner, as part of the greater process of protein synthesis. In eukaryotic translation, initiation typically occurs at the 5' end of mRNA molecules, since 5' cap recognition is required for the assembly of the initiation complex.

Instead of these large IRES sequences, a much shorter, self-cleaving 2A peptide can be used. Virus-derived peptide sequences mediate a ribosome-skipping event enables generation of multiple separate peptide products from one mRNA. Peptides from porcine teschovirus-1 (P2A) facilitate expression of multiple transgenes.

In the present invention, a member of the B7 family of costimulatory ligands and ADA transgenes are placed into a 2.7kbp of deleted E3 region, under the E3 promoter or CMV(Cuo) promoter. This restricts transgene expression to tumor cells that allow replication of the virus and subsequent activation of the E3 promoter. E3 promoter may be any exogenous or endogenous promoter known in the art, preferably endogenous promoter. In a preferred embodiment of the invention, a nucleic acid sequence encoding a member of the B7 family of costimulatory ligands and ADA are under the control of the viral E3 promoter. CMV(CuO) promoter contains a copy of the cumate operator (CuO). In "regular" 293 cells and other cell lines, the CuO operator is inactive and transcription takes place from the CMV promoter. In 293-CymR cells, which express the CymR repressor, the CymR repressor binds onto the CuO operator, thereby preventing the transcription of the transgene.

The vectors of the invention may also comprise other modifications than partial deletions of CR2 and E3 and insertion of ICOSL and ADA sequences as mentioned above. In a preferred embodiment of the invention, all the other regions of the Ad5 vector are of a wild type. In another preferred embodiment of the invention, the E4 region is of a wild type. In a preferred embodiment of the invention, a wild type region is located upstream of the E1 region. "Upstream" refers to immediately before the E1 region in the direction of expression. E1 B region may also be modified in the vector of the invention.

As used herein, "Ad5/3 chimerism" of the capsid refers to a chimerism, wherein the knob part of the fiber is from Ad serotype 3, and the rest of the fiber is from Ad serotype 5.

Expression cassettes are used for expressing transgenes in a target, such as a cell, by utilizing vectors. As used herein, the expression "expression cassette" refers to a DNA vector or a part thereof comprising nucleotide sequences, which encode cDNAs or genes, and nucleotide sequences, which control and/or regulate the expression of said cDNAs or genes. Similar or different expression cassettes may be inserted to one vector or to several different vectors. Ad5 vectors of the present invention may comprise either several or one expression cassettes. However, only one expression cassette is adequate. In a preferred embodiment of the invention, the oncolytic adenoviral vector comprises at least one expression cassette. In a preferred embodiment of the invention, the oncolytic adenoviral vector comprises only one expression cassette.

A cell comprising the adenoviral vector of the invention may be any cell such as a eukaryotic ceil, bacterial cell, animal cell, human cell, mouse cell etc. A cell may be an *in vitro, ex vivo* or *in vivo* cell. For example, the cell may be used for producing the adenoviral vector *in vitro, ex vivo* or *in vivo,* or the cell may be a target, such as a tumor cell, which has been infected with the adenoviral vector.

In a method of producing a member of the B7 family of costimulatory ligands and ADA transgenes in a cell, a vehicle comprising the vector of the invention is carried into a cell and furthermore a member of the B7 family of costimulatory ligands and ADA transgenes are expressed, and the protein is translated and secreted in a paracrine manner. "A vehicle" may be any viral vector, plasmid or other tool, such as a particle, which is able to deliver the vector of the invention to a target cell. Any conventional method known in the art can be used for delivering the vector to the cell.

Tumor specific immune response may be increased in a subject by the present invention. Helper T cells, cytotoxic T cells and/or natural killer cells are stimulated, produced and targeted as a consequence of the construct properties.

The recombinant Ad5 vectors of the invention have been constructed for replication competence in cells, which have defects in the Rb-pathway, specifically Rb-p16 pathway. These defective cells include all tumor cells in animals and humans. In a preferred embodiment of the invention, the vector is capable of selectively replicating in cells having defects in the Rb-pathway. As used herein "defects in the Rb-pathway" refers to mutations and/or epigenetic changes in any genes or proteins of the pathway. Due to these defects, tumor cells overexpress E2F and thus, binding of Rb by E1A CR2, that is normally needed for effective replication, is unnecessary.

Any cancers or tumors, including both malignant and benign tumors as well as primary tumors and metastasis may be targets of gene therapies. In a specific embodiment of the invention the cancer is any solid tumor. In a preferred embodiment of the invention, the cancer is selected from a group consisting of nasopharyngeal cancer, synovial cancer, hepatocellular cancer, renal cancer, cancer of connective tissues, melanoma, lung cancer, bowel cancer, colon cancer, rectal cancer, colorectal cancer, brain cancer, throat cancer, oral cancer, liver cancer, bone cancer, pancreatic cancer, choriocarcinoma, gastrinoma, pheochromocytoma, prolactinoma, T-cell leukemia/lymphoma, neuroma, von Hippel-Lindau disease, Zollinger-Ellison syndrome, adrenal cancer, anal cancer, bile duct cancer, bladder cancer, ureter cancer, brain cancer, oligodendroglioma, neuroblastoma, meningioma, spinal cord tumor, bone cancer, osteochondroma, chondrosarcoma, Ewing's sarcoma, cancer of unknown primary site, carcinoid, carcinoid of gastrointestinal tract, fibrosarcoma, breast cancer, Paget's disease, cervical cancer, colorectal cancer, rectal cancer, esophagus cancer, gall bladder cancer, head cancer, eye cancer, neck cancer, kidney cancer, Wilms' tumor, liver cancer, Kaposi's sarcoma, prostate cancer, lung cancer, testicular cancer, Hodgkin's disease, non-Hodgkin's lymphoma, oral cancer, skin cancer, mesothelioma, multiple myeloma, ovarian cancer, endocrine pancreatic cancer, glucagonoma, pancreatic cancer, parathyroid cancer, penis cancer, pituitary cancer, soft tissue sarcoma, retinoblastoma, small intestine cancer, stomach cancer, thymus cancer, thyroid cancer, trophoblastic cancer, hydatidiform mole, uterine cancer, endometrial cancer, vagina cancer, vulva cancer, acoustic neuroma, mycosis fungoides, insulinoma, carcinoid syndrome, somatostatinoma, gum cancer, heart cancer, Hp cancer, meninges cancer, mouth cancer, nerve cancer, palate cancer, parotid gland cancer, peritoneum cancer, pharynx cancer, pleural cancer, salivary gland cancer, tongue cancer and tonsil cancer.

A pharmaceutical composition of the invention comprises at least one type of the vectors of the invention. Furthermore, the composition may comprise at least two or three different vectors of the invention. In addition to the vector of the invention, a pharmaceutical composition may also comprise any other vectors, such as other adenoviral vectors, other therapeutically effective agents, any other agents such as pharmaceutically acceptable carriers, buffers, excipients, adjuvants, antiseptics, filling, stabilising or thickening agents, and/or any components normally found in corresponding products.

The pharmaceutical composition may be in any form, such as solid, semisolid or liquid form, suitable for administration. A formulation can be selected from a group consisting of, but not limited to, solutions, emulsions, suspensions, tablets, pellets and capsules.

The effective dose of vectors depends on at least the subject in need of the treatment, tumor type, location of the tumor and stage of the tumor. The dose may vary for example from about 10e8 viral particles (VP) to about 10e14 VP, preferably from about 5x10e9 VP to about 10e13 VP and more preferably from about 8x10e9 VP to about 10e12 VP. In one specific embodiment of the invention the dose is in the range of about 5x10e10 - 5x10e11 VP.

The vector or pharmaceutical composition of the invention may be administered to any eukaryotic subject selected from a group consisting of plants, animals and human beings, in a preferred embodiment of the invention, the subject is a human or an animal. An animal may be selected from a group consisting of pets, domestic animals and production animals.

Any conventional method may be used for administration of the vector or composition to a subject. The route of administration depends on the formulation or form of the composition, the disease, location of tumors, the patient, comorbidities and other factors. In a preferred embodiment of the invention, the administration is conducted through an intratumoral, intramuscular, intra-arterial, intravenous, intrapleural, intravesicular, intracavitary or peritoneal injection, or an oral administration.

Only one administration of oncolytic adenoviral vectors of the invention may have therapeutic effects. However, in a preferred embodiment of the invention, oncolytic adenoviral vectors or pharmaceutical compositions are administered several times during the treatment period. Oncolytic adenoviral vectors or pharmaceutical compositions may be administered for example from 1 to 10 times in the first 2 weeks, 4 weeks, monthly or during the treatment period. In one embodiment of the invention, administration is done three to seven times in the first 2 weeks, then at 4 weeks and then monthly. In a specific embodiment of the invention, administration is done four times in the first 2 weeks, then at 4 weeks and then monthly. The length of the treatment period may vary, and for example may last from two to 12 months or more.

The gene therapy of the invention is effective alone, but combination of adenoviral gene therapy with any other therapies, such as traditional therapy, may be more effective than either one alone. For example, each agent of the combination therapy may work independently in the tumor tissue, the adenoviral vectors may sensitize cells to chemotherapy or radiotherapy and/or chemotherapeutic agents may enhance the level of virus replication or effect the receptor status of the target cells. The agents of combination therapy may be administered simultaneously or sequentially.

In a preferred embodiment of the invention, the method or use further comprises administration of concurrent radiotherapy to a subject. In another preferred embodiment of the invention, the method or use further comprises administration of concurrent chemotherapy to a subject. As used herein "concurrent" refers to a therapy, which has been administered before, after or simultaneously with the gene therapy of the invention. The period for a concurrent therapy may vary from minutes to several weeks. Preferably the concurrent therapy lasts for some hours.

As used herein, the term "immune checkpoint inhibitor" or "checkpoint inhibitor" refers to molecules that totally or partially reduce, inhibit, interfere with or modulate one or more checkpoint proteins. Checkpoint proteins regulate T-cell activation or function. Central to the immune checkpoint process are the cytotoxic T-lymphocyte-associated antigen 4 (CTLA-4) and programmed death 1 (PD-1) immune checkpoint pathways. The CTLA-4 and PD-1 pathways are thought to operate at different stages of an immune response. CTLA-4 is considered the "leader" of the immune checkpoint inhibitors, as it stops potentially autoreactive T cells at the initial stage of naive T-cell activation, typically in lymph nodes. The PD-1 pathway regulates previously activated T cells at the later stages of an immune response, primarily in peripheral tissues.

Inhibition of the immune checkpoint pathways has led to the approval of several new drugs: ipilimumab (anti-CTLA-4; Yervoy®), pembrolizumab (anti-PD-1; Keytruda®), and nivolumab (anti-PD-1; Opdivo®). Also, PD-L1 inhibitors, such as Atezolizumab (MPDL3280), Avelumab (MSB0010718C) and Durvalumab (MEDI4736), are available. These antagonistic antibodies have been associated with objective clinical responses in cancer patients. Antibodies targeting CTLA-4 are already marketed (e.g. Ipilimumab, Yervoy, Bristol-Myers Squibb, BMS) for metastatic melanoma. Antibody therapies with anti PD-L1 (e.g. MPDL3280A, Roche), anti PD-1 (e.g. Nivolumab, BMS) are also ongoing.

Other immune-checkpoint inhibitors include lymphocyte activation gene-3 (LAG-3) inhibitors, such as IMP321, a soluble Ig fusion protein. Other immunecheckpoint inhibitors include B7 inhibitors, such as B7-H3 and B7-H4 inhibitors. In particular, the anti-B7-H3 antibody MGA271. Also included are TIM3 (T-cell immunoglobulin domain and mucin domain 3) inhibitors. In certain embodiments the PD-1 blockers include anti-PD-L1 antibodies. In certain other embodiments the PD-1 blockers include anti-PD-1 antibodies and similar binding proteins such as nivolumab (MDX 1106, BMS 936558, ONO 4538), a fully human IgG4 antibody 30 that binds to and blocks the activation of PD-1 by its ligands PD-L1 and PD-L2; lambrolizumab (MK-3475 or SCH 900475), a humanized monoclonal IgG4 antibody against PD-1 ; CT-011 a humanized antibody that binds PD-1 ; AMP-224 is a fusion protein of B7-DC; an antibody Fc portion; BMS-936559 (MDX- 1105-01) for PD-L1 (B7-H1) blockade. Further examples of PD-L1 inhibitors that can be used in certain embodiments are Atezolizumab (MPDL3280), Avelumab (MSB0010718C) and Durvalumab.

Preferably, anti-PD-1 antibodies pembrolizumab (Keytruda), and nivolumab (Opdivo) are used in the invention. Also, PD-L1 inhibitors, such as durvalumab can be used in combination with anti-PD-1-antibodies. The preferred checkpoint inhibitors of the present invention are thus those for PD-1 and PD-L1.

Agents suitable for combination therapy include but are not limited to All-trans retinoic acid, Azacitidine, Azathioprine, Bleomycin, Carboplatin, Capecitabïne, Cisplatin, Chlorambucil, Cyclophosphamide, Cytarabine, Daunorubicin, Docetaxel, Doxifluridine, Doxorubicin, Epirubicin, Epothilone, Etoposide, Fluorouracil, Gemcitabine, Hydroxyurea, Idarubicin, Imattnib, Mechlorethamine, Mercaptopurine, Methotrexate, Mitoxantrone, Oxaliplatin, Paclitaxel, Pemetrexed, Temozolomide, Teniposide, Tioguanine, Valrubicin, Vinblastine, Vincristine, Vindesine and Vinorelbine.

In a preferred embodiment of the invention, the method or use further comprises administration of verapamil or another calcium channel blocker to a subject. "Calcium channel blocker" refers to a class of drugs and natural substances which disrupt the conduction of calcium channels, and it may be selected from a group consisting of verapamil, dihydropyridines, gallopamil, diltiazem, mibefradil, bepridil, fluspirilene and fendiline.

In a preferred embodiment of the invention, the method or use further comprises administration of autophagy inducing agents to a subject. Autophagy refers to a catabolic process involving the degradation of a cell's own components through the lysosomal machinery. "Autophagy inducing agents" refer to agents capable of inducing autophagy and may be selected from a group consisting of, but not limited to, mTOR inhibitors, P13K inhibitors, lithium, tamoxifen, chloroquine, bafilomycin, temsirolimus, sirolimus and temozolomide. In a specific embodiment of the invention, the method further comprises administration of temozolomide to a subject. Temozolomide may be either oral or intravenous temozolomide.

In one embodiment of the invention, the method or use further comprises administration of chemotherapy or anti-CD20 therapy or other approaches for blocking of neutralizing antibodies. "Anti-CD20 therapy" refers to agents capable of killing CD20 positive cells and may be selected from a group consisting of rituximab and other anti-CD20 monoclonal antibodies. "Approaches for blocking of neutralizing antibodies" refers to agents capable of inhibiting the generation of anti-viral antibodies that normally result from infection and may be selected from a group consisting of different chemotherapeutics, immunomodulatory substances, corticoids and other drugs. These substances may be selected from a group consisting of, but not limited to, cyclophosphamide, cyclosporin, azathioprine, methylprenisolone, etoposide, CD40L, CTLA4Ig4, FK506 (tacrolismus), IL-12, IFN-gamma, interleukin 10, anti-CD8, anti-CD4 antibodies, myeloablation and oral adenoviral proteins.

The oncolytic adenoviral vector of the invention induces virion mediated oncolysis of tumor cells and activates human immune response against tumor cells. In a preferred embodiment of the invention, the method or use further comprises administration of substances capable to downregulating regulatory T-cells in a subject. "Substances capable to down- regulating regulatory T-cells" refers to agents that reduce the number of cells identified as T-suppressor or Regulatory T-cells. These cells have been identified as consisting one or many of the following immunophenotypic markers: CD4+, CD25+, FoxP3+, CD127- and GITR+. Such agents reducing T- suppressor or Regulatory T-cells may be selected from a group consisting of anti-CD25 antibodies or chemotherapeutics.

An object of the invention is an oncolytic adenoviral vector comprising an adenovirus serotype 5 (Ad5) nucleic acid backbone, a 24 bp deletion (D24) in the Rb binding constant region 2 of E1, a nucleic acid sequence encoding a member of the B7 family of costimulatory ligands and a nucleic acid sequence encoding ADA. A more preferred object of the invention is the oncolytic adenoviral vector, wherein a member of the B7 family of costimulatory ligands and ADA are placed under E3 promoter. Still another preferred embodiment is the oncolytic adenoviral vector, wherein a member of the B7 family of costimulatory ligands and ADA are placed under CMV(Cuo) promoter. Still a further preferred object is the oncolytic adenoviral vector, wherein there is a 2.7kbp deletion in the E3. Further, the oncolytic adenoviral vector, wherein the B7 family of costimulatory ligand is ICOSL and ADA is ADA1, is a preferred aspect of the invention. Also, the oncolytic adenoviral vector, wherein the nucleic acid sequence encoding a member of the B7 family of costimulatory ligands and the nucleic acid sequence encoding ADA are connected with IRES is a preferred aspect of the invention. The oncolytic adenoviral vector, wherein the nucleic acid sequence encoding a member of the B7 family of costimulatory ligands and the nucleic acid sequence encoding ADA are connected with P2A is also a preferred aspect of the invention. The oncolytic adenoviral vector comprising a native E1 A promoter is still another preferred aspect of the invention. Another object of the invention is this oncolytic adenoviral vector further comprising an Ad5/3 chimerism as a capsid modification. The nucleotide sequences of vector show at least 66%, preferably at least 70%, more preferably at least 75%, even more preferably at least 80% identity to SEQ ID NO: 1. In preferred object of the invention the oncolytic adenoviral vector comprises SEQ ID NO: 1.

One object of the invention is the oncolytic adenoviral vector comprising at least one expression cassette. In another object of the invention, the oncolytic adenoviral vector is capable of selectively replicating in cells having defects in the Rb-pathway.

Also, a cell comprising the adenoviral vector is one aspect of the invention.

Another aspect of the invention is a pharmaceutical composition comprising the adenoviral vector of the invention. The adenoviral vector for use in the treatment of cancer or in the manufacture of a medicament for treating cancer in a subject is also an aspect of the invention. The cancer can be selected from a group consisting of nasopharyngeal cancer, synovial cancer, hepatocellular cancer, renal cancer, cancer of connective tissues, melanoma, lung cancer, bowel cancer, colon cancer, rectal cancer, colorectal cancer, brain cancer, throat cancer, oral cancer, liver cancer, bone cancer, pancreatic cancer, choriocarcinoma, gastrinoma, pheochromocytoma, prolactinoma, T-cell leukemia/lymphoma, neuroma, von Hippel-Lindau disease, Zollinger-Ellison syndrome, adrenal cancer, anal cancer, bile duct cancer, bladder cancer, ureter cancer, brain cancer, oligodendroglioma, neuroblastoma, meningioma, spinal cord tumor, bone cancer, osteochondroma, chondrosarcoma, Ewing's sarcoma, cancer of unknown primary site, carcinoid, carcinoid of gastrointestinal tract, fibrosarcoma, breast cancer, Paget's disease, cervical cancer, colorectal cancer, rectal cancer, esophagus cancer, gall bladder cancer, head cancer, eye cancer, neck cancer, kidney cancer, Wilms' tumor, liver cancer, Kaposi's sarcoma, prostate cancer, lung cancer, testicular cancer, Hodgkin's disease, non-Hodgkin's lymphoma, oral cancer, skin cancer, mesothelioma, multiple myeloma, ovarian cancer, endocrine pancreatic cancer, glucagonoma, pancreatic cancer, parathyroid cancer, penis cancer, pituitary cancer, soft tissue sarcoma, retinoblastoma, small intestine cancer, stomach cancer, thymus cancer, thyroid cancer, trophoblastic cancer, hydatidiform mole, uterine cancer, endometrial cancer, vagina cancer, vulva cancer, acoustic neuroma, mycosis fungoides, insulinoma, carcinoid syndrome, somatostatinoma, gum cancer, heart cancer, lip cancer, meninges cancer, mouth cancer, nerve cancer, palate cancer, parotid gland cancer, peritoneum cancer, pharynx cancer, pleural cancer, salivary gland cancer, tongue cancer and tonsil cancer.

The subject of the treatment can be a human or an animal.

The treatment of cancer with adenoviral vector in a subject can be conducted through an intratumoral, intramuscular, intra-arterial, intravenous, intrapleural, intravesicular, intracavitary or peritoneal injection, or an oral administration. The injection or the administration can be conducted several times during the treatment period. Also, an oncolytic adenoviral vector having a different fiber knob of the capsid compared to the vector of the earlier treatment can be used. The treatment of cancer in a subject can further comprise an administration of concurrent radiotherapy and/or concurrent chemotherapy to a subject. The treatment of cancer in a subject can further comprise an administration of a checkpoint inhibitor, verapamil or another calcium channel blocker, autophagy inducing agents, temozolomide, chemotherapy or anti-CD20 therapy or other approaches for blocking of neutralizing antibodies, and/or substances capable to downregulating regulatory T-cells in a subject.

A method of producing a member of the B7 family of costimulatory ligands and ADA in a cell *in vitro,* wherein the method comprises:
a) carrying a vehicle comprising an oncolytic adenoviral vector to a cell, and
b) expressing a member of the B7 family of costimulatory ligands and ADA of said vector in the cell,
is an aspect of the present invention.

Also, a use of the oncolytic adenoviral vector for producing a member of the B7 family of costimulatory ligands and ADA in a cell *in vitro* is an aspect of the invention.

A further object of the invention is a method of treating cancer in a human subject in need thereof, wherein a therapeutically effective amount of a pharmaceutical formulation comprising an oncolytic adenovirus comprising at least 75%, even more preferably at least 80% identity to SEQ ID NO: 1. Still more preferably, the nucleotide sequence show at least 85% or at least 90% or 95%, more preferably at least 98%, most preferably 99% identity to the nucleic acid sequence of SEQ ID NO: 1.

A further object of the invention is a method of treating cancer in a human subject in need thereof, wherein a therapeutically effective amount of a pharmaceutical formulation comprising an oncolytic adenovirus comprising nucleotide sequence having at least 70% identity to SEQ ID NO: 1 and a diluent or carrier is administered. In the most preferred embodiment the nucleotide sequence is SEQ ID NO: 1. Another object of the invention is a method, wherein a therapeutically effective amount of a pharmaceutical formulation comprising a combination of oncolytic adenovirus comprising a nucleotide sequence having at least 70% identity to SEQ ID NO: 2 and oncolytic adenovirus comprising a nucleotide sequence having at least 70% identity to SEQ ID NO: 3 is administered. In a preferred object of the invention, a therapeutically effective amount of a pharmaceutical formulation comprising a combination of oncolytic adenovirus comprising SEQ ID NO: 2 and oncolytic adenovirus comprising SEQ ID NO: 3 is administered. Said methods can further comprise administering an effective amount of an oncolytic adenoviral vector or a pharmaceutical composition comprising the oncolytic adenoviral vector to the subject through intratumoral injection, wherein the oncolytic adenoviral vector comprises the adenovirus according to the invention. In said methods the effective amount of the oncolytic adenoviral vector contains between 5X10e10 - 5X10e11 viral particles. They also induce a specific antitumor immune response, including activation of CD8+ T cells, in tumors expressing a member of the B7 family of costimulatory ligands and ADA from the oncolytic adenoviral vector. In said methods for treatment, the oncolytic adenoviral vector can be administered three to seven times in the first two weeks of treatment.

In said treatment methods cancer can be selected from a group consisting of nasopharyngeal cancer, synovial cancer, hepatocellular cancer, renal cancer, cancer of connective tissues, melanoma, lung cancer, bowel cancer, colon cancer, rectal cancer, colorectal cancer, brain cancer, throat cancer, oral cancer, liver cancer, bone cancer, pancreatic cancer, choriocarcinoma, gastrinoma, pheochromocytoma, prolactinoma, T-cell leukemia/lymphoma, neuroma, von Hippel-Lindau disease, Zollinger-Ellison syndrome, adrenal cancer, anal cancer, bile duct cancer, bladder cancer, ureter cancer, brain cancer, oligodendroglioma, neuroblastoma, meningioma, spinal cord tumor, bone cancer, osteochondroma, chondrosarcoma, Ewing's sarcoma, cancer of unknown primary site, carcinoid, carcinoid of gastrointestinal tract, fibrosarcoma, breast cancer, Paget's disease, cervical cancer, colorectal cancer, rectal cancer, esophagus cancer, gall bladder cancer, head cancer, eye cancer, neck cancer, kidney cancer, Wilms' tumor, liver cancer, Kaposi's sarcoma, prostate cancer, lung cancer, testicular cancer, Hodgkin's disease, non-Hodgkin's lymphoma, oral cancer, skin cancer, mesothelioma, multiple myeloma, ovarian cancer, endocrine pancreatic cancer, glucagonoma, pancreatic cancer, parathyroid cancer, penis cancer, pituitary cancer, soft tissue sarcoma, retinoblastoma, small intestine cancer, stomach cancer, thymus cancer, thyroid cancer, trophoblastic cancer, hydatidiform mole, uterine cancer, endometrial cancer, vagina cancer, vulva cancer, acoustic neuroma, mycosis fungoides, insulinoma, carcinoid syndrome, somatostatinoma, gum cancer, heart cancer, lip cancer, meninges cancer, mouth cancer, nerve cancer, palate cancer, parotid gland cancer, peritoneum cancer, pharynx cancer, pleural cancer, salivary gland cancer, tongue cancer and tonsil cancer.

According to one embodiment, the administration of the adenoviral vector or vectors or pharmaceutical composition can be conducted through an intratumoral, intramuscular, intra-arterial, intravenous, intrapleural, intravesicular, intracavitary or peritoneal injection, or an oral administration. According to another embodiment, the adenoviral vector or vectors or pharmaceutical composition can be administered several times during a treatment period. According to a preferred embodiment, concurrent radiotherapy can be administered to the subject. According to a more preferred embodiment, concurrent chemotherapy can be administered to the subject. According to a still more preferred embodiment verapamil or another calcium channel blocker can be administered to the subject.

In one preferred object of the invention, is a method for treatment with the adenoviral vector or vectors further comprising administering substances capable to downregulating regulatory T-cells in the subject.

Any method or use of the invention may be either *in vivo, ex vivo* or *in vitro* method or use.

The present invention is illustrated by the following examples, which are not intended to be limiting in any way.

### EXAMPLES

### Cloning of oncolytic viruses

Oncolytic viruses were constructed using genetic engineering tools. The construction of oncolytic vectors included the following steps:

### 1. Shuttle Plasmid Constructions:

For TGX-04/05, the transgenes were inserted into the E3 region at the exact same location as the GM-CSF coding sequence in vector Ad5/3-d24-GMCSF. In that vector, a 0.9 kb BsiWI-Mfel fragment encompassing the E3 6.7K ORF and gp19K ORF was deleted and replaced by the GM-CSF coding sequence. For this cloning we used shuttle plasmid pAd1129-14, which contain the WT E3 region and a hybrid Ad5/3 fiber. The ICOSL ligand (ICOSL), ADA (ADARB1) sequences were inserted between the BsiWI and Mfel site of pAd1129-14 using standard cloning methods (Figure 1). The identity of the shuttle plasmids was verified by restriction analysis and sequencing.

For TGX-11, the dicistronic cassettes CMV-ICOSL-IRES-ADA-SV40pA was inserted in place of the E3 region deleted for most of its genes. We used shuttle plasmid pAd1129-27, which contains the Ad5 E3 region and a hybrid Ad5/3 fiber gene. A 2.7 kb BgIII fragment was deleted from the E3 region and replaced with a multiple cloning site. Both dicistronic cassettes were inserted into pAd1129-27 using standard cloning methods (Figure 2). The identity of the plasmids was verified by restriction analysis and sequencing.

### 2. Cosmid Construction

The entire genomes of the recombinant adenoviruses were reconstituted in cosmids (Figure 3). Transfection-grade cosmid DNAs were purified and their identity was confirmed by restriction analysis.

### 3. Virus rescue and characterization

Excision of the adenovirus genome from the cosmids obtained in step 2, and transfection into 293 or 293-Cymr or A549 cells. Three virus plaques per construct were harvested and amplified in small scale. Their identity will be verified by restriction analysis of their genome using 3 enzymes (Hirt supernatant).

### 4. Western Blot

Expression of the various transgenes from the viruses were assessed by infecting a reporter cell line with the various virus clones, lysing the cells 24 hours post-infection, separating the total protein content by PAGE, and performing western blotting (WB) by using anti-ICOSL and anti-ADA antibodies to detect the various transgene products by chemiluminescence. These experiments will enable us to select virus clones for amplification and purification.

### 5. Small-scale amplification

Small-scale amplification of the viral clones, purification on two CsCI gradients, dialysis against an isotonic buffer (GTS buffer: 2.5% glycerol, 25 mM NaCl, 20 mM Tris-HCI, pH 8.0), filtration through a low-protein binding 0.22 µm filter, and virus particle (VP) concentration determination by ultraviolet absorbance (Abs260-SDS method) was carried out.

### 6. Confirmation of virus identity by sequencing

The identity of the purified recombinant adenoviruses was confirmed by sequencing regions of the virus genomes specific for these recombinant viruses, including the 24-bp deletion in the E1A CDS, the entire mono- or dicistronic cassettes inserted into the E3 region, the Ad5/3 hybrid fiber, and the junctions between the DNA fragments ligated to each other during the cosmid construction. Experimental sequences were assembled into contigs and aligned with the expected sequences of the viruses. Viral genomic DNAs were extracted from CsCI-purified virus particles using proteinase K and resuspended into 30 µL TE pH 7.5. Their quality was assessed by spectrophotometry (Abs260/280/320 nm) and agarose gel electrophoresis. TGX viruses' DNAs were sent to a third party for shotgun library making and sequencing. Libraries were generated using a Nextera XT kit (Illumina, Inc). They were pooled, quantified by qPCR, and sequenced on a 2x300 MiSeq run (Illumina, Inc)..fastq files were processed using the program "BWA Aligner". The software Geneious v11.1.5 (Biomatters Ltd) was used to visualize the .bam files and calculate the consensus sequences. The consensus sequences were aligned to the predicted sequences of the virus genomes using the software SnapGene v4.2.6.

### 7. Titration of the viral suspension

Titration of the viral suspension: determination of the concentration of infectious units per mL virus suspension was performed by TCID50 assay.

### Cloning of TGX-04 (Ad5/3-D24-ICOSL) in more detail

To construct, amplify, purify, titrate and characterize an oncolytic adenovirus vector characterized by the following features:
- 24-bp deletion in E1A CR2
- a sequence encoding ICOSL embedded into the E3 region
- a hybrid Ad5/3 fiber

Recombinant adenovirus TGX04 was constructed by reconstituting its entire genome in a cosmid (pTGX04). The following 3 DNA fragments were ligated to each other:
1. The 27 kb Pacl-Spel fragment from pAd5/3-d24-WTE3
2. The 8833 bp Spel-Pacl fragment from pTGX103
3. The 8 kb Pacl fragment from pAd5dSfi

Where:
- pAd5/3-d24-WTE3 (pAd5/3-d24-GMCSF)
- pTGX103 is a plasmid that contains the right end of the virus genome (Spel-Pacl sequence from pAd5/3.d24-WTE3) with the ICOSL coding sequence inserted between the BsiWI and Mfel sites in the E3 region
- pAd5dSfil is a cosmid that contains the entire Ad5 genome. The 8 kb Pacl fragment provides the cosmid backbone for pTGX04

pTGX103 was constructed by inserting the 1070 bp BsiWI-Notl DNA fragment from pAd1129-ICOSL between the corresponding sites of pTGX101
Where:
- pTGX101 is a plasmid that contains the right end of the Ad5/3.d24-WTE3 genome (Spel-Pacl fragment from pAd5/3.d24-WTE3)
- pAd1129-ICOSL is a shuttle plasmid that contains the ICOSL coding sequencer between the BsiWI and Mfel site in the E3 region

pAd1129-ICOSL was constructed by inserting a 969 bp synthetic dsDNA encoding ICOSL between the BsiWI and Mfel sites of pAd1129-14
Where:
- pAd1129-14 is a shuttle plasmid containing the WT Ad5 E3 region and a hybrid Ad5/3 fiber
- gBlock-ICOSL (transcript variant 1, 909 bp, NM_015259.4)

### Cloning of TGX-05 (Ad5/3-D24-ADA) in more detail

To construct, amplify, purify, titrate and characterize an oncolytic adenovirus vector characterized by the following features:
- 24-bp deletion in E1A CR2
- a sequence encoding ADARB1 embedded into the E3 region
- a hybrid Ad5/3 fiber

Recombinant adenovirus TGX05 was constructed by reconstituting its entire genome in a cosmid (pTGX05). The following 3 DNA fragments were ligated to each other:
1. The 27 kb Pacl-Spel fragment from pAd5/3-d24-WTE3
2. The 10030 bp Spel-Pacl fragment from pTGX104
3. The 8 kb Pacl fragment from pAd5dSfi
Where:
- pAd5/3-d24-WTE3 (pAd5/3-d24-GMCSF)
- pTGX104 is a plasmid that contains the right end of the virus genome (Spel-Pacl sequence from pAd5/3.d24-WTE3) with the ADARB1 coding sequence inserted between the BsiWI and Mfel sites in the E3 region
- pAd5dSfil is a cosmid that contains the entire Ad5 genome. The 8 kb Pacl fragment provides the cosmid backbone for pTGX05

pTGX104 was constructed by inserting the 2267 bp BsiWI-Notl DNA fragment from pAd1129-ADARB1 between the corresponding sites of pTGX101:
Where:
- pTGX101 is a plasmid that contains the right end of the Ad5/3.d24-WTE3 genome (Spel-Pacl fragment from pAd5/3.d24-WTE3)
- pAd1129-ADARB1 is a shuttle plasmid that contains the ADARB1 coding sequence between the BsiWI and Mfel site in the E3 region

pAd1129-ADARB1 was constructed by inserting a 2166 bp synthetic dsDNA encoding ADARB1 between the BsiWI and Mfel sites of pAd1129-14:
Where:
- pAd1129-14 is a shuttle plasmid containing the WT Ad5 E3 region and a hybrid Ad5/3 fiber
- gBlock-ADARB1 (GenBank # NM_001112.3)

### Cloning of TGX-11 (Ad5/3-D24-ICOSL-ADA) in more detail

To construct, amplify, purify, titrate and characterize an oncolytic adenovirus vector characterized by the following features:
- 24-bp deletion in E1A CR2
- a dicistronic cassette expressing ICOSL and ADARB1 in place of the E3 region (2.7 kb BgIII deletion)
- a hybrid Ad5/3 fiber

Recombinant adenovirus TGX11 was constructed by reconstituting its entire genome in a cosmid (pTGX11). The following 3 DNA fragments were ligated to each other:
1. The 27 kb Pacl-Spel fragment from pAd5/3-d24-WTE3
2. The 10751 bp Spel-Pacl fragment from pTGX110c
3. The 8 kb Pacl fragment from pAd5dSfi
Where:
- pAd5/3-d24-WTE3 (pAd5/3-d24-GMCSF)
- pTGX110c is a plasmid that contains the right end of the virus genome (Spel-Pacl sequence from pAd5/3.d24-WTE3) with the CMV-ICOSL-IRES-ADARB1 cassette inserted in place of the E3 region
- pAd5dSfil is a cosmid that contains the entire Ad5 genome. The 8 kb Pacl fragment provides the cosmid backbone for pTGX11

pTGX110c was constructed by ligating the following 3 DNA fragments to each other:
1. the 1349 bp Csil-Rsrll fragment from pTGX101
2. the 6491 bp Rsrll-Nhel fragment from pTGX101
3. the 5482 bp Nhel-Csil fragment from pTGX110b
Where:
- pTGX101 is a plasmid that contains the right end of the Ad5/3.d24-WTE3 genome (Spel-Pacl fragment from pAd5/3.d24-WTE3)
- pTGX110b is a plasmid that contains the CMV-ICOSL-IRES-ADARB1-SV40pA signal cassette in place of the E3 region (2.7 kb BgIII deletion)

pTGX110b was constructed by digesting pTGX110 with Spel, filling-in the sticky ends with Klenow, and religating both fragments with each other, in order to destroy the Spel sites flanking the CMV-ICOSL-IRES-ADARB1 cassette.
Where:
- pTGX110 is a plasmid that contains the CMV-ICOSL-IRES-ADARB1-SV40pA signal cassette in place of the E3 region (2.7 kb BgIII deletion)

pTGX110 was constructed by inserting the following 4 fragments between the Sacll and Acc65I sites of pAd1129-GrB-F:
1. the 945 bp ICOSL coding sequence amplified by PCR using pAd1129-ICOSL as template and primers 288-17 and 288-41
2. the 645 bp IRES-att element amplified by PCR using pAd1129-IRES-att as template and primers 288-18 and 288-19
3. the 2112 bp ADARB1 coding sequence isolated from plasmid pAd1129-ADARB1 by BsiWI/Mfel digestion
4. the 174 bp SV40pA signal amplified by PCR using pAd1129-GrB-F as template and primers 288-20 and 288-21.
Where:
- pAd1129-ICOSL was made for the construction of TGX04
- pAd1129-ADARB1 was made for the construction of TGX05
- pAd1129-GrB-F was made for the construction of TGX01
- pAd1129-IRES-att is a plasmid that contains an attenuated version of the EMCV Internal Ribosome Entry Site (IRES)

### Primers:

288-17: 
ACACCGGGACCGATCCAGCCTCCGCGGCCGCCACCATGCGGCTGGGCAGTCCT
GGACTGC
288-41: TCAAACGTGGCCAGTGAGCTCTGTC
288-18: AGCTCACTGGCCACGTTTGACCCCTCTCCCTCCCCCCCCT
288-19: TCTTCATCTTCTATATCCATGTCGACTCTAGAGGATCCCG
288-20: AGTTCTCACTCACGCCCTGAAACTTGTTTATTGCAGCTTA
288-21: TAGTTAAAGGGAATAAGATCGGTACC.

### Cell lines

Melanoma cell lines, A2058 (ATCC® CRL-1147™), A375 (ATCC® CRL-1619™) and SK-Mel-2 (ATCC® HTB-68™) were cultured in DMEM high glucose medium supplemented with 10% FCS and penicillin/streptomycin. Trypsin-EDTA was used to detach and subculture cells at 80% confluence for *in vitro* assays.

### Data analyses

All parameters were analysed using GraphPad Prism software (version 7). Statistical analyses have been performed using RM-one-way ANOVA followed by a Tukey post-test or T test (Mann Whitney Test).

### EXAMPLE 1 In vitro - cell viability assay (MTS)

Cell viability was evaluated by using the MTS Cell Proliferation Assay kit (Abcam) according to the manufacturer's instructions with the following modifications. MTS assay kit is a colorimetric method for the sensitive quantification of viable cells. It can be used to assess cell proliferation, cell viability and cytotoxicity. The MTS assay is based on the reduction of the MTS tetrazolium compound by viable mammalian cells to generate a coloured formazan dye that is soluble in cell culture media. This conversion is thought to be carried out by NAD(P)H-dependent dehydrogenase enzymes in metabolically active cells. The formazan dye is quantified by measuring the absorbance at 490 nm. Cells were seeded in 96 well plate at a concentration of 3000 and 5000 cells/well. Treatment was initiated in a final volume of 200µL. 72 hours later, MTS assay was performed according to manufacturer protocol. Cells were incubated with and without 0.1, 1, 10, 100 and 1000 viral particles (VP) of tested virus. The plates were shaken briefly on a shaker and absorbance measured at 490 nm. Absorbance values for untreated cells were set to correspond to 100% viability, and viability of treated cells was expressed as a percentage of the untreated control absorbance value.

Double transgene virus coding for ICOSL and ADA (TGX-11) have shown anti-cancer superiority when administered at the concentration of 1000VP/cell (respectively 33,51% of untreated cells) over singe transgene viruses TGX-04 (coding for ICOSL, 60,94% of untreated cells) and TGX-05 (coding for ADA, 64,71% of untreated cells) *in vitro* against A375 cell line (Figure 7a).

Double transgene vector showed also anti-cancer superiority (TGX-11) over single transgene vectors (TGX-04 and TGX-05) in SK-MEL-2 cell lines when tested at the concentration of 1 and 100VP/cell (respectively for 1 VP/cell: 79,69; 114,22; 100,73 and 1000VP/cell: 45,83%; 69,19%; 91,56% of untreated cells), (Figure 7b and 7c).

Enhanced anticancer effect of TGX-11 (56,29 % of untreated cells) was recorded also in A2058 cell line also at the concentration 1000VP/cell over single transgene vectors TGX-04 and TGX-05 (receptively 67,72 and 75,5% of untreated cells), (Figure 7d).

### REFERENCES

Allard, B., Beavis, P. A., Darcy, P. K. & Stagg, J. 2016. Immunosuppressive Activities of Adenosine In Cancer. Curr Opin Pharmacol, 29, 7-16.
Fu, T., He, Q. & Sharma, P. 2011. The Icos/IcosI Pathway is Required for Optimal Antitumor Responses Mediated by Anti-Ctla-4 Therapy. Cancer Res, 71, 5445-54.
Hutloff, A., Dittrich, A. M., Beier, K. C., Eljaschewitsch, B., Kraft, R., Anagnostopoulos, I. & Kroczek, R. A. 1999. Icos is an Inducible T-Cell Co-Stimulator Structurally and Functionally Related To Cd28. Nature, 397, 263-6.
Khayyamian, S., Hutloff, A., Buchner, K., Grafe, M., Henn, V., Kroczek, R. A. & Mages, H. W. 2002. Icos-Ligand, Expressed on Human Endothelial Cells, Costimulates Th1 And Th2 Cytokine Secretion By Memory Cd4+ T Cells. Proc Natl Acad Sci U S A, 99, 6198-203.
Kuryk, L., Haavisto, E., Garofalo, M., Capasso, C., Hirvinen, M., Pesonen, S., Ranki, T., Vassilev, L. & Cerullo, V. 2016. Synergistic Anti-Tumor Efficacy of Immunogenic Adenovirus Oncos-102 (Ad5/3-D24-Gm-Csf) And Standard of Care Chemotherapy in Preclinical Mesothelioma Model. Int J Cancer, 139, 1883-93.
Kuryk, L., Møller, A.-S. W. & Jaderberg, M. 2018. Combination of Immunogenic Oncolytic Adenovirus Oncos-102 With Anti-Pd-1 Pembrolizumab Exhibits Synergistic Antitumor Effect in Humanized A2058 Melanoma Hunog Mouse Model. Oncoimmunology, 8, 1-11.
Kutryb-Zajac, B., Koszalka, P., Mierzejewska, P., Bulinska, A., Zabielska, M. A., Brodzik, K., Skrzypkowska, A., Zelazek, L., Pelikant-Malecka, I., Slominska, E. M. & Smolenski, R. T. 2018. Adenosine Deaminase Inhibition Suppresses Progression Of 4t1 Murine Breast Cancer by Adenosine Receptor-Dependent Mechanisms. J Cell Mol Med, 22, 5939-5954.
Londoño-R, L. M., Cowell, J., Wang, L., Zhao, Q., Huang, L., Thanos, C., Labarre, M. J., Li, X. & Cekic, C. 2018. Abstract 1755: Pegylated Adenosine Deaminase (Ada2) Prevents Adenosine-Mediated Increase in Tumor Growth and Improves Antitumor Immune Responses. Cancer Research, 78, 1755-1755.
Muller-Haegele, S., Muller, L. & Whiteside, T. L. 2014. Immunoregulatory Activity of Adenosine and Its Role in Human Cancer Progression. Expert Rev Clin Immunol, 10, 897-914.
Ohta, A. 2016. A Metabolic Immune Checkpoint: Adenosine in Tumor Microenvironment. Front Immunol, 7, 109.

## Claims

1. An oncolytic adenoviral vector comprising an adenovirus serotype 5 (Ad5) nucleic acid backbone, a 24 bp deletion (D24) in the Rb binding constant region 2 of E1, a nucleic acid sequence encoding a member of the B7 family of costimulatory ligands and a nucleic acid sequence encoding ADA in the E3 region.

2. The oncolytic adenoviral vector according to claim 1, wherein the member of the B7 family of costimulatory ligands and ADA are placed under E3 promoter.

3. The oncolytic adenoviral vector according to claim 1, wherein the member of the B7 family of costimulatory ligands and ADA are placed under CMV(Cuo) promoter.

4. The oncolytic adenoviral vector according to any one of claims 1-3, wherein the B7 family of costimulatory ligand is ICOSL and ADA is ADA1.

5. The oncolytic adenoviral vector according to any one of claims 1-4, wherein the nucleic acid sequence encoding a member of the B7 family of costimulatory ligands and the nucleic acid sequence encoding ADA are connected with IRES.

6. The oncolytic adenoviral vector according to any one of claims 1-4, wherein the nucleic acid sequence encoding the member of the B7 family of costimulatory ligands and the nucleic acid sequence encoding ADA are connected with P2A.

7. The oncolytic adenoviral vector according to any one of claims 1-6 comprising a native E1 A promoter.

8. The oncolytic adenoviral vector according to any one of claims 1-7 further comprising an Ad5/3 chimerism as a capsid modification.

9. The oncolytic adenoviral vector according to any one of claims 1, 3-5 or 7-8 comprising a nucleotide sequence having at least 70% identity to SEQ ID NO: 1.

10. A cell comprising the adenoviral vector according to any one of claims 1-9.

11. A pharmaceutical composition comprising the adenoviral vector according to any one of claims 1-9.

12. The adenoviral vector according to any one of claims 1-9 for use in the treatment of cancer or in the manufacture of a medicament for treating cancer in a subject.

13. The adenoviral vector for use according to claim 12, wherein the cancer is selected from a group consisting of nasopharyngeal cancer, synovial cancer, hepatocellular cancer, renal cancer, cancer of connective tissues, melanoma, lung cancer, bowel cancer, colon cancer, rectal cancer, colorectal cancer, brain cancer, throat cancer, oral cancer, liver cancer, bone cancer, pancreatic cancer, choriocarcinoma, gastrinoma, pheochromocytoma, prolactinoma, T-cell leukemia/lymphoma, neuroma, von Hippel-Lindau disease, Zollinger-Ellison syndrome, adrenal cancer, anal cancer, bile duct cancer, bladder cancer, ureter cancer, brain cancer, oligodendroglioma, neuroblastoma, meningioma, spinal cord tumor, bone cancer, osteochondroma, chondrosarcoma, Ewing's sarcoma, cancer of unknown primary site, carcinoid, carcinoid of gastrointestinal tract, fibrosarcoma, breast cancer, Paget's disease, cervical cancer, colorectal cancer, rectal cancer, esophagus cancer, gall bladder cancer, head cancer, eye cancer, neck cancer, kidney cancer, Wilms' tumor, liver cancer, Kaposi's sarcoma, prostate cancer, lung cancer, testicular cancer, Hodgkin's disease, non-Hodgkin's lymphoma, oral cancer, skin cancer, mesothelioma, multiple myeloma, ovarian cancer, endocrine pancreatic cancer, glucagonoma, pancreatic cancer, parathyroid cancer, penis cancer, pituitary cancer, soft tissue sarcoma, retinoblastoma, small intestine cancer, stomach cancer, thymus cancer, thyroid cancer, trophoblastic cancer, hydatidiform mole, uterine cancer, endometrial cancer, vagina cancer, vulva cancer, acoustic neuroma, mycosis fungoides, insulinoma, carcinoid syndrome, somatostatinoma, gum cancer, heart cancer, lip cancer, meninges cancer, mouth cancer, nerve cancer, palate cancer, parotid gland cancer, peritoneum cancer, pharynx cancer, pleural cancer, salivary gland cancer, tongue cancer and tonsil cancer.

14. The adenoviral vector for use according to claim 12 or 13, wherein the treatment of cancer in a subject is conducted through an intratumoral, intramuscular, intra-arterial, intravenous, intrapleural, intravesicular, intracavitary or peritoneal injection, or an oral administration.

15. The adenoviral vector for use according to claim 14, wherein the injection or the administration is conducted several times during the treatment period, or wherein an oncolytic adenoviral vector having a different fiber knob of the capsid compared to the vector of the earlier treatment is used.

16. The adenoviral vector for use according to any one of claims 12-15, wherein the treatment of cancer in a subject further comprises an administration of a checkpoint inhibitor, verapamil or another calcium channel blocker, autophagy inducing agents, temozolomide, chemotherapy or anti-CD20 therapy or other approaches for blocking of neutralizing antibodies, substances capable to downregulating regulatory T-cells in a subject, and/or cyclophosphamide to a subject.

17. A method of producing a member of the B7 family of costimulatory ligands and ADA in a cell *in vitro,* wherein the method comprises:
i. carrying a vehicle comprising an oncolytic adenoviral vector according to any one of claims 1-9 to a cell, and
ii. expressing a member of the B7 family of costimulatory ligands and ADA of said vector in the cell.

18. A use of the oncolytic adenoviral vector according to any one of claims 1-9 for producing a member of the B7 family of costimulatory ligands and ADA in a cell *in vitro.*
